(19) Europäisches Patentamt
European Patent Office
Office européen des brevets

(11) Numéro de publication: **0 097 572**
**B1**

(12) # FASCICULE DE BREVET EUROPÉEN

(45) Date de publication du fascicule du brevet: **05.11.86**

(21) Numéro de dépôt: **83401178.5**

(22) Date de dépôt: **09.06.83**

(51) Int. Cl.⁴: **C 07 J 1/00,** C 07 J 41/00, C 07 J 43/00, C 07 J 51/00, C 07 J 71/00, A 61 K 31/565, A 61 K 31/50

(54) **Nouveaux 19-nor stéroides substitués en 11 et éventuellement en 2,leur préparation, leur application comme médicaments, les compositions les renfermant et les nouveaux intermédiaires obtenus.**

(30) Priorité: **11.06.82 FR 8210205**

(43) Date de publication de la demande: **04.01.84 Bulletin 84/1**

(45) Mention de la délivrance du brevet: **05.11.86 Bulletin 86/45**

(84) Etats contractants désignés: **AT BE CH DE FR GB IT LI LU NL SE**

(56) Documents cité:
**EP-A-0 057 115**
**FR-A-2 377 418**
**FR-A-2 377 419**

(73) Titulaire: **ROUSSEL- UCLAF, 35, boulevard des Invalides, F-75007 Paris (FR)**

(72) Inventeur: **Teutsch, Jean Georges, Résidence Lavoisier bât. 3 3, rue Lavoisier, F-93500 Pantin (FR)**
Inventeur: **Torelli Vesperto, 5, rue la Convention, F-94700 Maisons- Alfort (FR)**
Inventeur: **Deraedt, Roger, 23, Alée Jean- Baptiste Clément, F-93320 Pavillons- sous- Bois (FR)**
Inventeur: **Philibert, Daniel, 16, rue Chevalier, F-94210 La Varenne Saint- Hilaire (FR)**

(74) Mandataire: **Bourgouin, André, Département des Brevets ROUSSEL UCLAF B.P. no 9, F-93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 097 572 B1

LIBER, STOCKHOLM 1986

## Description

La présente invention concerne de nouveaux 19-nor stéroides substitués en 11β et éventuellement en 2, leur procédé de préparation, leur application comme médicaments, les compositions les renfermant et les nouveaux intermédiaires obtenus.

La demande de brevet européen EP 0.057.115 qui a été publiée après la date de priorité de la présente demande, décrit des produits comportant des cycles A et B différents de ceux des produits de la présente demande.

Les demandes de brevets français BF 2.377.418 et 2.377.419 décrivent des produits ayant des propriétés pharmacologiques différentes de celles des produits de la présente demande. En particulier les produits de la demande BF 2.377.419 présentent une forte activité estrogène.

L'invention a pour objet les produits de formule générale (I):

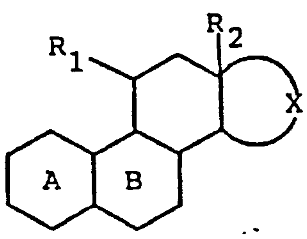

(I)

dans laquelle $R_1$ représente un radical organique renfermant de 1 à 18 atomes de carbone et éventuellement un ou plusieurs hétéroatomes, l'atome immédiatement adjacent au carbone en 11 étant un atome de carbone, $R_2$ représente un radical hydrocarboné renfermant de 1 à 8 atomes de carbone, X représente le reste d'un: cycle pentagonal ou hexagonal éventuellement substitué et éventuellement porteur d'une insaturation, les cycles A et B ayant l'une des structures suivantes:

a) - Soit A et B représentent le groupement:

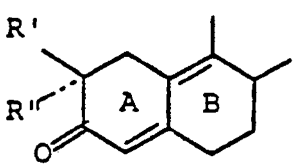

dans lequel R' et R'' identiques ou différents représentent un atome d'hydrogène, un radical nitrile ou un radical alkyle ayant de 1 à 4 atomes de carbone, étant entendu que l'un au moins des radicaux R' ou R'' ne représente pas un atome d'hydrogène; b) - Soit A et B représentent le groupement:

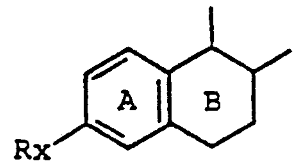

Rx représentant un atome d'hydrogène ou un groupement -ORe, dans lequel Re représente un atome d'hydrogène, un radical alkyle ayant de 1 à 6 atomes de carbone éventuellement substitué ou un radical acyle;

c) - Soit A et B représentent le groupement:

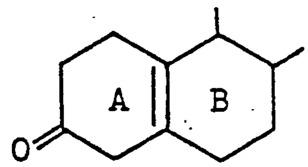

d) - Soit A et B représentent le groupement:

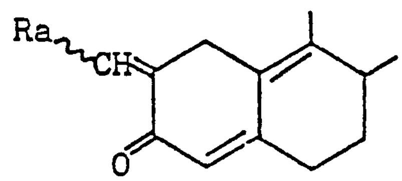

dans lequel Ra représente un radical

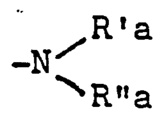

dans lequel R'a et R"a représentent soit un radical alkyle ayant de 1 à 4 atomes de carbone, soit R'a et R"a représentent avec l'atome d'azote auquel ils sont liés, un hétérocycle à 5 ou 6 chaînons comportant éventuellement un autre hétéro atome,ou Ra représente un radical acyloxy, le trait ondulé signifiant que Ra peut se trouver dans la Position E ou Z,

e) - Soit A et B représentent le groupement

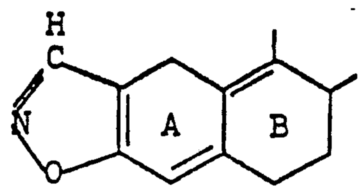

étant entendu que lorsque A et B représentent le groupement

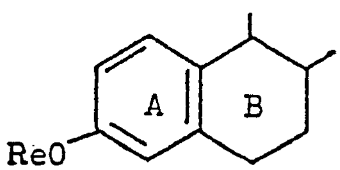

le radical $R_1$ contient au moins un atome d'azote, de phosphore ou de silicium, et que lorsque A et B représentent le groupement

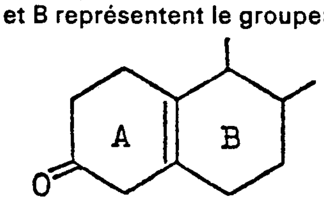

le radical $R_1$ ne représente pas un radical alkyle sature linéaire, ainsi que les sels d'addition des produits de formule I avec les acides.

Le radical $R_1$ peut représenter un radical alkyle saturé ou insaturé, linéaire ou ramifié ayant de 1 à 12 atomes de carbone.

Il s'agit alors de préférence du radical méthyle, éthyle, propyle, isopropyle, butyle, isobutyle ou tert,butyle, n- pentyle, n-hexyle, 2-méthyl pentyle, 2,3-diméthyl butyle, n-heptyle, 2-méthylhexyle, 2,2-diméthylpentyle, 3,3-diméthyl pentyle, 3-éthylpentyle, n-octyle, 2,2-diméthylhexyle, 3,3-diméthylhexyle, 3-méthyl-3-éthylpentyle, nonyle, 2,4-diméthyl heptyle, ou n-décyle. Il peut s'agir également des radicaux vinyle, isopropényle, allyle, 2-méthylallyle ou isobuténýle.

Les radicaux précités peuvent être substitués. Parmi les substituants possibles on peut citer les radicaux thioalkyle tels que thiométhyle ou thioéthyle; $R_1$ peut également être substitué par un ou plusieurs atomes d'halogènes tels que fluor, chlore, brome, iode, ou par les radicaux amino substitués tels que diméthylamino, $R_1$ peut également représenter un radical aryle ou aralcoyle. Il s'agit alors de préférence du radical phényle ou benzyle. Ces radicaux aromatiques peuvent être substitués en ortho, méta ou para par un ou plusieurs radicaux alkyles renfermant de préférence de 1 à 4 atomes de carbone; un ou plusieurs radicaux alkoxy ayant préférentiellement-de 1 à 4 atomes de carbone tels que les radicaux méthoxy, éthoxy, propyloxy, isopropyloxy, butyloxy isobutyloxy, tert-butyloxy; alkényloxy tel que vinyloxy ou allyloxy; un ou plusieurs atomes d'halogène, de préférence chlore ou fluor; par un ou plusieurs radicaux choisis parmi les radicaux hydroxyle, trifluorométhyle, alkylthio ayant de 1 à 4 atomes de carbone éventuellement oxydé sous forme de sulfoxyde ou de sulfone tel que méthylthio, éthylthio; bien entendu les radicaux aryle ou aralkyle peuvent être substitués par une combinaison de ces différents radicaux tel que par exemple 3-fluoro, 4-diméthylamino phényle;

$R_1$ peut également représenter un radical aryle hétérocyclique éventuellement substitué par les différents radicaux envisagés ci-dessus. On peut citer les radicaux thiényle, furyle, isothiényle, isofuryle, thiazolyle, isothiazolyle, oxazolyle, isoxazolyle, thiadiazolyle, pyridinyle ou pipéridinyle et les hétérocycles connus de l'homme de métier;

$R_1$ peut également représenter un radical cycloalkyle tel que cyclopropyle, cyclobutyle, cyclopentyle ou cyclohexyle, cycloalkényle tel que cyclobutényle ou cyclopropényle; $R_1$ représente également de préférence un radical comportant un noyau arylique substitué soit par une fonction amine éventuellement substituée par un ou deux radicaux alkyle ayant de 1 à 8 atomes de carbone, soit par un radical amine incorporé dans un hétérocycle comportant éventuellement un autre hétéroatome choisi dans le groupe formé par l'oxygène, l'azote et le soufre, tel que les radicaux morpholino ou pipéridinyle.

Le radical arylique est alors de préférence le noyau phényle. Comme substituant sur le noyau arylique on peut également envisager un radical amino (substitué), alkyle tel que le radical diméthylamino méthyle, diméthylamino éthyle; un radical amino (substitué) alkyloxy tel que le radical diméthylamino éthyloxy.

On peut également citer les radicaux comportant un atome de silicium tel que le radical triméthylsilyl phényle.

Les radicaux précédemment cités comportant un atome d'azote peuvent être oxydés.

De manière générale, on préfère les produits dans lesquels le substituant $R_1$ comporte un hétéroatome de préférence l'azote ou le soufre.

Le radical $R_2$ représente de préférence un radical alkyle saturé, linéaire ou ramifié, renfermant de 1 à 4 atomes de carbone, par exemple un radical'méthyle, éthyle, propyle ou butyle.

Préférentiellement $R_2$ représente un radical méthyle ou éthyle. Plus préférentiellement $R_2$ représente un radical méthyle.

Parmi les valeurs de X qui seront détaillées ultérieurement, X représenta préférentiellement le reste d'un cycle pentagonal éventuellement substitué.

Les valeurs de R' et R" peuvent être choisies parmi les valeurs alkyles précédemment citées.

Lorsque Re représente un radical alkyle substitué, il s'agit de préférence d'un radical alkyle substitué par un radical dialkylamino tel que diméthylamino, diéthylamino ou méthyléthylamino.

Le radical

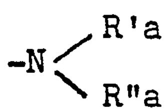

peut représenter préférentiellement un radical dialkylamino tel que diméthylamino, diéthylamino, méthyléthylamino; il peut également représenter un radical pyrrolidino, pipéridino, morpholino.

Ra peut également représenter un radical alkanoyloxy tel que acétyloxy, propionyloxy et les homologues supérieurs.

Ra peut également représenter un radical arylcarbonyloxy tel que benzoyloxy.

L'invention s'étend naturellement aux sels d'addition avec les acides des composés de formule (I) salifiables, comme par example les sels formés avec les acides chlorhydrique, bromhydrique, nitrique, sulfurique, phosphorique, acétique, formique, propionique, benzoique, maléique, fumarique, succinique, tartrique, citrique, oxalique,glyoxylique, aspartique, alcane sulfoniques tels que les acides méthane ou éthane sulfoniques, arylsulfoniques, tels que les acides benzène ou paratoluène sulfoniques et arylcarboxyliques.

La presente invention a notamment pour objet, les produits de formule I telle que définie précédemment dans laquelle X représente la reste d'un cycle de formule:

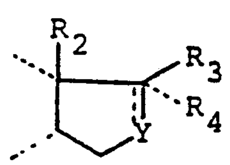

dans lequel $R_2$ conserve la même signification que précédemment le trait pointillé en 16-17 symbolise la présence éventuelle d'une double liaison, Y représente un radical

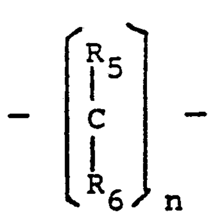

dans lequel n représente le nombre 1 ou 2, $R_5$ représente un atome d'hydrogène, un radical alkyle renfermant de 1 à 8 atomes de carbone, un radical alkényle ou alkynyle renfermant de 2 à 8 atomes de carbone, un radical aryle renfermant de 6 à 14 atomes de carbone, ou un radical aralkyle renfermant de 7 à 15 atomes de carbone, $R_6$ identique ou différent de $R_5$, peut prendre l'une des valeurs indiquées pour $R_5$ et peut également représenter un radical hydroxyle, $R_3$ et $R_4$ identiques ou différents représentent

soit un atome d'hydrogène,

soit un radical choisi dans le groupe formé par les radicaux OH, $Oalc_4$, $O\text{-}CO\text{-}alc_5$ dans lesguels $alc_4$ et $alc_5$ représentent un radical alkyle renfermant de 1 à 8 atomes de carbone ou aralkyle renfermant de 7 à 15 atomes de carbone,

soit un radical alkényle ou alkynyle renfermant de 2 à 8 atomes de carbone,

soit un radical

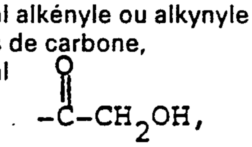

soit un radical $-COCH_2OCOalc_6$, dans lequel $alc_6$ représente un radical alkyle renfermant de 1 à 8 atomes de carbone éventuellement substitué ou un radical aralkyle renfermant de 7 à 15 atomes de carbone,

soit un radical $CO\text{-}CO_2H$,

soit un radical $CO\text{-}CO_2\text{-}alc_7$ dans lequel $alc_7$ représente un radical alkyle renfermant de 1 à 8 atomes de carbone,

soit un radical

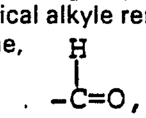

soit un radical

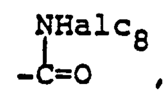

dans lequel $alc_8$ représente un radical alkyle renfermant de 1 à 8 atomes de carbone ou un radical aralkyle renfermant de 7 à 15 atomes de carbone,

soit un radical $-C\equiv N$,

soit $R_3$ et $R_4$ forment ensemble un radical

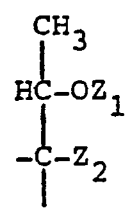

dans lequel $Z_1$ représente un atome d'hydrogène, un radical alkyle ou un radical acyle renfermant de 1 à 8 atomes de carbone et $Z_2$ un radical alkyle renfermant de 1 à 8 atomes de carbone, soit $R_3$ et $R_4$ forment ensemble un radical

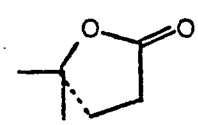

Lorsque $R_5$ ou $R_6$ représente un radical alkyle, il s'agit de préference du radical méthyle ou éthyle.

Lorsque $R_5$ ou $R_6$ représente un radical alkényle, il s'agit de préference du radical vinyle, isopropényle ou allyle.

Lorsque $R_5$ ou $R_6$ représante un radical alkynyle, il s'agit de préference du radical éthynyle ou propynyle.

Lorsque $R_5$ ou $R_6$ représente un radical aryle ou aralkyle, il s'agit de préférence du radical phényle ou benzyle.

Lorsque $R_3$ ou $R_4$ représente un radical $Oalc_4$ ou $OCOalc_5$, $alc_4$ et $alc_5$ représentent de préférence un radical méthyle, éthyle, n-propyle, butyle, pentyle, hexyle ou benzyle.

Lorsque $R_3$ ou $R_4$ représente un radical alkényle, il s'agit de préférence du radical vinyle, isopropényle, allyle ou 2-mé-thylallyle.

Lorsque $R_3$ ou $R_4$ représente un radical alkynyle, il s'agit de préférence du radical -C = CH, ou -C = C-$alc_9$, $alc_9$ représentant de préférence un radical méthyle, éthyle, propyle, isopropyle, isopropényle, butyle, benzyle ou trifluorométhyle.

$Alc_6$, $alc_7$ et $alc_8$ représentent de préférence une des valeurs préférentielles de $alc_4$ ou $alc_5$.

Les produits préférés sont ceux pour lesquels les radicaux $R_3$ et $R_4$ sont différents sauf dans le cas où $R_3$ et $R_4$ représentent chacun un atome d'hydrogène.

Parmi les valeurs préférées du radical

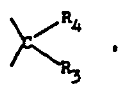 , on

peut citer les radicaux :

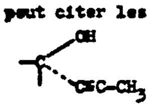 ; 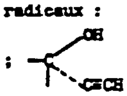 ; 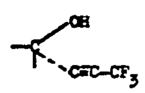 ; 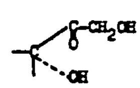

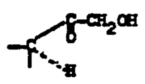 ; 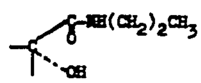 ou 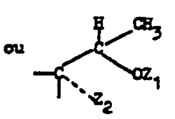 ,

dans lequel $Z_1$ représente un atome d'hydrogène, un radical alkyle renfermant de 1 à 8 atomes de carbone ou un radical acyle renfermant de 2 à 8 atomes de carbone, par exemple un radical acétyloxy ou benzoyle et $Z_2$ représente un radical alkyle renfermant de 1 à 8 atomes de carbone, par exemple un radical méthyle. Plus spécialement on préfère le radical

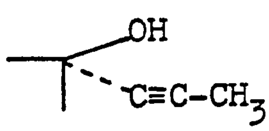

L'invention a notamment pour objet les produits de formule I telle que définie précédemment dans laquelle le cycle D ne porte pas d'insaturation, $R_5$ et $R_6$ représentent un atome d'hydrogène et n est égal à 1.

Plus particulièrement, l'invention a pour objet les produits de formule I telle que définie précédemment dans laquelle $R_3$ représente un radical OH ou $OCOalc_3$ et $R_4$ représente un radical alkényle ou alkynyle ayant au plus 4 atomes de carbone, $alc_5$ gardant la même signification que précédemment.

Parmi les produits de formule I on peut distinguer les produits dans lesquels $R_1$ représente un radical hydrocarboné renfermant de 1 à 18 atomes de carbone et contenant au moins un atome d'azote.

Parmi ces produits on peut citer tout spécialement les produits pour lesquels $R_1$ représente un radical alkyle primaire secondaire ou tertiaire, renfermant de 1 à 8 atomes de carbone, comportant un ou plusieurs hétéroatomes choisis dans le groupe constitué par l'oxygène, l'azote et la soufre, dont au moins un atome d'azote, ou substitué par un hétérocycle renfermant au moins un atome d'azote.

Par radical alkyle, on entend de préférence, les radicaux méthyle, éthyle, n-propyle, isopropyle, butyle, isobutyle, terbutyle, pentyle, hexyle, cyclopropyle, cyclobutyle, cyclopentyle ou cyclohexyle.

Par hétérocycle renfermant au moins un atome d'azote, on peut citer les radicaux 2,3- ou 4-pyridyle, les radicaux thiazolyle ou piperidinyle.

On peut également citer comme produits de l'invention, les produits pour lesquels $R_1$ représente un radical hétérocyclique comportant au moins un atome d'azote, éventuellement substitué par un radical alkyle renfermant de 1 à 8 atomes de carbone. Les radicaux hétérocycliques sont de préférence ceux cités ci-dessus. Le radical alkyle préferé est alors un radical méthyle, éthyle ou propyle.

Parmi les produits préférés de l'invention on peut citer les produits de formule I telle que définie ci-dessus dans laquelle $R_1$ représente un radical aryle ou aralkyle portant une fonction amine

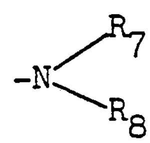

dans laquelle $R_7$ et $R_8$ représentent un radical alkyle renfermant de 1 à 8 atomes de carbone ou un radical alkyle primaire, secondaire ou tertiaire renfermant de 1 à 8 atomes de carbone, comportant un ou plusieurs hétéroatomes choisi dans le groupe constitué par l'oxygène, l'azote,le soufre et le silicium, dont au moins un atome d'azote, ou substitué par un hétérocycle comportant au moins un atome d'azote.

Les radicaux alkyle, aryle, aralkyle ou hétérocyclique sont de préférence ceux cités ci-dessus.

L'invention a tout spécialement pour objet les

produits de formule I dans laquelle $R_1$ représenta un radical 2,3 ou 4-pyridyle, un radical

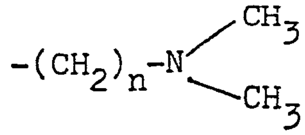

(n ≥ 3),

un radical:

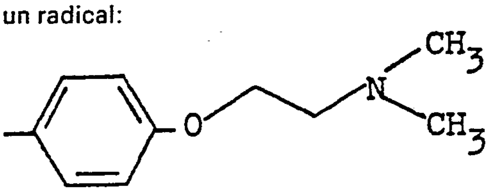

un radical

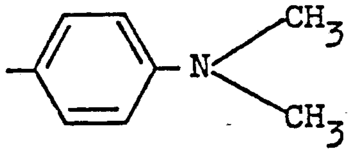

un radical

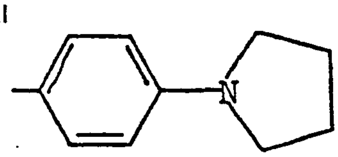

ou un radical

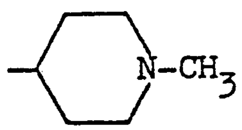

Une autre catégorie préférée de produits de l'invention est constituée par les produits de formule I dans laquelle $R_1$ représente un radical thiényle, furyle, cycloalkyle ayant de 3 à 6 atomes de carbone ou phényle éventuellement substitué par un ou plusieurs radicaux choisis parmi les radicaux hydroxy, halogène, trifluorométhyle, alkyle, alkoxy, alkylthio éventuellement oxydé sous forme de sulfoxyde ou de sulfone, étant entendu que les cycles A et B ne représentent pas le groupement:

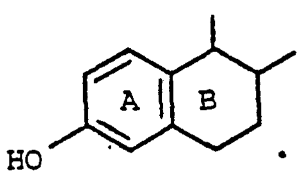

Les substituants préférés sont choisis dans les listes énoncées ci-dessus. Parmi les valeurs préférées de $R_1$ on peut alors citer le radical phényle substitué par un radical choisi dans le groupe formé par les radicaux chloro, fluoro, méthylthio, méthylsulfonyle, méthoxy, hydroxy et allyloxy.

Parmi les produits préférés figurent également les produits de formule I telle que définie précédemment dans laquelle les cycles A et B représentent le groupement

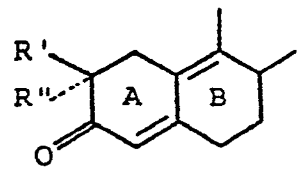

dans leguel R' et R'' sont tels que:

- soit R' et R'' identiques représentent chacun un radical méthyle ou un radical nitrile,
- soit l'un représente un atome d'hydrogène et l'autre représente un radical méthyle ou nitrile,

ainsi que les produits dans lesquels les mêmes cycles A et B représentent le groupement

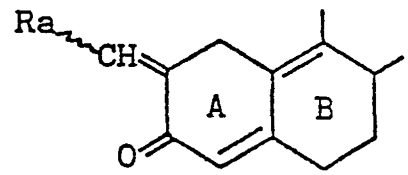

dans lequel Ra représente un radical morpholino ou un radical acétyloxy.

Parmi les produits préférés de l'invention, on peut donc naturellement citer les produits décrits ci-après dans les exemples, c'est-à-dire:

- la 2,2-dimétbyl 17β-hydroxy 11β-(4-diméthylaminphényl) 17α-(prop-1-ynyl) estra 4,9-dièn-3-one,
- la 2,2-dicyano 11β-(4-diméthylaminophényl) 17β-hydroxy 17α-(prop-1-ynyl) estra 4,9-dièn-3-one,
- la 2α-méthyl 11β -(4-diméthylaminophényl) 17β-hydroxy 17α-(prop-1-ynyl) estra 4,9-dièn-3-one,
- la 2β-méthyl 11β-(4-diméthylaminophényl) 17β-hydroxy 17α-(prop-1-ynyl) estra 4,9-dièn-3-one,
- la 2-cyano 11β-(4-diméthylaminophényl) 17β-hydroxy 17α-(prop-1-ynyl) estra 4,9-dièn-3-one,
- le 11β-(4-diméthylaminophényl) 17α-(prop-1-ynyl) estra 1,3,5(10) trièn-3 17-diol,
- le 17β-acétoxy 11β-(4-diméthylaminophényl) 17α-(prop-1-ynyl) estra 1,3,5(10 trièn-3-ol,
- le 11β -(4-diméthylaminophényl) 3-méthoxy 17α-(prop-1-ynyl) estra 1,3,5(10) trièn-17β-ol,
- le 17β-acétoxy 11β-(4-diméthylaminophényl) 3-méthoxy 17α-(prop-1-ynyl) estra 1,3,5(10) triène,
- la 17β-hydroxy IIβ-(3-méthoxyphényl) 17α-(prop-1-ynyl) estra 5(10) èn-3-one,
- la E 17β-hydroxy 11β-(3-methoxyphenyl) 17α(prop-l-enyl) estra 5(10) èn-3-one,
- la 17β-hydroxy 11β -(4-diméthylaminophényl) 17α-(prop-1-ynyl) estr 5(10) èn-3-one,
- la 2-/(acétyloxy) méthylène/11β -(4-diméthylaminophényl) 17β-hydroxy 17α-(prop-1-ynyl) estra 4,9-dièn-3-one,
- la 11β-(4-diméthylaminophényl) 17β-hydroxy

2-(4-morpholinyl-méthylène) 17α-(prop-1-ynyl) estra 4,9-dièn-3-one,

- le 11β-(4-diméthylaminophényl) 17α-(prop-1-ynyl) isoxazolo /4,5-b/ estra 4,9-dièn-17-ol,
- le 11β -/4-(2-diméthylaminoéthoxy) phényl/ estra 1,3,5(10) trièn-3,17β-diol,
- le 11β -/4-(2-diméthylaminoéthoxy) phényl/ estra 1,3,5(10) trièn-17β-ol,
- le 3-(2-diméthylamino) éthoxy IIβ,-phényl estra 1,3,5(10) trièn-17β-ol,

et leurs sels.

La présente invention a également pour objet un procédé de préparation des produits de formule générale I telle que définie ci-dessus, caractérisé en ce que:

a) pour préparer les produits de formule $I_A$

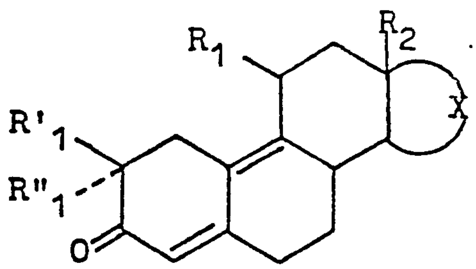

$(I_A)$

dans laquelle $R_1$, $R_2$ et X conservent la même signification que ci-dessus et $R'_1$ et $R''_1$ sont tels que, ou bien $R'_1$ et $R''_1$ représentent chacun un radical alkyle ou bien l'un représente un atome d'hydrogène et l'autre représente un radical alkyle ou bien $R'_1$ et $R''_1$ représentent chacun un radical nitrile ou bien l'un représente un radical alkyle et l'autre un radical nitrile, on soumet un produit de formule II:

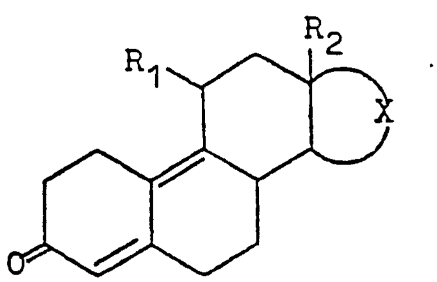

(II)

dans laquelle $R_1$, $R_2$ et X conservent la même signification que ci-dessus, d'abord éventuellement à l'action d'un réactif de protection des groupements fonctionnels puis à l'action d'une base forte et/ou bien d'un halogénure d'alkyle ou bien du cyanure de tosyle, ou bien d'abord à l'action d'un halogénure d'alkyle puis du cyanure de tosyle, puis élimine si nécessaire le groupement protecteur;

b) 1) pour préparer les produits de formule $I_B$:

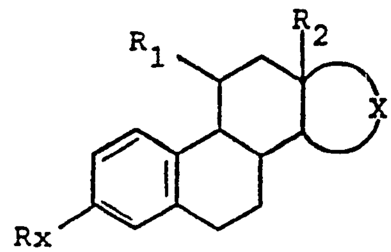

$(I_B)$

répondant à la formule $IB_1$:

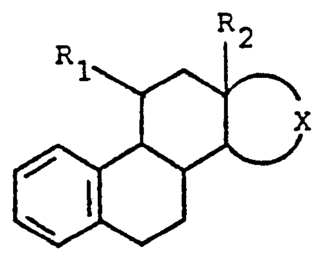

$(IB_1)$

dans laquelle $R_1$, $R_2$ et X conservent la même signification que ci-dessus, on soumet un produit de formule II à l'action d'un agent réducteur, pour obtenir un produit de formule $II_1$:

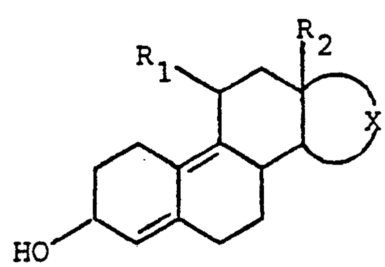

$(II_1)$

dans laquelle $R_1$, $R_2$ et X conservent la même signification que ci-dessus, que l'on soumet à l'action d'un agent d'aromatisation acide;

b) 2) pour préparer les produits de formule $I_B$ répondant à la formule $IB_2$:

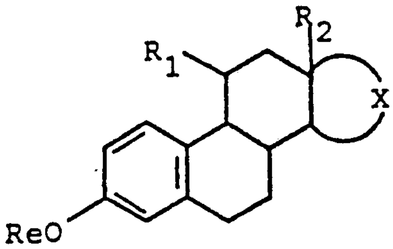

($IB_2$)

dans laquelle $R_1$, $R_2$, Re et X conservent la même signification que ci-dessus, on soumet un produit de formule II à l'action d'un agent d'aromatisation puis de saponification, puis éventuellement à un réactif d'alkylation ou d'acylation;

c) pour préparer les produits de formule $I_C$:

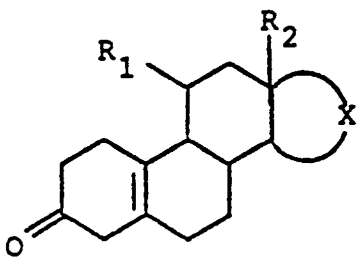

($I_C$)

dans laquelle $R_1$, $R_2$ et X conservent la même signification que ci-dessus, on soumet un produit de formule II à l'action d'un agent réducteur;

d) pour préparer les produits de formule $I_D$:

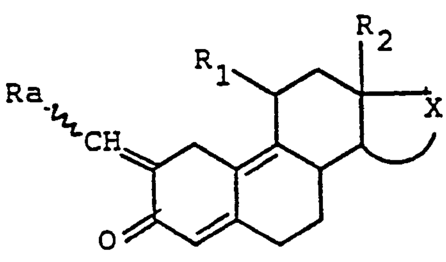

($I_D$)

dans laquelle $R_1$, $R_2$, X et Ra conservent la même signification que ci-dessus, on soumet un produit de formule II à l'action d'un réactif de formylation pour obtenir un produit de formule III:

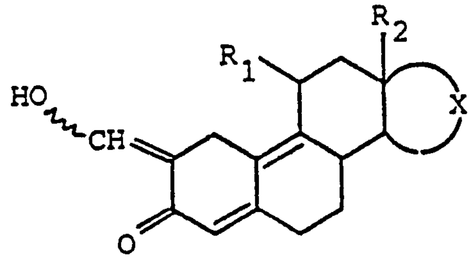

(III)

produit de formule III que, ou bien l'on fait réagir avec un réactif d'acylation, ou bien l'on fait réagir avec une amine de formule

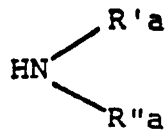

dans laquelle R'a et R"a conservent la même signification que ci-dessus;

e) pour préparer les produits de formule $I_E$:

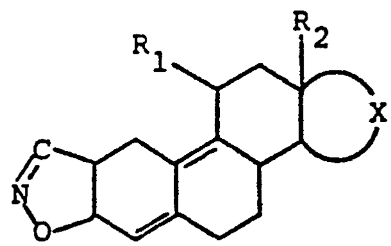

($I_E$)

dans laquelle $R_1$, $R_2$ et X conservent la même signification que ci-dessus, on fait agir l'hydroxylamine sur un produit de formule III;

f) pour préparer les produits de formule $I'_A$:

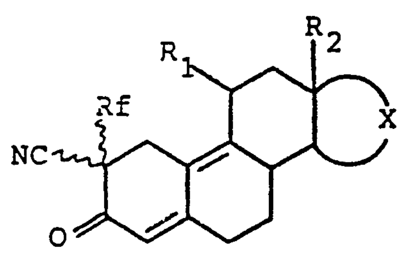

($I'_A$)

dans laquelle Rf représente un atome d'hydrogène ou un radical alkyle et $R_1$, $R_2$ et X conservent la même signification que ci- dessus, on fait agir une base sur les produits de formule $I_E$ pour obtenir les produits de formule $I'_A$ dans laquelle Rf représente un atome d'hydrogène et si désiré l'on soumet les produits ainsi obtenus à l'action d'une base forte et d'un halogénure d'alkyle pour obtenir les produits de formule $I'_A$

dans laquelle Rf représente un radical alkyle.

Lorsque le produit de formule II contient une ou plusieurs fonctions susceptibles de réagir par exemple avec l'halogénure d'alkyle utilisé, cette fonction est préférentiellement protégée à l'aide d'un groupement protecteur connu dans la littérature.

Ceci est particulièrement le cas lorsque le substituant

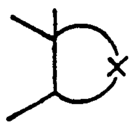

contient par exemple un radical hydroxyle en position 17β. On utilise alors de préférence la protection par le groupement tétrahydropyrannyle obtenu par action sur le produit de formule II, du dihydropyranne.

La base forte que l'on utilise préférentiellement peut être un alcoolate alcalin tel que le tert-butylate de potassium. On peut également utiliser un amidure de métal alcalin tel que l'amidure de sodium ou de lithium éventuellement préparé in situ.

L'halogénure d'alkyle que l'on utilise est de préférence l'iodure, tel que l'iodure de méthyle.

De manière générale on obtient la gem-dialkylation en position 2 lorsqu'au moins deux équivalents de base forte sont utilisés, la réaction étant effectuée en présence d'un excès d'iodure d'alkyle.

La monoalkylation est effectuée au contraire lorsqu'un équivalent de base environ est utilisé.

En général, on obtient alors un mélange d'isomères α et β qui peuvent être séparés par les techniques usuelles telles que la chromatographie.

L'addition de deux radicaux nitriles en position 2 est effectuée à l'aide de cyanure de tosyle dont la préparation est décrite dans Chem. Com. 440 (1968) et l'utilisation dans Tetrahedron Letters n° 50, 5011 (1981). L'addition d'abord d'un halogénure d'alkyle puis du cyanure de tosyle pour obtenir les produits dans lesquels l'un de $R'_1$ et $R''_1$ représente un radical alkyle et l'autre représente un radical nitrile est effectuée dans les conditions précédentes.

La déprotection éventuelle des fonctions bloquées est effectuée selon les méthodes classiques. Une hydrolyse acide peut par exemple être utilisée pour le tétrahydropyrannyle. On utilise alors l'acide chlorhydrique de préférence.

L'agent de réduction que l'on utilise pour préparer le produit de formule $II_1$ est de préférence un borohydrure alcalin tel que le borohydrure de sodium et l'on opère au sein d'un alcanol, tel que le méthanol ou l'éthanol.

L'agent d'aromatisation que l'on utilise de préférence pour préparer les produits de formule $IB_1$ est un acide minéral tel que l'acide chlorhydrique ou l'acide sulfurique, ou encore un agent tel que le pentachlorure de phosphore, le tribromure de phosphore, l'oxychlorure de phosphore, ou encore un anhydride tel que l'anhydride acétique ou trifluoroacétique.

L'agent d'aromatisation que l'on utilise de préférence pour préparer les produits de formule $IB_2$ est un halogénure d'acyle par exemple, le bromure d'acétyle ou l'anhydride acétique ou un mélange de ces produits.

L'agent de saponification utilisé est de préférence une base alcaline comme la soude ou la potasse, l'amidure de sodium, le tert-butylate de potassium ou l'acétylure de lithium dans l'éthylène diamine. La réaction a lieu, de préférence au sein d'un alcool inférieur tel que le méthanol ou l'éthanol.

Selon les conditions employées et dans le cas où par exemple le substituant

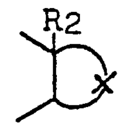

comprend une fonction réactionnelle, par exemple un radical hydroxyle en position 17β, on peut obtenir une acétylation partielle de cette fonction. On recueille alors, en plus du produit 17β-OH attendu, un pourcentage variable de produit 17β-OAc.

Ces produits 17-OH et 17-OAc peuvent être séparés par les methodes usuelles telles que la chromatographie.

On peut encore utiliser au départ un composé de formule II dans laquelle X représente le reste d'un cycle pentagonal portant un groupement cétonique en 17, lequel est ensuite réduit, par exemple par action du borohydrure de sodium.

L'alkylation éventuelle est effectuée selon les méthodes usuelles. On utilise un réactif d'alkylation qui est de préférence un halogénure d'alkyle tel que l'iodure d'alkyle ou le sulfate d'alkyle.

On utilise de préférence le sulfate de méthyle.

L'acylation est également réalisée selon les méthodes usuelles. On utilise de préférence un halogénure d'acyle.

L'agent réducteur que l'on utilise pour transformer les produits de formule II en produits de formule $I_C$ est de préférence un métal alcalin dans l'ammoniac liquide. On utilise de préférence le lithium mais le sodium peut également être envisagé.

Selon les quantités de métal employées, on peut également opérer des modifications à d'autres endroits de la molécule. Ceci peut être le cas lorsque par exemple le substituant

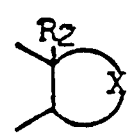

comporte une triple liaison acétylénique en 17α tel que le radical propynyle. Lorsque seulement deux atomes de métal alcalin sont employés, la

réduction 3-céto Δ4 9(10) en 3-céto Δ5 (10) est pratiquement sélective.

Si une quantité supplémentaire de réducteur est employée on peut obtenir simultanément la réduction d'une fonction acétylénique en fonction trans oléfine, par exemple un radical 17α prop-1-ynyle peut être réduite en 17α prop-1-ényle.

La séparation des deux produits obtenus peut être réalisée par les méthodes classiques telle que la chromatographie.

La formylation des produits de formule II en produits de formule III est réalisée dans les conditions connues dans la littérature. On opère en présence d'une base forte telle que l'hydrure de sodium et on utilise un formiate, de préférence un formiate d'alkyle tel que le formiate d'éthyle.

L'acylation du produit de formule III est réalisée également dans les conditions normales.

On utilise de préférence un halogénure ou un anhydride d'acide tel que le chlorure d'acétyle.

On opère alors de préférence en présence d'un capteur d'acide halohydrique, tel que la pyridine. L'action de l'amine de formule

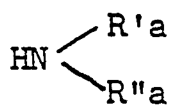

est effectuée dans les conditions usuelles, de préférence par chauffage.

L'action de l'hydroxylamine, de préférence sous forme de sel tel que le chlorhydrate sur le produit de formule III est effectuée de préférence au sein d'un alcool tel que le tert-butanol, à la température du reflux.

L'hydrolyse des produits de formule $I_E$ en produits de formule $I'_A$ est effectuée par une base telle que la soude ou la potasse, de préférence dans le méthanol. L'alkylation éventuelle des produits de formule $I'_A$ dans laquelle Rf représente un atome d'hydrogène est effectuée dans les conditions indiquées précédemment.

L'invention a plus particulièrement pour objet un procédé de préparation des produits de formule I telle que définie précédemment caractérisé en ce que l'on met en oeuvre le procédé précédent au départ de produits de formule II dans laquelle le substituant

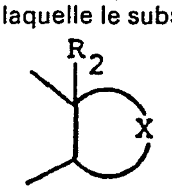

représente un radical

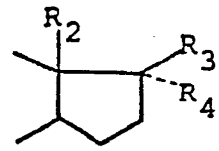

dans lequel $R_2$ représente un radical méthyle, $R_3$ représente un radical-OH ou 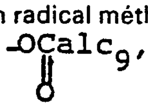

$alc_9$ étant un radical alkyle ayant de 1 à 4 atomes de carbone, $R_4$ représente un radical alkényle ou alkynyle ayant de 2 à 4 atomes de carbone et $R_1$ représente un radical phényle substitué par un radical-$OAlc_{10}$ $Alc_{10}$ étant un radical alkyle ayant de 1 à 4 atomes de carbone, ou par un radical

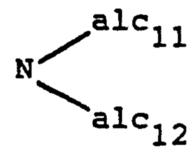,

$alc_{11}$,et $alc_{12}$ étant des radicaux alkyles ayant de 1 à 4 atomes de carbone.

Les produits de formule I ainsi que leurs sels d'addition avec les acides pharmaceutiquement acceptables sont des produits particulièrement intéressants du point de vue pharmacologique; ils possèdent en particulier une remarquable activité antiglucocorticoïde comme le montrent les résultats des tests joints en annexe.

L'étude des produits sur les récepteurs hormonaux a permis de mettre en évidence des activités progestomimétiques ou antiprogestomimétiques, androgènes ou anti-androgènes.

Les produits de formule I ainsi que leurs sels d'addition avec les acides pharmaceutiquement acceptables peuvent donc être utilisés comme médicaments pour lutter principalement contre les effets secondaires des glucocorticoïdes; ils permettent de lutter également contre les troubles dus à une hypersécrétion de glucocorticoïdes et notamment contre le vieillissement en général et plus particulièrement contre l'hypertension, l'athérosclérose, l'ostéoporose, le diabète, l'obésité ainsi que la dépression de l'immunité et l'insomnie.

Les produits de formule I ainsi que leurs sels d'addition avec les acides pharmaceutiquement acceptables, qui possèdent des propriétés antiprogestomimétiques peuvent être utilisés pour préparer des contraceptifs originaux ou comme agents d'interruption de grossesse.

Ces produits peuvent ainsi être utilisés comme inducteurs de règles chez la femme et plus généralement chez les animaux femelles à sang chaud.

Les produits sont alors administrés pendant les périodes où la progestérone joue un rôle physiologique essentiel, c'est-à-dde formule I pendant 1 à 5 jours se situant préférentiellement

à la fin du cycle. Ces produits sont administrés alors préférentiellement par la voie orale ou in vagino mais ils peuvent également être utilisés par la voie parentérale.

Les produits peuvent également être utilisés par la voie endonasale.

Ces produits de formule I possédant des propriétés antiprogestomimétiques peuvent également être utilisés contre les dérèglements hormonaux et, par ailleurs, ils peuvent présenter un intérêt dans le traitement des tumeurs hormonodépendantes.

Leurs actions sur les sécrétions hypophysaires rendent les produits utilisables dans la ménopause.

Ces produits peuvent également être utilisés dans la synchronisation de l'estrus chez les animaux d'élevage, en particulier les bovins et les ovins.

Les produits peuvent également être utilisés pour contrôler la fertilité des animaux familiers tels que chiens ou chats.

Certains produits de formule I ainsi que leurs sels d'addition avec les acides pharmaceutiquement acceptables peuvent également présenter des propriétés progestomimétiques et peuvent ainsi être utilisés dans le traitement des aménorrhées, des dysménorrhées et das insuffisances lutéales.

Les produits de formule I ainsi que leurs sels d'addition avec les acides pharmaceutiquement acceptables, qui présentent des propriétés anti-androgènes peuvent être utilisés dans le traitement des hypertrophies et du cancer de la prostate, de l'hyperandrogénie, de l'anémie, de l'hirsutisme et de l'acné.

Ils peuvent également être utilisés pour la contraception chez l'homme.

Certains produits de formule I, ainsi que leurs sels d'addition avec les acides pharmaceutiquement acceptables peuvent également présenter des propriétés estrogènes. Ils peuvent ainsi notamment être utilisés dans le traitement des troubles liés à une hypofolliculinie, par exemple les aménorrhées, les dysménorrhées, les avortements répétés, les troubles prémenstruels, ainsi que dans le traitement de la ménopause.

Certains produits de formule I, ainsi que leurs sels d'addition avec les acides pharmaceutiquement acceptables peuvent egalement présenter des propriétés anti estrogènes. Ils peuvent aussi être utilisés dans le traitement du carcinome mammaire et de ses métastases.

L'invention a donc pour objet à titre de médicament les produits de formule I pharmaceutiquement acceptables, c'est- à-dire non toxiques aux doses utilisées ainsi que leurs sels d'addition avec les acides pharmaceutiquement acceptables.

L'invention a plus spécialement pour objet à titre de médicaments, les produits suivants:

- la 2,2-diméthyl 17β-hydroxy 11β-(4-diméthylamino phényl) 17α-(prop-1-ynyl) estra 4,9-dièn-3-one,
- la 2,2-dicyano 11β-(4-diméthylamino phényl) 17β-hydroxy 17α-(prop-1-ynyl) estra 4,9-dièn-3-one,
- la 2α-méthyl 11β-(4-diméthylamino phényl) 17β-hydroxy 17α-(prop-1-ynyl) estra 4,9-dièn-3-one,
- la 2β-méthyl 11β-(4-diméthylamino phényl) 17β-hydroxy 17α-(prop-1-ynyl) estra 4,9-dièn-3-one,
- la 2-cyano 11β-(4-diméthylamino phényl) 17β-hydroxy 17α-(prop-1-ynyl) estra 4,9-dièn-3-one,
- le 11β-(4-diméthylamino phényl) 17α-(prop-1-ynyl) estra 1,3,5 (10) triène 3,17 diol,
- le 17β-acétoxy 11β-(4-diméthylamino phényl) l7α(prop-1-ynyl) estra 1,3,5 (10) trièn-3-ol,
- le 11β-(4-diméthylamino phényl) 3-méthoxy 17α-(prop-1-ynyl) estra 1,3,5 (10) trièn-17β-ol,
- le 17β-acétoxy 11β-(4-diméthylamino phényl) 3-méthoxy 17α-(prop-1-ynyl) estra 1,3,5 (10) triène,
- la 17β-hydroxy 11β-(3-méthoxy phényl) 17α-(prop-1-ynyl) estra 5(10) en-3-one,
- la (E) 17β-hydroxy 11β-(3-méthoxy phényl) 17α-(prop-1-ényl) estra 5(10) en-3-one,
- la 17β-hydroxy 11β-(4-diméthylamino phényl) 17α-(prop-1-ynyl) estr 5(10) en-3-one,
- la 2/(acétyloxy)méthylène/ 11β-(4-diméthylamino phényl) 17β hydroxy 17α-(prop-1-ynyl) estra 4,9-dièn-3-one,
- la 11β-(4-diméthylamino phényl) 17β-hydroxy 2-(4-morpholinyl-methylène) 17α-(prop-1-ynyl) estra 4,9-dièn-3-one,
- la 11β-(4-diméthylamino phényl) 17α-(prop-1-ynyl) isoxazolo /4,5-b/ estra 4,9-diéne-17-ol,
- le 11β-/4-(2-diméthylaminoéthoxy) phényl/ estra 1,3,5(10) triène 3,17β-diol,
- le 11β-/4-(2-diméthylaminoéthoxy) phényl/ estra 1,3,5(10) trien-17β-ol,
- le 3-(2-diméthylamino) éthoxy 11β-phényl estra 1,3,5(10) trien 17β-ol ainsi que leurs sels pharmaceutiquement acceptables.

La posologie utile varie en fonction de l'affection à traiter et de la voie d'administration; elle peut varier par exemple de 10 mg à 1 g par jour chez l'adulte par voie orale.

Les nouveaux produits de formule I, et leurs sels, tels que définis ci-dessus peuvent être employés pour préparer des compositions pharmaceutiques renfermant, à titre de principe actif, l'un au moins desdits produits.

Les produits de formule I et leurs sels sont utilisés par voie digestive, parentérale ou locale. Ils peuvent être prescrits sous forme de comprimés simples ou dragéifiés, de gélules, de granulés, de suppositoires, d'ovules, de preparations injectables, de pommades, de crèmes, de gels, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules

aqueux ou non, les corps gras d'origine animale ou végétale, les dérivés paraffiniques, les glycols, les divers agents mouillants, dispersants ou émulsifiants, les conservateurs.

L'invention a donc pour objet les compositions pharmaceutiques renfermant comme principe actif au moins un produit de formule I.

L'invention a également pour objet, à titre de produits industriels nouveaux et notamment à titre de produits intermédiaires nécessaires à la préparation des produits de formule I, telle que définie ci-dessus, les produits de formule générale III

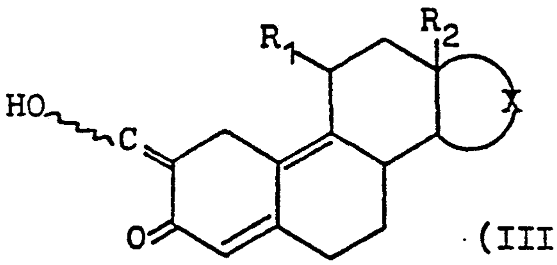

(III)

dans laquelle $R_1$, $R_2$ et X conservent la signification précédente.

Les produits de formule II utilisés au départ du procédé de la présente invention peuvent être préparés comme suit:

On fait agir un produit de formule $(R_1)_2$ Cu Li, $R_1$ Mg Hal ou $R_1$ Li sur un produit de formule A

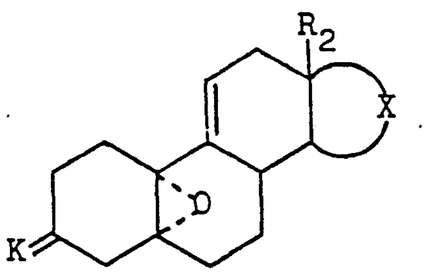

(A)

dans lequel K représente un groupement cétonique bloqué sous forme de cétal, de thiocétal, d'oxime, ou de méthyloxime pour obtenir un produit de formule B:

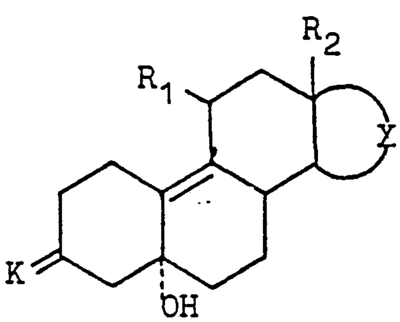

(B)

que l'on soumet à l'action d'un agent de déshydratation susceptible de libérer la ou les fonctions protégées pour obtenir un produit de formule II.

Comme agent de déshydratation on choisit de préférence une résine sulfonique à support de polystyrène ou un acide minéral tel que l'acide chlorhydrique ou sulfurique.

Les produits de formule A peuvent être préparés par action d'un oxydant tel que l'eau oxygénée en présence d'un catalyseur ou d'un peracide organique sur un produit de formule:

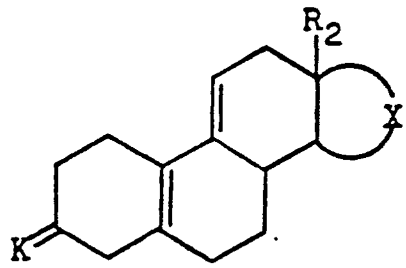

On trouvera dans la partie expérimentale ci-après des exemples de préparation de produits de départ de formule II.

En plus des exemples suivants qui illustrent l'invention sans toutefois la limiter, les produits suivants constituent des produits pouvant être obtenus dans le cadre de la présente invention.

| 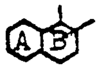 | $R_1$ | $R_2$ | $R_3$ | $R_4$ |
|---|---|---|---|---|
| 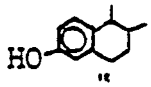 | 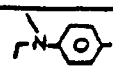 | $CH_3$ | OH | $-C{\equiv}C-H$ |
| " | " | " | " | $-C{\equiv}C-CF_3$ |
| " | " | " | " | $-C{\equiv}C-CH_2CH_3$ |
| " | " | " | " | $-CH_2-C{\equiv}C-H$ |
| " | " | " | " | $-C{\equiv}C-SiMe_3$ |
| " | " | " | $-C{\equiv}C-H$ | OH |
| " | " | " | $-C{\equiv}C-SiMe_3$ | OH |
| " | " | $CH_2CH_3$ | OH | $-C{\equiv}C-H$ |
| " | " | " | OH | $-C{\equiv}C-CH_3$ |
| " | " | " | OH | $-CH_2-C{\equiv}C-H$ |
| " | " | $CH_3$ | $-\underset{O}{\underset{\|}{C}}-CH_2OH$ | H |
| " | " | " | " | OH |
| " | 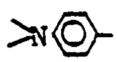 | " | OH | $-C{\equiv}C-H$ |
| " | " | " | | 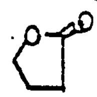 |
| " | " | " | $-C{\equiv}C-H$ | OH |
| " | " | " | OH | $-CH_2-C{\equiv}C-H$ |
| " | 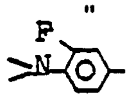 | " | OH | $-C{\equiv}C-H$ |
| " | " | " | " | $-C{\equiv}C-CH_3$ |
| " | " | " | " | $-CH_2-C{\equiv}C-H$ |
| " | " | " | $-C{\equiv}C-H$ | OH |
| " | 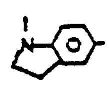 | " | OH | $-C{\equiv}C-H$ |
| " | " | " | " | $-C{\equiv}C-CF_3$ |
| " | " | " | " | $-CH_2-C{\equiv}C-H$ |
| " | " | " | " | $-C{\equiv}C-CH_2CH_3$ |
| " | " | " | " | $-C{\equiv}C-Cl$ |
| " | " | " | " | $-C{\equiv}C-SiMe_3$ |
| " | " | " | $-C{\equiv}C-H$ | OH |
| " | " | " | $-C{\equiv}C-SiMe_3$ | OH |

| 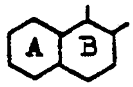 | $R_1$ | $R_2$ | $R_3$ | $R_4$ |
|---|---|---|---|---|
| 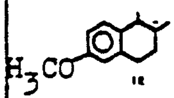 | 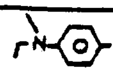 | $CH_3$ | OH | $-C≡C-H$ |
| " | " | " | " | $-C≡C-CF_3$ |
| " | " | " | " | $-C≡C-CH_2CH_3$ |
| " | " | " | " | $-CH_2-C≡C-H$ |
| " | " | " | " | $-C≡C-SiMe_3$ |
| " | " | " | $-C≡C-H$ | OH |
| " | " | " | $-C≡C-SiMe_3$ | OH |
| " | " | $CH_2CH_3$ | OH | $-C≡C-H$ |
| " | " | " | OH | $-C≡C-CH_3$ |
| " | " | " | OH | $-CH_2-C≡C-H$ |
| " | " | $CH_3$ | $-\underset{O}{\underset{\|}{C}}-CH_2OH$ | H |
| " | " | " | " | OH |
| " | 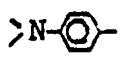 | " | OH | $-C≡C-H$ |
| " | " | " | 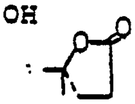 | |
| " | " | " | $-C≡C-H$ | OH |
| " | " | " | OH | $-CH_2-C≡C-H$ |
| " | 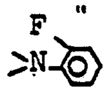 | " | OH | $-C≡C-H$ |
| " | " | " | " | $-C≡C-CH_3$ |
| " | " | " | " | $-CH_2-C≡C-H$ |
| " | " | " | $-C≡C-H$ | OH |
| " | 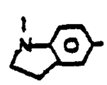 | " | OH | $-C≡C-H$ |
| " | " | " | " | $-C≡C-CF_3$ |
| " | " | " | " | $-CH_2-C≡C-H$ |
| " | " | " | " | $-C≡C-CH_2CH_3$ |
| " | " | " | " | $-C≡C-Cl$ |
| " | " | " | " | $-C≡C-SiMe_3$ |
| " | " | " | $-C≡C-H$ | OH |
| " | " | " | $-C≡C-SiMe_3$ | OH |

| 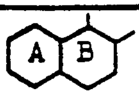 | $R_1$ | $R_2$ | $R_3$ | $R_4$ |
|---|---|---|---|---|
| 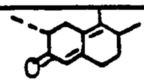 | 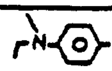 | $CH_3$ | OH | $-C\equiv C-H$ |
| " | " | " | " | $-C\equiv C-CF_3$ |
| " | " | " | " | $-C\equiv C-CH_2CH_3$ |
| " | " | " | " | $-CH_2-C\equiv C-H$ |
| " | " | " | " | $-C\equiv C-SiMe_3$ |
| " | " | " | $-C\equiv C-H$ | OH |
| " | " | " | $-C\equiv C-SiMe_3$ | OH |
| " | " | $CH_2CH_3$ | OH | $-C\equiv C-H$ |
| " | " | " | OH | $-C\equiv C-CH_3$ |
| " | " | " | OH | $-CH_2-C\equiv C-H$ |
| " | " | $CH_3$ | 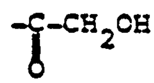 | H |
| " | " | " | " | OH |
| " | 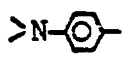 | " | OH | $-C\equiv C-H$ |
| " | " | " | 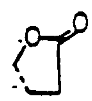 | |
| " | " | " | $-C\equiv C-H$ | OH |
| " | " | " | OH | $-CH_2-C\equiv C-H$ |
| " | 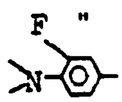 | " | OH | $-C\equiv C-H$ |
| " | " | " | " | $-C\equiv C-CH_3$ |
| " | " | " | " | $-CH_2-C\equiv C-H$ |
| " | " | " | $-C\equiv C-H$ | OH |
| " | 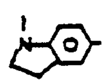 | " | OH | $-C\equiv C-H$ |
| " | " | " | " | $-C\equiv C-CF_3$ |
| " | " | " | " | $-CH_2-C\equiv C-H$ |
| " | " | " | " | $-C\equiv C-CH_2CH_3$ |
| " | " | " | " | $-C\equiv C-Cl$ |
| " | " | " | " | $-C\equiv C-SiMe_3$ |
| " | " | " | $-C\equiv C-H$ | OH |
| " | " | " | $-C\equiv C-SiMe_3$ | OH |

| 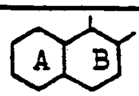 | $R_1$ | $R_2$ | $R_3$ | $R_4$ |
|---|---|---|---|---|
| 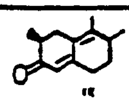 | 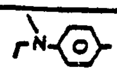 | $CH_3$ | OH | $-C\equiv C-H$ |
| " | " | " | " | $-C\equiv C-CF_3$ |
| " | " | " | " | $-C\equiv C-CH_2CH_3$ |
| " | " | " | " | $-CH_2-C\equiv C-H$ |
| " | " | " | " | $-C\equiv C-SiMe_3$ |
| " | " | " | $-C\equiv C-H$ | OH |
| " | " | " | $-C\equiv C-SiMe_3$ | OH |
| " | " | $CH_2CH_3$ | OH | $-C\equiv C-H$ |
| " | " | " | OH | $-C\equiv C-CH_3$ |
| " | " | " | OH | $-CH_2-C\equiv C-H$ |
| " | " | $CH_3$ | $-\underset{O}{\underset{\|}{C}}-CH_2OH$ | H |
| " | " | " | " | OH |
| " | 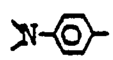 | " | OH | $-C\equiv C-H$ |
| " | " | " | 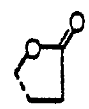 | |
| " | " | " | $-C\equiv C-H$ | OH |
| " | " | " | OH | $-CH_2-C\equiv C-H$ |
| " | 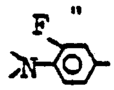 | " | OH | $-C\equiv C-H$ |
| " | " | " | " | $-C\equiv C-CH_3$ |
| " | " | " | " | $-CH_2-C\equiv C-H$ |
| " | " | " | $-C\equiv C-H$ | OH |
| " | 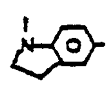 | " | OH | $-C\equiv C-H$ |
| " | " | " | " | $-C\equiv C-CF_3$ |
| " | " | " | " | $-CH_2-C\equiv C-H$ |
| " | " | " | " | $-C\equiv C-CH_2CH_3$ |
| " | " | " | " | $-C\equiv C-Cl$ |
| " | " | " | " | $-C\equiv C-SiMe_3$ |
| " | " | " | $-C\equiv C-H$ | OH |
| " | " | " | $-C\equiv C-SiMe_3$ | OH |

| 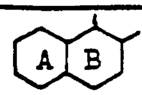 | $R_1$ | $R_2$ | $R_3$ | $R_4$ |
|---|---|---|---|---|
| 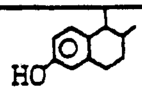 | 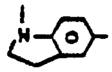 | $CH_2CH_3$ | OH | $-C≡C-H$ |
| " | " | " | " | $-C≡C-CH_3$ |
| " | " | " | " | $-CH_2-C≡C-H$ |
| " | " | $CH_3$ | $-\underset{O}{\underset{\|}{C}}-CH_2OH$ | -H |
| " | " | " | " | -OH |
| " | " | " | $-\underset{O}{\underset{\|}{C}}-CH_3$ | -H |
| 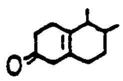 | 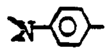 | " | -OH | $-C≡C-H$ |
| " | " | " | 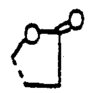 | |
| " | " | " | " | $-C≡C-CH_2CH_3$ |
| " | " | " | " | $-CH_2-C≡C-H$ |
| " | " | " | $-C≡C-H$ | -OH |
| " | " | " | $-\overset{O}{\overset{\|}{C}}-CH_2OH$ | H |
| " | 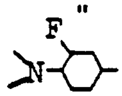 | " | " | " |
| " | " | " | OH | $-C≡C-H$ |
| " | " | " | " | $-C≡C-CH_3$ |
| " | " | " | " | $-C≡C-CH_2CH_3$ |
| " | " | " | " | $-CH_2-C≡C-H$ |
| " | " | " | $-C≡C-H$ | -OH |
| 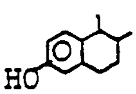 | 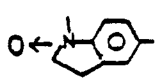 | " | " | " |
| " | " | " | OH | $-C≡C-H$ |
| " | " | " | " | $-C≡C-CF_3$ |
| " | " | " | " | $-C≡C-Cl$ |
| " | " | " | " | $-C≡C-CH_2CH_3$ |
| " | " | " | " | $-CH_2-C≡C-H$ |
| " | " | " | " | $-C≡C-SiMe_3$ |
| " | " | " | $-\underset{O}{\underset{\|}{C}}-CH_2OH$ | -H |

| 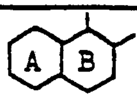 | $R_1$ | $R_2$ | $R_3$ | $R_4$ |
|---|---|---|---|---|
| 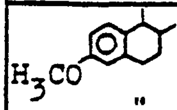 | 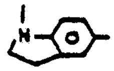 | $CH_2CH_3$ | OH | $-C{\equiv}C-H$ |
| " | " | " | " | $-C{\equiv}C-CH_3$ |
| " | " | " | " | $-CH_2-C{\equiv}C-H$ |
| " | " | $CH_3$ | $-\underset{O}{\underset{\|}{C}}-CH_2OH$ | -H |
| " | " | " | " | -OH |
| " | " | " | $-\underset{O}{\underset{\|}{C}}-CH_3$ | -H |
| 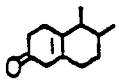 | 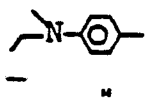 | " | -OH | $-C{\equiv}C-H$ |
| " | " | " | " | $-C{\equiv}C-CH_3$ |
| " | " | " | " | $-C{\equiv}C-CH_2CH_3$ |
| " | " | " | " | $-CH_2-C{\equiv}C-H$ |
| " | " | " | $-C{\equiv}C-H$ | -OH |
| " | " | " | $-\overset{O}{\overset{\|}{C}}-CH_2OH$ | H |
| " | " | " | 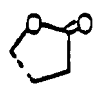 | |
| 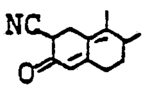 | " | " | OH | $-C{\equiv}C-H$ |
| " | " | " | " | $-C{\equiv}C-CH_3$ |
| " | " | " | " | $-C{\equiv}C-CH_2CH_3$ |
| " | " | " | " | $-CH_2-C{\equiv}C-H$ |
| " | " | " | $-C{\equiv}C-H$ | -OH |
| 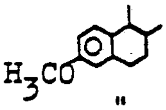 | 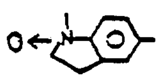 | " | " | " |
| " | " | " | OH | $-C{\equiv}C-H$ |
| " | " | " | " | $-C{\equiv}C-CF_3$ |
| " | " | " | " | $-C{\equiv}C-Cl$ |
| " | " | " | " | $-C{\equiv}C-CH_2CH_3$ |
| " | " | " | " | $-CH_2-C{\equiv}C-H$ |
| " | " | " | " | $-C{\equiv}C-SiMe_3$ |
| " | " | " | $-\underset{O}{\underset{\|}{C}}-CH_2OH$ | -H |

| 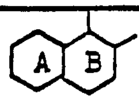 | $R_1$ | $R_2$ | $R_3$ | $R_4$ |
|---|---|---|---|---|
| 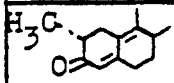 | 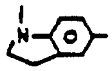 | $CH_2CH_3$ | OH | $-C\equiv C-H$ |
| " | " | " | " | $-C\equiv C-CH_3$ |
| " | " | " | " | $-CH_2-C\equiv C-H$ |
| " | " | $CH_3$ | $-\underset{\overset{\|}{O}}{C}-CH_2OH$ | -H |
| " | " | " | " | -OH |
| " | " | " | $-\underset{\overset{\|}{O}}{C}-CH_3$ | -H |
| 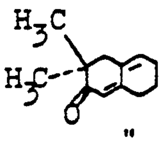 | 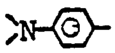 | " | -OH | $-C\equiv C-H$ |
| " | " | " | 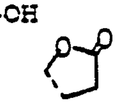 | |
| " | " | " | " | $-C\equiv C-CH_2CH_3$ |
| " | " | " | " | $-CH_2-C\equiv C-H$ |
| " | " | " | $-C\equiv C-H$ | -OH |
| " | " | " | $-\overset{\overset{O}{\|}}{C}-CH_2OH$ | H |
| " | 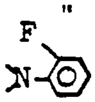 | " | " | " |
| " | " | " | OH | $-C\equiv C-H$ |
| " | " | " | " | $-C\equiv C-CH_3$ |
| " | " | " | " | $-C\equiv C-CH_2CH_3$ |
| " | " | " | " | $-CH_2-C\equiv C-H$ |
| " | " | " | $-C\equiv C-H$ | -OH |
| 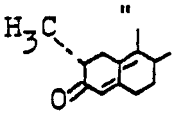 | 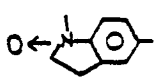 | " | " | " |
| " | " | " | OH | $-C\equiv C-H$ |
| " | " | " | " | $-C\equiv C-CF_3$ |
| " | " | " | " | $-C\equiv C-Cl$ |
| " | " | " | " | $-C\equiv C-CH_2CH_3$ |
| " | " | " | " | $-CH_2-C\equiv C-H$ |
| " | " | " | " | $-C\equiv C-SiMe_3$ |
| " | " | " | $-\underset{\overset{\|}{O}}{C}-CH_2OH$ | -H |

| 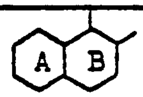 | $R_1$ | $R_2$ | $R_3$ | $R_4$ |
|---|---|---|---|---|
| 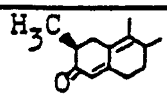 | 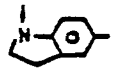 | $CH_2CH_3$ | OH | $-C{\equiv}C-H$ |
| " | " | " | " | $-C{\equiv}C-CH_3$ |
| " | " | " | " | $-CH_2-C{\equiv}C-H$ |
| " | " | $CH_3$ | $-\underset{O}{\underset{\|}{C}}-CH_2OH$ | -H |
| " | " | " | " | -OH |
| " | " | " | $-\underset{O}{\underset{\|}{C}}-CH_3$ | -H |
| 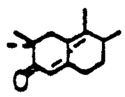 | " | " | -OH | $-C{\equiv}C-H$ |
| " | " | " | " | $-C{\equiv}C-CH_3$ |
| " | " | " | " | $-C{\equiv}C-CH_2CH_3$ |
| " | " | " | " | $-CH_2-C{\equiv}C-H$ |
| " | " | " | $-C{\equiv}C-H$ | -OH |
| " | " | " | $-\overset{O}{\overset{\|}{C}}-CH_2OH$ | H |
| " | 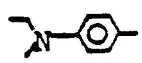 | " | " | " |
| " | " | " | OH | $-C{\equiv}C-H$ |
| " | " | " | " | $-C{\equiv}C-CH_3$ |
| " | " | " | " | $-C{\equiv}C-CH_2CH_3$ |
| " | " | " | " | $-CH_2-C{\equiv}C-H$ |
| " | " | " | $-C{\equiv}C-H$ | -OH |
| 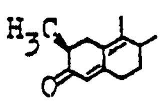 | 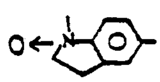 | " | " | " |
| " | " | " | OH | $-C{\equiv}C-H$ |
| " | " | " | " | $-C{\equiv}C-CF_3$ |
| " | " | " | " | $-C{\equiv}C-Cl$ |
| " | " | " | " | $-C{\equiv}C-CH_2CH_3$ |
| " | " | " | " | $-CH_2-C{\equiv}C-H$ |
| " | " | " | " | $-C{\equiv}C-SiMe_3$ |
| " | " | " | $-\underset{O}{\underset{\|}{C}}-CH_2OH$ | -H |

| 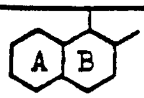 | $R_1$ | $R_2$ | $R_3$ | $R_4$ |
|---|---|---|---|---|
| 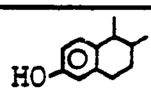 | 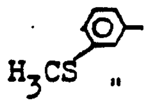 | $CH_3$ | OH | $-C≡C-H$ |
| " | " | " | " | $-C≡C-CH_3$ |
| " | " | " | " | $-CH_2-C≡C-H$ |
| " | " | " | $-C≡C-H$ | $-OH$ |
| " | 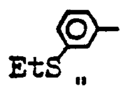 | " | " | " |
| " | " | " | OH | $-C≡C-H$ |
| " | " | " | " | $-C≡C-CH_3$ |
| " | " | " | " | $-CH_2-C≡C-H$ |
| " | 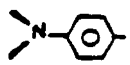 | " | " | $-C≡C-CH_2CH_3$ |
| " | " | " | " | $-C≡C-CF_3$ |
| " | " | " | $-C≡C-SiMe_3$ | $-OH$ |
| " | " | " | 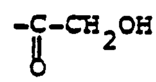 | $-H$ |
| " | " | " | " | $-OH$ |
| " | " | " | 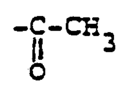 | $-H$ |
| " | " | $-CH_2CH_3$ | OH | $-C≡C-H$ |
| " | " | " | " | $-C≡C-CH_3$ |
| " | " | " | " | $-C≡C-Cl$ |
| " | " | " | " | $-C≡C-CH_2-CH_3$ |
| " | " | " | " | $-C≡C-SiMe_3$ |
| " | " | " | " | $-CH_2-C≡C-H$ |
| " | " | " | 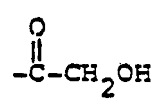 | $-H$ |
| " | 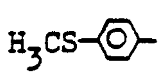 | $CH_3$ | $-C≡C-H$ | $-OH$ |
| " | " | " | 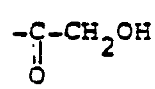 | $-H$ |
| " | " | " | OH | $-CH_2-C≡C-H$ |
| " | 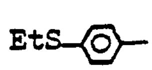 | " | " | " |

| 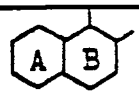 | $R_1$ | $R_2$ | $R_3$ | $R_4$ |
|---|---|---|---|---|
| 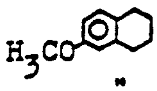 | 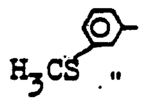 | $CH_3$ | OH | $-C\equiv C-H$ |
| " | " | " | " | $-C\equiv C-CH_3$ |
| " | " | " | " | $-CH_2-C\equiv C-H$ |
| " | " | " | $-C\equiv C-H$ | $-OH$ |
| " | 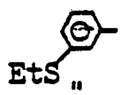 | " | " | " |
| " | " | " | OH | $-C\equiv C-H$ |
| " | " | " | " | $-C\equiv C-CH_3$ |
| " | " | " | " | $-CH_2-C\equiv C-H$ |
| " | 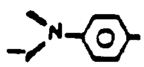 | " | " | $-C\equiv C-CH_2CH_3$ |
| " | " | " | " | $-C\equiv C-CF_3$ |
| " | " | " | $-C\equiv C-SiMe_3$ | $-OH$ |
| " | " | " | 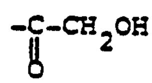 | $-H$ |
| " | " | " | " | $-OH$ |
| " | " | " | 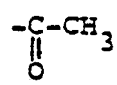 | $-H$ |
| " | " | $-CH_2CH_3$ | OH | $-C\equiv C-H$ |
| " | " | " | " | $-C\equiv C-CH_3$ |
| " | " | " | " | $-C\equiv C-Cl$ |
| " | " | " | " | $-C\equiv C-CH_2-CH_3$ |
| " | " | " | " | $-C\equiv C-SiMe_3$ |
| " | " | " | " | $-CH_2-C\equiv C-H$ |
| " | " | " | 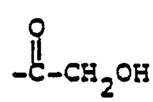 | $-H$ |
| " | 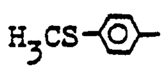 | $CH_3$ | $-C\equiv C-H$ | $-OH$ |
| " | " | " | 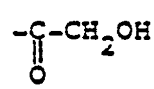 | $-H$ |
| " | " | " | OH | $-CH_2-C\equiv C-H$ |
| " | 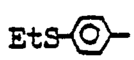 | " | " | " |

| 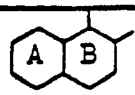 | $R_1$ | $R_2$ | $R_3$ | $R_4$ |
|---|---|---|---|---|
| 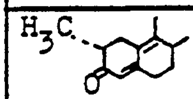 | 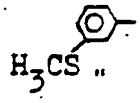 | $CH_3$ | OH | $-C\equiv C-H$ |
| " | " | " | " | $-C\equiv C-CH_3$ |
| " | " | " | " | $-CH_2-C\equiv C-H$ |
| " | " | " | $-C\equiv C-H$ | -OH |
| " | 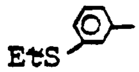 | " | " | " |
| " | " | " | OH | $-C\equiv C-H$ |
| " | " | " | " | $-C\equiv C-CH_3$ |
| " | " | " | " | $-CH_2-C\equiv C-H$ |
| " | 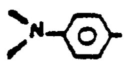 | " | " | $-C\equiv C-CH_2CH_3$ |
| " | " | " | " | $-C\equiv C-CF_3$ |
| " | " | " | $-C\equiv C-SiMe_3$ | -OH |
| " | " | " | 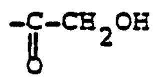 | -H |
| " | " | " | " | -OH |
| " | " | " | 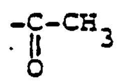 | -H |
| " | " | $-CH_2CH_3$ | OH | $-C\equiv C-H$ |
| " | " | " | " | $-C\equiv C-CH_3$ |
| " | " | " | " | $-C\equiv C-Cl$ |
| " | " | " | " | $-C\equiv C-CH_2-CH_3$ |
| " | " | " | " | $-C\equiv C-SiMe_3$ |
| " | " | " | " | $-CH_2-C\equiv C-H$ |
| " | " | " | 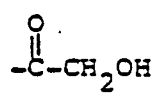 | -H |
| " | 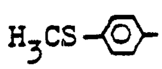 | $CH_3$ | $-C\equiv C-H$ | -OH |
| " | " | " | 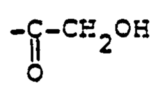 | -H |
| " | " | " | OH | $-CH_2-C\equiv C-H$ |
| " | 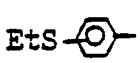 | " | " | " |

| 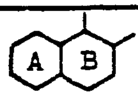 | $R_1$ | $R_2$ | $R_3$ | $R_4$ |
|---|---|---|---|---|
| 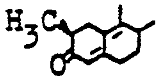 | 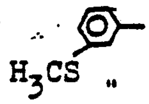 | $CH_3$ | OH | $-C\equiv C-H$ |
| " | " | " | " | $-C\equiv C-CH_3$ |
| " | " | " | " | $-CH_2-C\equiv C-H$ |
| " | " | " | $-C\equiv C-H$ | $-OH$ |
| " | 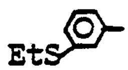 | " | " | " |
| " | " | " | OH | $-C\equiv C-H$ |
| " | " | " | " | $-C\equiv C-CH_3$ |
| " | " | " | " | $-CH_2-C\equiv C-H$ |
| " | 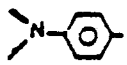 | " | " | $-C\equiv C-CH_2CH_3$ |
| " | " | " | " | $-C\equiv C-CF_3$ |
| " | " | " | $-C\equiv C-SiMe_3$ | $-OH$ |
| " | " | " | $-\underset{\underset{O}{\Vert}}{C}-CH_2OH$ | $-H$ |
| " | " | " | " | $-OH$ |
| " | " | " | $-\underset{\underset{O}{\Vert}}{C}-CH_3$ | $-H$ |
| " | " | $-CH_2CH_3$ | OH | $-C\equiv C-H$ |
| " | " | " | " | $-C\equiv C-CH_3$ |
| " | " | " | " | $-C\equiv C-Cl$ |
| " | " | " | " | $-C\equiv C-CH_2-CH_3$ |
| " | " | " | " | $-C\equiv C-SiMe_3$ |
| " | " | " | " | $-CH_2-C\equiv C-H$ |
| " | " | " | $-\overset{\overset{O}{\Vert}}{C}-CH_2OH$ | $-H$ |
| " | 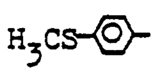 | $CH_3$ | $-C\equiv C-H$ | $-OH$ |
| " | " | " | $-\underset{\underset{O}{\Vert}}{C}-CH_2OH$ | $-H$ |
| " | " | " | OH | $-CH_2-C\equiv C-H$ |
| " | 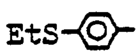 | " | " | " |

| 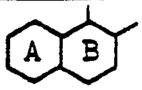 | $R_1$ | $R_2$ | $R_3$ | $R_4$ |
|---|---|---|---|---|
| 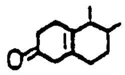 | 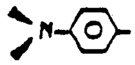 | $CH_3$ | $-C{\equiv}C-H$ | $-OH$ |
| " | " | " | 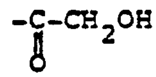 | $-H$ |
| 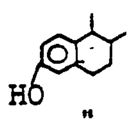 | 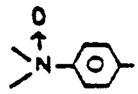 | " | " | " |
| " | " | " | $-C{\equiv}C-H$ | $-OH$ |
| " | " | " | OH | $-CH_2-C{\equiv}C-H$ |
| " | " | " | " | $-C{\equiv}C-CH_2CH_3$ |
| " | " | " | " | $-C{\equiv}C-CF_3$ |
| " | " | " | " | $-C{\equiv}C-H$ |
| " | " | " | " | $-C{\equiv}C-SiMe_3$ |
| 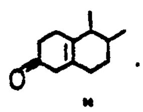 | " | " | OH | $-C{\equiv}C-H$ |
| " | " | " | " | $-C{\equiv}C-CH_3$ |
| " | " | " | " | $-C{\equiv}C-CH_2CH_3$ |
| " | " | " | " | $-C{\equiv}C-Cl$ |
| " | " | " | " | $-C{\equiv}C-SiMe_3$ |
| " | " | " | " | $-CH_2-C{\equiv}C-H$ |
| " | " | " | $-C{\equiv}C-H$ | $-OH$ |
| 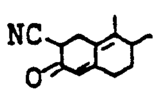 | " | " | " | " |
| " | " | " | OH | $-C{\equiv}C-H$ |
| " | " | " | " | $-C{\equiv}C-CH_3$ |
| " | " | " | " | $-C{\equiv}C-CH_2-CH_3$ |
| " | " | " | " | $-C{\equiv}C-Cl$ |
| " | " | " | " | $-C{\equiv}C-SiMe_3$ |
| " | " | " | " | $-CH_2-C{\equiv}C-H$ |
| 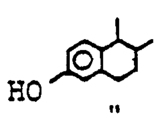 | 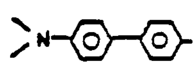 | " | OH | $-C{\equiv}C-H$ |
| " | " | " | " | $-C{\equiv}C-CF_3$ |
| " | " | " | " | $-C{\equiv}C-CH_3$ |
| " | " | " | " | $-C{\equiv}C-Cl$ |

| 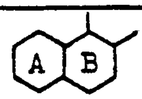 | $R_1$ | $R_2$ | $R_3$ | $R_4$ |
|---|---|---|---|---|
| 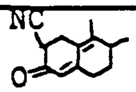 | 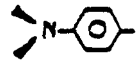 | $CH_3$ | $-C \equiv C-H$ | $-OH$ |
| " | " | " | $-\underset{\overset{\|}{O}}{C}-CH_2OH$ | $-H$ |
| 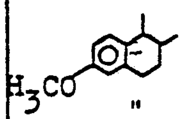 | 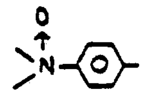 | " | " | " |
| " | " | " | $-C \equiv C-H$ | $-OH$ |
| " | " | " | OH | $-CH_2-C \equiv C-H$ |
| " | " | " | " | $-C \equiv C-CH_2CH_3$ |
| " | " | " | " | $-C \equiv C-CF_3$ |
| " | " | " | " | $-C \equiv C-H$ |
| " | " | " | " | $-C \equiv C-SiMe_3$ |
| 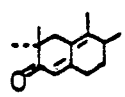 | " | " | OH | $-C \equiv C-H$ |
| " | " | " | " | $-C \equiv C-CH_3$ |
| " | " | " | " | $-C \equiv C-CH_2CH_3$ |
| " | " | " | " | $-C \equiv C-Cl$ |
| " | " | " | " | $-C \equiv C-SiMe_3$ |
| " | " | " | " | $-CH_2-C \equiv C-H$ |
| " | " | " | $-C \equiv C-H$ | $-OH$ |
| 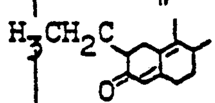 | " | " | " | " |
| " | " | " | OH | $-C \equiv C-H$ |
| " | " | " | " | $-C \equiv C-CH_3$ |
| " | " | " | " | $-C \equiv C-CH_2-CH_3$ |
| " | " | " | " | $-C \equiv C-Cl$ |
| " | " | " | " | $-C \equiv C-SiMe_3$ |
| " | " | " | " | $-CH_2-C \equiv C-H$ |
| 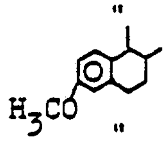 | 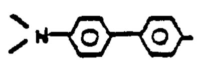 | " | OH | $-C \equiv C-H$ |
| " | " | " | " | $-C \equiv C-CF_3$ |
| " | " | " | " | $-C \equiv C-CH_3$ |
| " | " | " | " | $-C \equiv C-Cl$ |

| 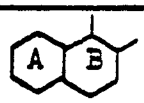 | $R_1$ | $R_2$ | $R_3$ | $R_4$ |
|---|---|---|---|---|
| 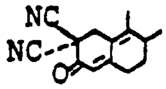 | 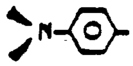 | $CH_3$ | $-C\equiv C-H$ | $-OH$ |
| " | " | " | $-\underset{\overset{\|}{O}}{C}-CH_2OH$ | $-H$ |
| 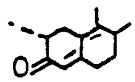 | 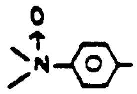 | " | " | " |
| " | " | " | $-C\equiv C-H$ | $-OH$ |
| " | " | " | OH | $-CH_2-C\equiv C-H$ |
| " | " | " | " | $-C\equiv C-CH_2CH_3$ |
| " | " | " | " | $-C\equiv C-CF_3$ |
| " | " | " | " | $-C\equiv C-H$ |
| " | " | " | " | $-C\equiv C-SiMe_3$ |
| 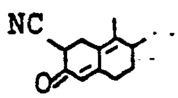 | " | " | OH | $-C\equiv C-H$ |
| " | " | " | " | $-C\equiv C-CH_3$ |
| " | " | " | " | $-C\equiv C-CH_2CH_3$ |
| " | " | " | " | $-C\equiv C-Cl$ |
| " | " | " | " | $-C\equiv C-SiMe_3$ |
| " | " | " | " | $-CH_2-C\equiv C-H$ |
| " | " | " | $-C\equiv C-H$ | $-OH$ |
| 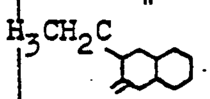 | " | " | " | " |
| " | " | " | OH | $-C\equiv C-H$ |
| " | " | " | " | $-C\equiv C-CH_3$ |
| " | " | " | " | $-C\equiv C-CH_2-CH_3$ |
| " | " | " | " | $-C\equiv C-Cl$ |
| " | " | " | " | $-C\equiv C-SiMe_3$ |
| " | " | " | " | $-CH_2-C\equiv C-H$ |
| 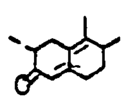 | 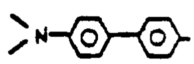 | " | OH | $-C\equiv C-H$ |
| " | " | " | " | $-C\equiv C-CF_3$ |
| " | " | " | " | $-C\equiv C-CH_3$ |
| " | " | " | " | $-C\equiv C-Cl$ |

| 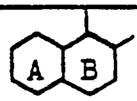 | $R_1$ | $R_2$ | $R_3$ | $R_4$ |
|---|---|---|---|---|
| 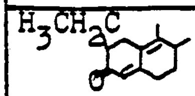 | 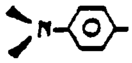 | $CH_3$ | $-C{\equiv}C-H$ | $-OH$ |
| " | " | " | $-\underset{\overset{\Vert}{O}}{C}-CH_2OH$ | $-H$ |
| 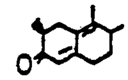 | 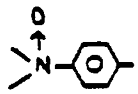 | " | " | " |
| " | " | " | $-C{\equiv}C-H$ | $-OH$ |
| " | " | " | OH | $-CH_2-C{\equiv}C-H$ |
| " | " | " | " | $-C{\equiv}C-CH_2CH_3$ |
| " | " | " | " | $-C{\equiv}C-CF_3$ |
| " | " | " | " | $-C{\equiv}C-H$ |
| " | " | " | " | $-C{\equiv}C-SiMe_3$ |
| 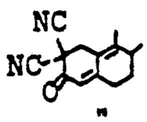 | " | " | OH | $-C{\equiv}C-H$ |
| " | " | " | " | $-C{\equiv}C-CH_3$ |
| " | " | " | " | $-C{\equiv}C-CH_2CH_3$ |
| " | " | " | " | $-C{\equiv}C-Cl$ |
| " | " | " | " | $-C{\equiv}C-SiMe_3$ |
| " | " | " | " | $-CH_2-C{\equiv}C-H$ |
| " | " | " | $-C{\equiv}C-H$ | $-OH$ |
| 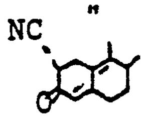 | " | " | " | " |
| " | " | " | OH | $-C{\equiv}C-H$ |
| " | " | " | " | $-C{\equiv}C-CH_3$ |
| " | " | " | " | $-C{\equiv}C-CH_2-CH_3$ |
| " | " | " | " | $-C{\equiv}C-Cl$ |
| " | " | " | " | $-C{\equiv}C-SiMe_3$ |
| " | " | " | " | $-CH_2-C{\equiv}C-H$ |
| 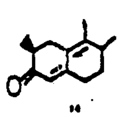 | 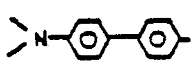 | " | OH | $-C{\equiv}C-H$ |
| " | " | " | " | $-C{\equiv}C-CF_3$ |
| " | " | " | " | $-C{\equiv}C-CH_3$ |
| " | " | " | " | $-C{\equiv}C-Cl$ |

| 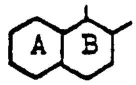 | $R_1$ | $R_2$ | $R_3$ | $R_4$ |
|---|---|---|---|---|
| 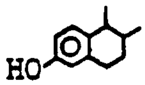 | 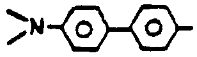 | $CH_3$ | OH | $-CH_2-C≡C-H$ |
| " | " | " | " | -H |
| " | " | " | -C≡C-H | -OH |
| " | " | " | 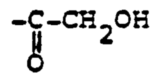 | -H |
| " | 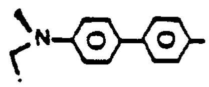 | " | " | " |
| " | " | " | OH | -C≡C-H |
| " | " | " | " | $-C≡C-CH_3$ |
| " | " | " | " | -C≡C-Cl |
| " | " | " | " | $-CH_2-C≡C-H$ |
| " | " | " | -C≡C-H | -OH |
| " | 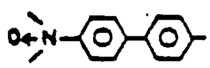 | " | " | " |
| " | " | " | OH | -C≡C-H |
| " | " | " | " | $-C≡C-CF_3$ |
| " | " | " | " | $-C≡C-CH_3$ |
| " | " | " | " | $-CH_2-C≡C-H$ |
| " | " | " | " | -H |
| " | 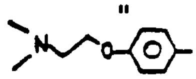 | " | " | -C≡C-H |
| " | " | " | " | $-CH_2-C≡C-H$ |
| " | " | " | -C≡C-H | -OH |
| " | " | " | 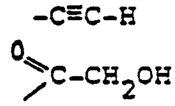 | -H |
| " | 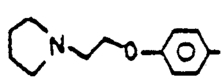 | " | " | " |
| " | " | " | -C≡C-H | -OH |
| " | " | " | -OH | -C≡C-H |
| " | " | " | " | $-C≡C-CH_3$ |
| " | " | " | " | $-CH_2-C≡C-H$ |
| " | 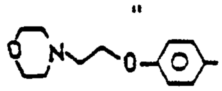 | " | -OH | -C≡C-H |
| " | " | " | " | $-C≡C-CH_3$ |

| 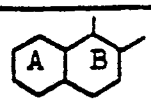 | $R_1$ | $R_2$ | $R_3$ | $R_4$ |
|---|---|---|---|---|
| 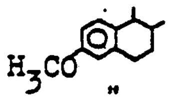 | 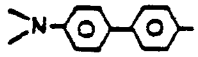 | $CH_3$ | OH | $-CH_2-C\equiv C-H$ |
| " | " | " | " | -H |
| " | " | " | $-C\equiv C-H$ | -OH |
| " | " | " | 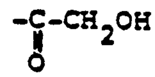 | -H |
| " | 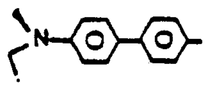 | " | " | " |
| " | " | " | OH | $-C\equiv C-H$ |
| " | " | " | " | $-C\equiv C-CH_3$ |
| " | " | " | " | $-C\equiv C-Cl$ |
| " | " | " | " | $-CH_2-C\equiv C-H$ |
| " | " | " | $-C\equiv C-H$ | -OH |
| " | 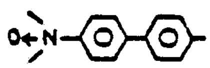 | " | " | " |
| " | " | " | OH | $-C\equiv C-H$ |
| " | " | " | " | $-C\equiv C-CF_3$ |
| " | " | " | " | $-C\equiv C-CH_3$ |
| " | " | " | " | $-CH_2-C\equiv C-H$ |
| " | " | " | " | -H |
| " | 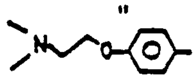 | " | " | $-C\equiv C-H$ |
| " | " | " | " | $-CH_2-C\equiv C-H$ |
| " | " | " | $-C\equiv C-H$ | -OH |
| " | " | " | 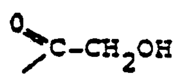 | -H |
| " | 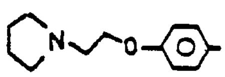 | " | " | " |
| " | " | " | $-C\equiv C-H$ | -OH |
| " | " | " | -OH | $-C\equiv C-H$ |
| " | " | " | " | $-C\equiv C-CH_3$ |
| " | " | " | " | $-CH_2-C\equiv C-H$ |
| " | 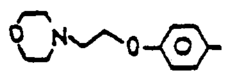 | " | -OH | $-C\equiv C-H$ |
| " | " | " | " | $-C\equiv C-CH_3$ |

| 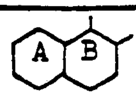 | $R_1$ | $R_2$ | $R_3$ | $R_4$ |
|---|---|---|---|---|
| 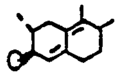 | 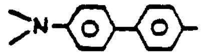 | $CH_3$ | OH | $-CH_2-C\equiv C-H$ |
| " | " | " | " | -H |
| " | " | " | $-C\equiv C-H$ | -OH |
| " | " | " | 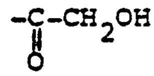 | -H |
| " | 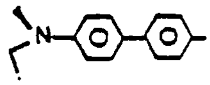 | " | " | " |
| " | " | " | OH | $-C\equiv C-H$ |
| " | " | " | " | $-C\equiv C-CH_3$ |
| " | " | " | " | $-C\equiv C-Cl$ |
| " | " | " | " | $-CH_2-C\equiv C-H$ |
| " | " | " | $-C\equiv C-H$ | -OH |
| " | 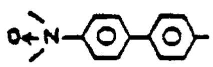 | " | " | " |
| " | " | " | OH | $-C\equiv C-H$ |
| " | " | " | " | $-C\equiv C-CF_3$ |
| " | " | " | " | $-C\equiv C-CH_3$ |
| " | " | " | " | $-CH_2-C\equiv C-H$ |
| " | " | " | " | -H |
| " | 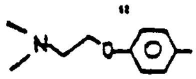 | " | " | $-C\equiv C-H$ |
| " | " | " | " | $-CH_2-C\equiv C-H$ |
| " | " | " | $-C\equiv C-H$ | -OH |
| " | " | " | 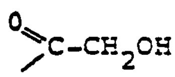 | -H |
| " | 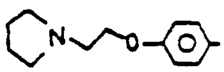 | " | " | " |
| " | " | " | $-C\equiv C-H$ | -OH |
| " | " | " | -OH | $-C\equiv C-H$ |
| " | " | " | " | $-C\equiv C-CH_3$ |
| " | " | " | " | $-CH_2-C\equiv C-H$ |
| " | 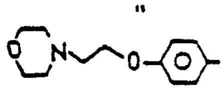 | " | -OH | $-C\equiv C-H$ |
| " | " | " | " | $-C\equiv C-CH_3$ |

| 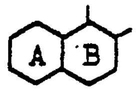 | $R_1$ | $R_2$ | $R_3$ | $R_4$ |
|---|---|---|---|---|
| 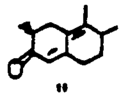 | 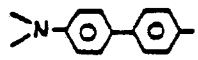 | $CH_3$ | OH | $-CH_2-C\equiv C-H$ |
| " | " | " | " | -H |
| " | " | " | $-C\equiv C-H$ | -OH |
| " | " | " | 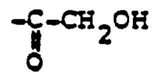 | -H |
| " | 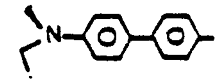 | " | " | " |
| " | " | " | OH | $-C\equiv C-H$ |
| " | " | " | " | $-C\equiv C-CH_3$ |
| " | " | " | " | $-C\equiv C-Cl$ |
| " | " | " | " | $-CH_2-C\equiv C-H$ |
| " | " | " | $-C\equiv C-H$ | -OH |
| " | 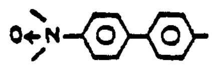 | " | " | " |
| " | " | " | OH | $-C\equiv C-H$ |
| " | " | " | " | $-C\equiv C-CF_3$ |
| " | " | " | " | $-C\equiv C-CH_3$ |
| " | " | " | " | $-CH_2-C\equiv C-H$ |
| " | " | " | " | -H |
| " | 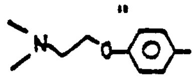 | " | " | $-C\equiv C-H$ |
| " | " | " | " | $-CH_2-C\equiv C-H$ |
| " | " | " | $-C\equiv C-H$ | -OH |
| " | " | " | 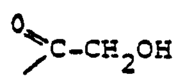 | -H |
| " | 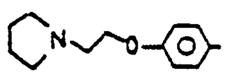 | " | " | " |
| " | " | " | $-C\equiv C-H$ | -OH |
| " | " | " | -OH | $-C\equiv C-H$ |
| " | " | " | " | $-C\equiv C-CH_3$ |
| " | " | " | " | $-CH_2-C\equiv C-H$ |
| " | 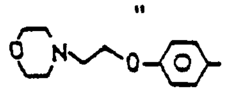 | " | -OH | $-C\equiv C-H$ |
| " | " | " | " | $-C\equiv C-CH_3$ |

| 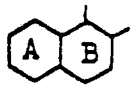 | $R_1$ | $R_2$ | $R_3$ | $R_4$ |
|---|---|---|---|---|
| 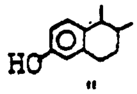 | 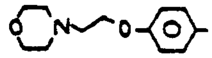 | $CH_3$ | -OH | $-CH_2-C≡C-H$ |
| " | " | " | -C≡C-H | -OH |
| " | " | " | 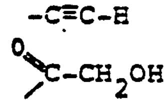 | -H |
| " | 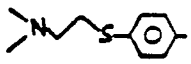 | " | " | " |
| " | " | " | -C≡C-H | OH |
| " | " | " | OH | $-C≡C-CF_3$ |
| " | " | " | OH | -C≡C-H |
| " | " | " | " | $-CH_2-CH=CH_2$ |
| " | " | " | " | $CH_2-C≡C-H$ |
| " | " | " | " | $-CH_2$ |
| " | " | " | 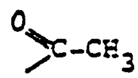 | $-CH_3$ |
| " | " | " | OH | $-CH_2-CN$ |
| 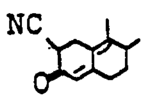 | " | " | OH | -C≡C-H |
| " | " | " | " | $-C≡C-CH_3$ |
| " | " | " | " | $-C≡C-CH_2CH_3$ |
| " | " | " | " | -C≡C-Cl |
| " | " | " | " | $-CH_2-C≡C-H$ |
| " | " | " | -C≡C-H | -OH |
| " | " | " | 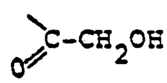 | -H |
| 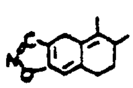 | 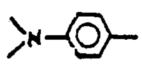 | " | " | " |
| " | " | " | -C≡C-H | -OH |
| " | " | " | OH | -C≡C-H |
| " | " | " | " | $-C≡C-CH_3$ |
| " | " | " | " | $-C≡C-CH_2-CH_3$ |
| " | " | " | " | -C≡C-Cl |
| " | " | " | " | $-CH_2-C≡C-H$ |
|  | " | $CH_2CH_3$ | " | -C≡C-H |
| " | " | " | " | $-C≡C-CH_3$ |

| 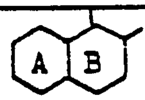 | $R_1$ | $R_2$ | $R_3$ | $R_4$ |
|---|---|---|---|---|
| 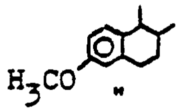 | 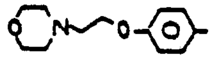 | $CH_3$ | $-OH$ | $-CH_2-C\equiv C-H$ |
| " | " | " | $-C\equiv C-H$ | $-OH$ |
| " | " | " | 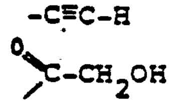 | $-H$ |
| " | 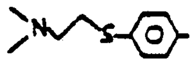 | " | " | " |
| " | " | " | $-C\equiv C-H$ | OH |
| " | " | " | OH | $-C\equiv C-CF_3$ |
| " | " | " | OH | $-C\equiv C-H$ |
| " | " | " | " | $-CH_2-CH=CH_2$ |
| " | " | " | " | $CH_2-C\equiv C-H$ |
| " | " | " | " | $-CH_2$ |
| " | " | " | 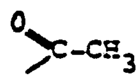 | $-CH_3$ |
| " | " | " | OH | $-CH_2-CN$ |
| 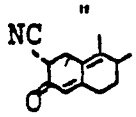 | " | " | OH | $-C\equiv C-H$ |
| " | " | " | " | $-C\equiv C-CH_3$ |
| " | " | " | " | $-C\equiv C-CH_2CH_3$ |
| " | " | " | " | $-C\equiv C-Cl$ |
| " | " | " | " | $-CH_2-C\equiv C-H$ |
| " | " | " | $-C\equiv C-H$ | $-OH$ |
| " | " | " | 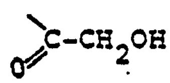 | $-H$ |
| 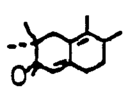 | " | " | " | " |
| " | " | " | $-C\equiv C-H$ | $-OH$ |
| " | " | " | OH | $-C\equiv C-H$ |
| " | " | " | " | $-C\equiv C-CH_3$ |
| " | " | " | " | $-C\equiv C-CH_2-CH_3$ |
| " | " | " | " | $-C\equiv C-Cl$ |
| " | " | " | " | $-CH_2-C\equiv C-H$ |
| | " | $CH_2CH_3$ | " | $-C\equiv C-H$ |
| " | " | " | " | $-C\equiv C-CH_3$ |

| 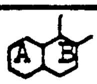 | $R_1$ | $R_2$ | $R_3$ | $R_4$ |
|---|---|---|---|---|
| 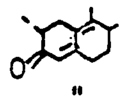 | 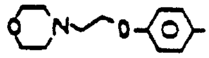 | $CH_3$ | -OH | $-CH_2-C\equiv C-H$ |
| " | " | " | $-C\equiv C-H$ | -OH |
| " | " | " | 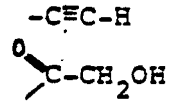 | -H |
| " | 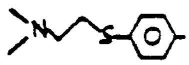 | " | " | " |
| " | " | " | $-C\equiv C-H$ | OH |
| " | " | " | OH | $-C\equiv C-CF_3$ |
| " | " | " | OH | $-C\equiv C-H$ |
| " | " | " | " | $-CH_2-CH=CH_2$ |
| " | " | " | " | $CH_2-C\equiv C-H$ |
| " | " | " | " | $-CH_2$ |
| " | " | " | 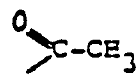 | $-CH_3$ |
| " | " | " | OH | $-CH_2-CN$ |
| 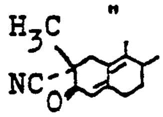 | " | " | OH | $-C\equiv C-H$ |
| " | " | " | " | $-C\equiv C-CH_3$ |
| " | " | " | " | $-C\equiv C-CH_2CH_3$ |
| " | " | " | " | $-C\equiv C-Cl$ |
| " | " | " | " | $-CH_2-C\equiv C-H$ |
| " | " | " | $-C\equiv C-H$ | -OH |
| " | " | " | 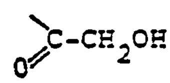 | -H |
| 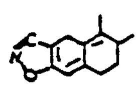 | 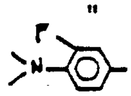 | " | " | " |
| " | " | " | $-C\equiv C-H$ | -OH |
| " | " | " | OH | $-C\equiv C-H$ |
| " | " | " | " | $-C\equiv C-CH_3$ |
| " | " | " | " | $-C\equiv C-CH_2-CH_3$ |
| " | " | " | " | $-C\equiv C-Cl$ |
| " | " | " | " | $-CH_2-C\equiv C-H$ |
|  | " | $CH_2CH_3$ | " | $-C\equiv C-H$ |
| " | " | " | " | $-C\equiv C-CH_3$ |

| 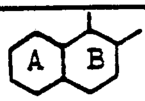 | $R_1$ | $R_2$ | $R_3$ | $R_4$ |
|---|---|---|---|---|
| 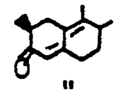 | 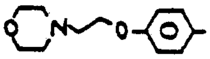 | $CH_3$ | -OH | $-CH_2-C≡C-H$ |
| " | " | " | -C≡C-H | -OH |
| " | " | " | 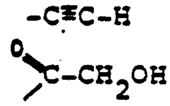 | -H |
| " | 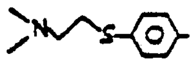 | " | " | " |
| " | " | " | -C≡C-H | OH |
| " | " | " | OH | $-C≡C-CF_3$ |
| " | " | " | OH | -C≡C-H |
| " | " | " | " | $-CH_2-CH=CH_2$ |
| " | " | " | " | $CH_2-C≡C-H$ |
| " | " | " | " | $-CH_2$ |
| " | " | " | 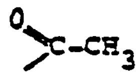 | $-CH_3$ |
| " | " | " | OH | $-CH_2-CN$ |
| 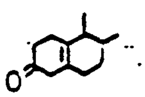 | " | " | OH | -C≡C-H |
| " | " | " | " | $-C≡C-CH_3$ |
| " | " | " | " | $-C≡C-CH_2CH_3$ |
| " | " | " | " | -C≡C-Cl |
| " | " | " | " | $-CH_2-C≡C-H$ |
| " | " | " | -C≡C-H | -OH |
| " | " | " | 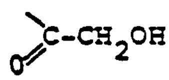 | -H |
| 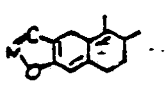 | 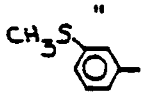 | " | " | " |
| " | " | " | -C≡C-H | -OH |
| " | " | " | OH | -C≡C-H |
| " | " | " | " | $-C≡C-CH_3$ |
| " | " | " | " | $-C≡C-CH_2-CH_3$ |
| " | " | " | " | -C≡C-Cl |
| " | " | " | " | $-CH_2-C≡C-H$ |
|  | " | $CH_2CH_3$ | " | -C≡C-H |
| " | " | " | " | $-C≡C-CH_3$ |

| 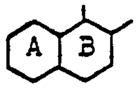 | $R_1$ | $R_2$ | $R_3$ | $R_4$ |
|---|---|---|---|---|
| 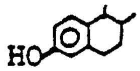 | 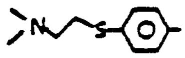 | $CH_2CH_3$ | OH | $-CH_2-C≡C-H$ |
| " | " | " | " | $-CH_2-CH=CH_2$ |
| " | " | " | 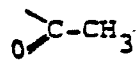 | $-CH_3$ |
| " | " | " | 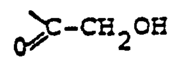 | -H |
| " | " | " | -C≡C-H | -OH |
| " | 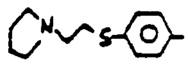 | $CH_3$ | OH | -C≡C-H |
| " | " | " | " | $-C≡C-CH_3$ |
| " | " | " | " | -C≡C-Cl |
| " | " | " | " | $-C≡C-CH_2CH_3$ |
| " | " | " | " | $-CH_2-C≡C-H$ |
| " | " | " | " | $-CH_2-CH=CH_2$ |
| " | " | " | -C≡C-H | -OH |
| " | " | " | 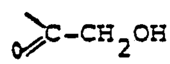 | -H |
| " | " | " | 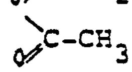 | $-CH_3$ |
| " | " | " | 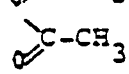 | -H |
| " | 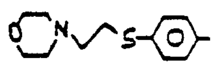 | " | " | " |
| " | " | " | " | $-CH_3$ |
| " | " | " | 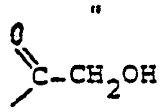 | -H |
| " | " | " | -C≡C-H | -OH |
| " | " | " | OH | -C≡C-H |
| " | " | " | " | $-C≡C-CH_3$ |
| " | " | " | " | -C≡C-Cl |
| " | " | " | " | $-C≡C-CH_2CH_3$ |
| " | " | " | " | $-CH_2-C≡C-H$ |
| " | " | " | " | $-CH_2-CH=CH_2$ |
| " | 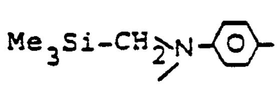 | " | " | -C≡C-H |
| " | " | " | " | $-C≡C-CH_3$ |
| " | " | " | " | $-CH_2-C≡C-H$ |

| 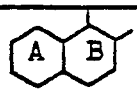 | $R_1$ | $R_2$ | $R_3$ | $R_4$ |
|---|---|---|---|---|
| 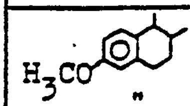 | 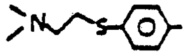 | $CH_2CH_3$ | OH | $-CH_2-C\equiv C-H$ |
| " | " | " | " | $-CH_2-CH=CH_2$ |
| " | " | " | 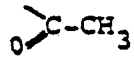 | $-CH_3$ |
| " | " | " | 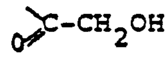 | -H |
| " | " | " | $-C\equiv C-H$ | -OH |
| " | 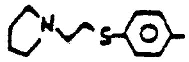 | $CH_3$ | OH | $-C\equiv C-H$ |
| " | " | " | " | $-C\equiv C-CH_3$ |
| " | " | " | " | $-C\equiv C-Cl$ |
| " | " | " | " | $-C\equiv C-CH_2CH_3$ |
| " | " | " | " | $-CH_2-C\equiv C-H$ |
| " | " | " | " | $-CH_2-CH=CH_2$ |
| " | " | " | $-C\equiv C-H$ | -OH |
| " | " | " | 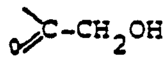 | -H |
| " | " | " | 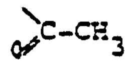 | $-CH_3$ |
| " | " | " | 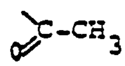 | -H |
| " | 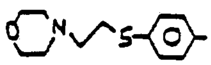 | " | " | " |
| " | " | " | " | $-CH_3$ |
| " | " | " | 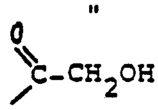 | -H |
| " | " | " | $-C\equiv C-H$ | -OH |
| " | " | " | OH | $-C\equiv C-H$ |
| " | " | " | " | $-C\equiv C-CH_3$ |
| " | " | " | " | $-C\equiv C-Cl$ |
| " | " | " | " | $-C\equiv C-CH_2CH_3$ |
| " | " | " | " | $-CH_2-C\equiv C-H$ |
| " | " | " | " | $-CH_2-CH=CH_2$ |
| " | 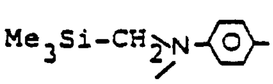 | " | " | $-C\equiv C-H$ |
| " | " | " | " | $-C\equiv C-CH_3$ |
| " | " | " | " | $-CH_2-C\equiv C-H$ |

| 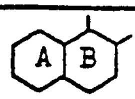 | $R_1$ | $R_2$ | $R_3$ | $R_4$ |
|---|---|---|---|---|
| 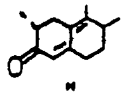 | 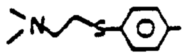 | $CH_2CH_3$ | OH | $-CH_2-C{\equiv}C-H$ |
| " | " | " | " | $-CH_2-CH{=}CH_2$ |
| " | " | " | 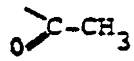 | $-CH_3$ |
| " | " | " | 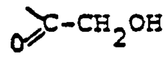 | $-H$ |
| " | " | " | $-C{\equiv}C-H$ | $-OH$ |
| " | 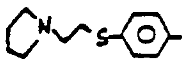 | $CH_3$ | OH | $-C{\equiv}C-H$ |
| " | " | " | " | $-C{\equiv}C-CH_3$ |
| " | " | " | " | $-C{\equiv}C-Cl$ |
| " | " | " | " | $-C{\equiv}C-CH_2CH_3$ |
| " | " | " | " | $-CH_2-C{\equiv}C-H$ |
| " | " | " | " | $-CH_2-CH{=}CH_2$ |
| " | " | " | $-C{\equiv}C-H$ | $-OH$ |
| " | " | " | 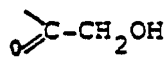 | $-H$ |
| " | " | " | 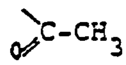 | $-CH_3$ |
| " | " | " | 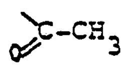 | $-H$ |
| " | 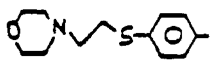 | " | " | " |
| " | " | " | " | $-CH_3$ |
| " | " | " | 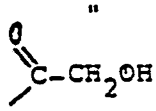 | $-H$ |
| " | " | " | $-C{\equiv}C-H$ | $-OH$ |
| " | " | " | OH | $-C{\equiv}C-H$ |
| " | " | " | " | $-C{\equiv}C-CH_3$ |
| " | " | " | " | $-C{\equiv}C-Cl$ |
| " | " | " | " | $-C{\equiv}C-CH_2CH_3$ |
| " | " | " | " | $-CH_2-C{\equiv}C-H$ |
| " | " | " | " | $-CH_2-CH{=}CH_2$ |
| " | 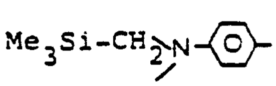 | " | " | $-C{\equiv}C-H$ |
| " | " | " | " | $-C{\equiv}C-CH_3$ |
| " | " | " | " | $-CH_2-C{\equiv}C-H$ |

| 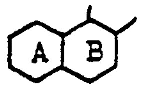 | $R_1$ | $R_2$ | $R_3$ | $R_4$ |
|---|---|---|---|---|
| 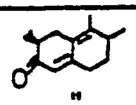 | 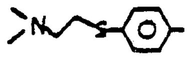 | $CH_2CH_3$ | OH | $-CH_2-C{\equiv}C-H$ |
| " | " | " | " | $-CH_2-CH{=}CH_2$ |
| " | " | " | 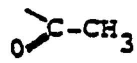 | $-CH_3$ |
| " | " | " | 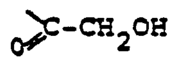 | -H |
| " | " | " | $-C{\equiv}C-H$ | -OH |
| " | 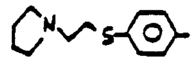 | $CH_3$ | OH | $-C{\equiv}C-H$ |
| " | " | " | " | $-C{\equiv}C-CH_3$ |
| " | " | " | " | $-C{\equiv}C-Cl$ |
| " | " | " | " | $-C{\equiv}C-CH_2CH_3$ |
| " | " | " | " | $-CH_2-C{\equiv}C-H$ |
| " | " | " | " | $-CH_2-CH{=}CH_2$ |
| " | " | " | $-C{\equiv}C-H$ | -OH |
| " | " | " | 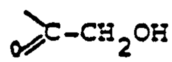 | -H |
| " | " | " | 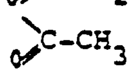 | $-CH_3$ |
| " | " | " | 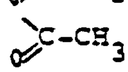 | -H |
| " | 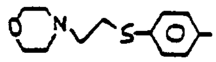 | " | " | " |
| " | " | " | " | $-CH_3$ |
| " | " | " | 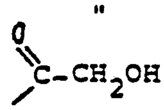 | -H |
| " | " | " | $-C{\equiv}C-H$ | -OH |
| " | " | " | OH | $-C{\equiv}C-H$ |
| " | " | " | " | $-C{\equiv}C-CH_3$ |
| " | " | " | " | $-C{\equiv}C-Cl$ |
| " | " | " | " | $-C{\equiv}C-CH_2CH_3$ |
| " | " | " | " | $-CH_2-C{\equiv}C-H$ |
| " | " | " | " | $-CH_2-CH{=}CH_2$ |
| " | 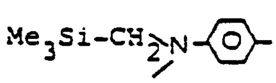 | " | " | $-C{\equiv}C-H$ |
| " | " | " | " | $-C{\equiv}C-CH_3$ |
| " | " | " | " | $-CH_2-C{\equiv}C-H$ |

| 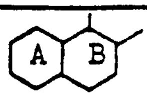 | $R_1$ | $R_2$ | $R_3$ | $R_4$ |
|---|---|---|---|---|
| 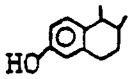 | 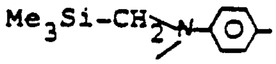 | $CH_3$ | $-C\equiv C-H$ | $-OH$ |
| " | " | " | 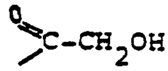 | $-H$ |
| " | 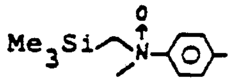 | " | OH | $-C\equiv C-H$ |
| " | " | " | " | $-C\equiv C-CH_3$ |
| " | " | " | " | $-CH_2-C\equiv C-H$ |
| " | " | " | $-C\equiv C-H$ | $-OH$ |
| " | " | " | 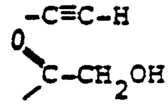 | $-H$ |
| " | 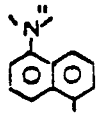 | " | OH | $-C\equiv C-CH_3$ |
| " | " | " | " | $-CH_2-C\equiv C-H$ |
| " | 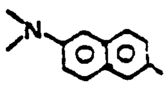 | " | " | $-C\equiv C-H$ |
| " | " | " | " | $-C\equiv C-CH_3$ |
| " | " | " | " | $-C\equiv C-Cl$ |
| " | " | " | " | $-CH_2-C\equiv C-H$ |
| " | " | " | $-C\equiv C-H$ | $-OH$ |
| " | " | " | 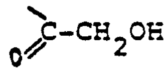 | $-H$ |
| " | 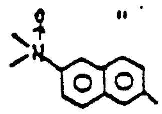 | " | " | " |
| " | " | " | $-C\equiv C-H$ | $-OH$ |
| " | " | " | $-OH$ | $-C\equiv C-H$ |
| " | " | " | " | $-C\equiv C-CH_3$ |
| " | " | " | " | $-C\equiv C-Cl$ |
| " | " | " | " | $-CH_2-C\equiv C-H$ |
| " | $Me_3Si$ $CH_2-$ | " | " | $-C\equiv C-H$ |
| " | " | " | " | $-C\equiv C-CH_3$ |
| " | " | " | " | $-CH_2-C\equiv C-H$ |
| " | " | " | $-C\equiv C-H$ | $-OH$ |
| 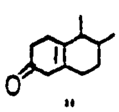 | " | " | " | " |
| " | " | " | OH | $-C\equiv C-H$ |
| " | " | " | " | $-C\equiv C-CH_3$ |

| 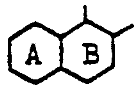 | $R_1$ | $R_2$ | $R_3$ | $R_4$ |
|---|---|---|---|---|
| 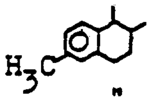 | 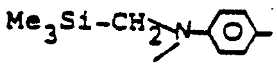 | $CH_3$ | $-C\equiv C-H$ | $-OH$ |
| " | " | " | 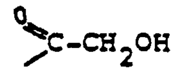 | $-H$ |
| " | 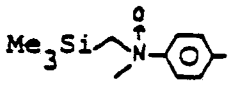 | " | OH | $-C\equiv C-H$ |
| " | " | " | " | $-C\equiv C-CH_3$ |
| " | " | " | " | $-CH_2-C\equiv C-H$ |
| " | " | " | $-C\equiv C-H$ | $-OH$ |
| " | " | " | 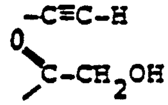 | $-H$ |
| " | 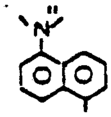 | " | OH | $-C\equiv C-CH_3$ |
| " | " | " | " | $-CH_2-C\equiv C-H$ |
| " | 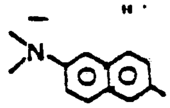 | " | " | $-C\equiv C-H$ |
| " | " | " | " | $-C\equiv C-CH_3$ |
| " | " | " | " | $-C\equiv C-Cl$ |
| " | " | " | " | $-CH_2-C\equiv C-H$ |
| " | " | " | $-C\equiv C-H$ | $-OH$ |
| " | " | " | 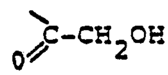 | $-H$ |
| " | 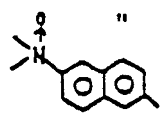 | " | " | " |
| " | " | " | $-C\equiv C-H$ | $-OH$ |
| " | " | " | $-OH$ | $-C\equiv C-H$ |
| " | " | " | " | $-C\equiv C-CH_3$ |
| " | " | " | " | $-C\equiv C-Cl$ |
| " | " | " | " | $-CH_2-C\equiv C-H$ |
| " | $Me_3Si\ CH_2-$ | " | " | $-C\equiv C-H$ |
| " | " | " | " | $-C\equiv C-CH_3$ |
| " | " | " | " | $-CH_2-C\equiv C-H$ |
| " | " | " | $-C\equiv C-H$ | $-OH$ |
| 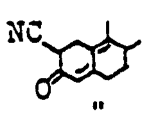 | " | " | " | " |
| " | " | " | OH | $-C\equiv C-H$ |
| " | " | " | " | $-C\equiv C-CH_3$ |

| 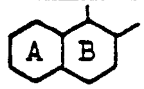 | $R_1$ | $R_2$ | $R_3$ | $R_4$ |
|---|---|---|---|---|
| 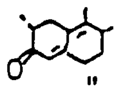 | 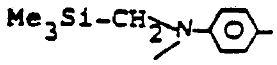 | $CH_3$ | $-C\equiv C-H$ | $-OH$ |
| " | " | " | 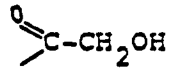 | $-H$ |
| " | 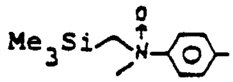 | " | OH | $-C\equiv C-H$ |
| " | " | " | " | $-C\equiv C-CH_3$ |
| " | " | " | " | $-CH_2-C\equiv C-H$ |
| " | " | " | $-C\equiv C-H$ | $-OH$ |
| " | " | " | 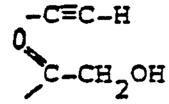 | $-H$ |
| " | 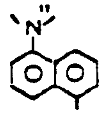 | " | OH | $-C\equiv C-CH_3$ |
| " | " | " | " | $-CH_2-C\equiv C-H$ |
| " | 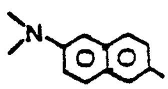 | " | " | $-C\equiv C-H$ |
| " | " | " | " | $-C\equiv C-CH_3$ |
| " | " | " | " | $-C\equiv C-Cl$ |
| " | " | " | " | $-CH_2-C\equiv C-H$ |
| " | " | " | $-C\equiv C-H$ | $-OH$ |
| " | " | " | 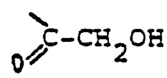 | $-H$ |
| " | 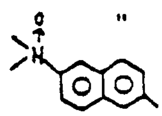 | " | " | " |
| " | " | " | $-C\equiv C-H$ | $-OH$ |
| " | " | " | $-OH$ | $-C\equiv C-H$ |
| " | " | " | " | $-C\equiv C-CH_3$ |
| " | " | " | " | $-C\equiv C-Cl$ |
| " | " | " | " | $-CH_2-C\equiv C-H$ |
| " | $Me_3Si\ CH_2-$ | " | " | $-C\equiv C-H$ |
| " | " | " | " | $-C\equiv C-CH_3$ |
| " | " | " | " | $-CH_2-C\equiv C-H$ |
| " | " | " | $-C\equiv C-H$ | $-OH$ |
| 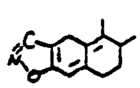 | " | " | " | " |
| " | " | " | OH | $-C\equiv C-H$ |
| " | " | " | " | $-C\equiv C-CH_3$ |

| 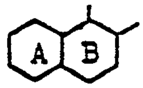 | $R_1$ | $R_2$ | $R_3$ | $R_4$ |
|---|---|---|---|---|
| 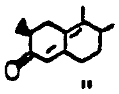 | 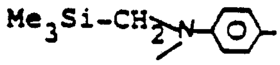 | $CH_3$ | $-C\equiv C-H$ | -OH |
| " | " | " | 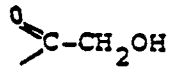 | -H |
| " | 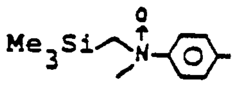 | " | OH | $-C\equiv C-H$ |
| " | " | " | " | $-C\equiv C-CH_3$ |
| " | " | " | " | $-CH_2-C\equiv C-H$ |
| " | " | " | $-C\equiv C-H$ | -OH |
| " | " | " | 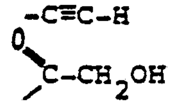 | -H |
| " | 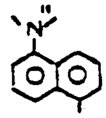 | " | OH | $-C\equiv C-CH_3$ |
| " | " | " | " | $-CH_2-C\equiv C-H$ |
| " | 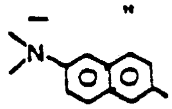 | " | " | $-C\equiv C-H$ |
| " | " | " | " | $-C\equiv C-CH_3$ |
| " | " | " | " | $-C\equiv C-Cl$ |
| " | " | " | " | $-CH_2-C\equiv C-H$ |
| " | " | " | $-C\equiv C-H$ | -OH |
| " | " | " | 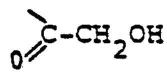 | -H |
| " | 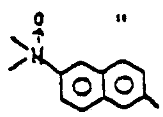 | " | " | " |
| " | " | " | $-C\equiv C-H$ | -OH |
| " | " | " | -OH | $-C\equiv C-H$ |
| " | " | " | " | $-C\equiv C-CH_3$ |
| " | " | " | " | $-C\equiv C-Cl$ |
| " | " | " | " | $-CH_2-C\equiv C-H$ |
| " | $Me_3Si\ CH_2-$ | " | " | $-C\equiv C-H$ |
| " | " | " | " | $-C\equiv C-CH_3$ |
| " | " | " | " | $-CH_2-C\equiv C-H$ |
| " | " | " | $-C\equiv C-H$ | -OH |
| 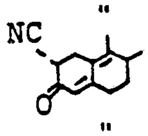 | " | " | " | " |
| " | " | " | OH | $-C\equiv C-H$ |
| " | " | " | " | $-C\equiv C-CH_3$ |

| 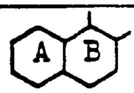 | $R_1$ | $R_2$ | $R_3$ | $R_4$ |
|---|---|---|---|---|
| 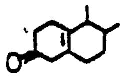 | $Me_3Si\ CH_2-$ | $CH_3$ | OH | $-CH_2-C\equiv C-H$ |
| " | " | $CH_2CH_3$ | $-C\equiv C-H$ | -OH |
| " | " | " | OH | $-C\equiv C-H$ |
| " | " | " | " | $-C\equiv C-CH_3$ |
| " | " | " | " | $-CH_2-C\equiv C-H$ |
| 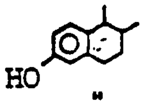 | 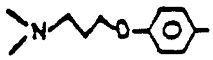 | " | " | $-C\equiv C-CH_3$ |
| " | " | " | 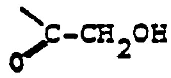 | -H |
| " | 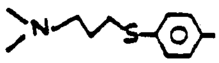 | " | " | " |
| " | " | " | OH | $-C\equiv C-H$ |
| " | " | " | " | $-C\equiv C-CH_3$ |
| " | " | " | " | $-CH_2-C\equiv C-H$ |
| " | " | " | " | $-CH_2-CH=CH_2$ |
| " | " | " | " | $-CH_2CN$ |
| " | " | " | $-C\equiv C-H$ | -OH |
| " | " | " | 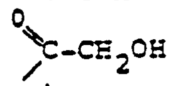 | -H |
| " | " | " | 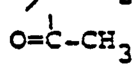 | $-CH_3$ |
| " | " | " | OH | -H |
| " | 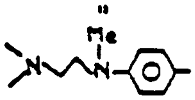 | " | OH | $-C\equiv C-H$ |
| " | " | " | " | $-C\equiv C-CF_3$ |
| " | " | " | " | $-C\equiv C-CH_2CH_3$ |
| " | " | " | " | $-C\equiv C-Cl$ |
| " | " | " | " | $-CH_2-C\equiv C-H$ |
| " | " | " | $-C\equiv C-H$ | -OH |
| " | " | " | 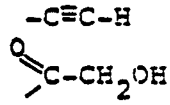 | -H |
| " | " | " | 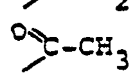 | $-CH_3$ |
| " | " | $CH_2CH_3$ | OH | $-C\equiv C-H$ |
| " | " | " | " | $-C\equiv C-CH_3$ |
| " | " | " | " | $-CH_2-C\equiv C-H$ |

| 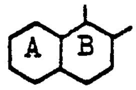 | $R_1$ | $R_2$ | $R_3$ | $R_4$ |
|---|---|---|---|---|
| 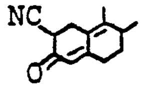 | $Me_3Si\ CH_2-$ | $CH_3$ | OH | $-CH_2-C{\equiv}C-H$ |
| " | " | $CH_2CH_3$ | $-C{\equiv}C-H$ | -OH |
| " | " | " | OH | $-C{\equiv}C-H$ |
| " | " | " | " | $-C{\equiv}C-CH_3$ |
| " | " | " | " | $-CH_2-C{\equiv}C-H$ |
| 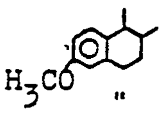 | 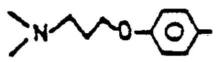 | " | " | $-C{\equiv}C-CH_3$ |
| " | " | " | 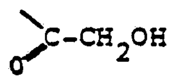 | -H |
| " | 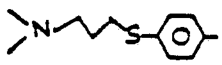 | " | " | " |
| " | " | " | OH | $-C{\equiv}C-H$ |
| " | " | " | " | $-C{\equiv}C-CH_3$ |
| " | " | " | " | $-CH_2-C{\equiv}C-H$ |
| " | " | " | " | $-CH_2-CH{=}CH_2$ |
| " | " | " | " | $-CH_2CN$ |
| " | " | " | $-C{\equiv}C-H$ | -OH |
| " | " | " | 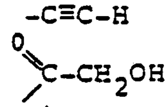 | -H |
| " | " | " | 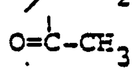 | $-CH_3$ |
| " | " | " | OH | -H |
| " | 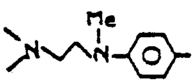 | " | OH | $-C{\equiv}C-H$ |
| " | " | " | " | $-C{\equiv}C-CF_3$ |
| " | " | " | " | $-C{\equiv}C-CH_2CH_3$ |
| " | " | " | " | $-C{\equiv}C-Cl$ |
| " | " | " | " | $-CH_2-C{\equiv}C-H$ |
| " | " | " | $-C{\equiv}C-H$ | -OH |
| " | " | " | 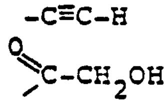 | -H |
| " | " | " | 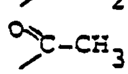 | $-CH_3$ |
| " | " | $CH_2CH_3$ | OH | $-C{\equiv}C-H$ |
| " | " | " | " | $-C{\equiv}C-CH_3$ |
| " | " | " | " | $-CH_2-C{\equiv}C-H$ |

| 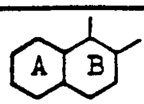 | $R_1$ | $R_2$ | $R_3$ | $R_4$ |
|---|---|---|---|---|
| 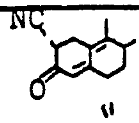 | $Me_3Si\ CH_2$- | $CH_3$ | OH | $-CH_2-C≡C-H$ |
| " | " | $CH_2CH_3$ | $-C≡C-H$ | -OH |
| " | " | " | OH | $-C≡C-H$ |
| " | " | " | " | $-C≡C-CH_3$ |
| " | " | " | " | $-CH_2-C≡C-H$ |
| 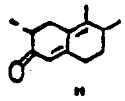 | 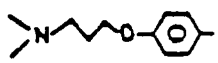 | " | " | $-C≡C-CH_3$ |
| " | " | " | 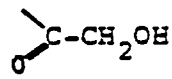 | -H |
| " | 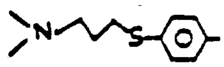 | " | " | " |
| " | " | " | OH | $-C≡C-H$ |
| " | " | " | " | $-C≡C-CH_3$ |
| " | " | " | " | $-CH_2-C≡C-H$ |
| " | " | " | " | $-CH_2-CH=CH_2$ |
| " | " | " | " | $-CH_2CN$ |
| " | " | " | $-C≡C-H$ | -OH |
| " | " | " | 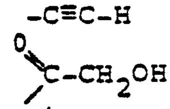 | -H |
| " | " | " | 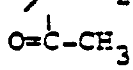 | $-CH_3$ |
| " | " | " | OH | -H |
| " | 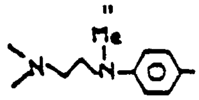 | " | OH | $-C≡C-H$ |
| " | " | " | " | $-C≡C-CF_3$ |
| " | " | " | " | $-C≡C-CH_2CH_3$ |
| " | " | " | " | $-C≡C-Cl$ |
| " | " | " | " | $-CH_2-C≡C-H$ |
| " | " | " | $-C≡C-H$ | -OH |
| " | " | " | 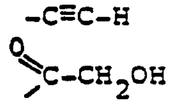 | -H |
| " | " | " | 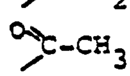 | $-CH_3$ |
| " | " | $CH_2CH_3$ | OH | $-C≡C-H$ |
| " | " | " | " | $-C≡C-CH_3$ |
| " | " | " | " | $-CH_2-C≡C-H$ |

| 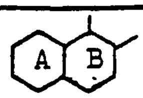 | $R_1$ | $R_2$ | $R_3$ | $R_4$ |
|---|---|---|---|---|
| 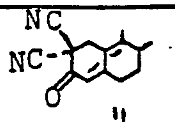 | $Me_3Si\ CH_2-$ | $CH_3$ | OH | $-CH_2-C{\equiv}C-H$ |
| " | " | $CH_2CH_3$ | $-C{\equiv}C-H$ | -OH |
| " | " | " | OH | $-C{\equiv}C-H$ |
| " | " | " | " | $-C{\equiv}C-CH_3$ |
| " | " | " | " | $-CH_2-C{\equiv}C-H$ |
| 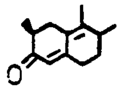 | 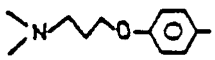 | " | " | $-C{\equiv}C-CH_3$ |
| " | " | " | 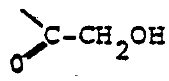 | -H |
| " | 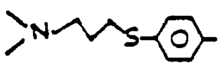 | " | " | " |
| " | " | " | OH | $-C{\equiv}C-H$ |
| " | " | " | " | $-C{\equiv}C-CH_3$ |
| " | " | " | " | $-CH_2-C{\equiv}C-H$ |
| " | " | " | " | $-CH_2-CH=CH_2$ |
| " | " | " | " | $-CH_2CN$ |
| " | " | " | $-C{\equiv}C-H$ | -OH |
| " | " | " | 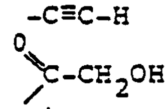 | -H |
| " | " | " | 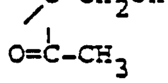 | $-CH_3$ |
| " | " | " | OH | -H |
| " | 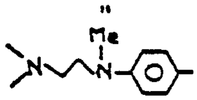 | " | OH | $-C{\equiv}C-H$ |
| " | " | " | " | $-C{\equiv}C-CF_3$ |
| " | " | " | " | $-C{\equiv}C-CH_2CH_3$ |
| " | " | " | " | $-C{\equiv}C-Cl$ |
| " | " | " | " | $-CH_2-C{\equiv}C-H$ |
| " | " | " | $-C{\equiv}C-H$ | -OH |
| " | " | " | 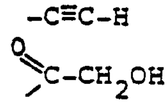 | -H |
| " | " | " | 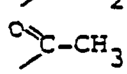 | $-CH_3$ |
| " | " | $CH_2CH_3$ | OH | $-C{\equiv}C-H$ |
| " | " | " | " | $-C{\equiv}C-CH_3$ |
| " | " | " | " | $-CH_2-C{\equiv}C-H$ |

| 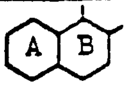 | $R_1$ | $R_2$ | $R_3$ | $R_4$ |
|---|---|---|---|---|
| 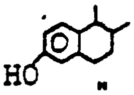 | 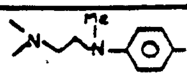 | $CH_2CH_3$ | $-C\equiv C-H$ | $-OH$ |
| " | " | " | 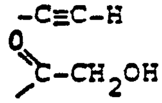 | $-H$ |
| " | 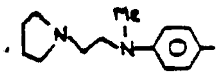 | $CH_3$ | OH | $-C\equiv C-H$ |
| " | " | " | " | $-C\equiv C-CH_3$ |
| " | " | " | " | $-CH_2-C\equiv C-H$ |
| " | " | " | $-C\equiv C-H$ | $-OH$ |
| " | " | " | 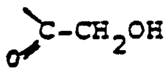 | $-H$ |
| " | 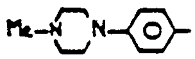 | " | " | " |
| " | " | " | $-C\equiv C-H$ | $-OH$ |
| " | " | " | OH | $-C\equiv C-H$ |
| " | " | " | " | $-CH_2-C\equiv C-H$ |
| " | " | " | " | $-CH_2-CH=CH_2$ |
| " | " | " | " | $-CH_2CN$ |
| " | 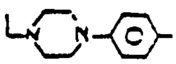 | " | " | $-C\equiv C-H$ |
| " | " | " | " | $-C\equiv C-CH_3$ |
| " | " | " | " | $-CH_2-C\equiv C-H$ |
| " | " | " | " | $-CH_2-CH=CH_2$ |
| " | " | " | " | $-CH_2-CN$ |
| " | " | " | $-C\equiv C-H$ | $-OH$ |
| " | 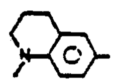 | " | $-OH$ | $-C\equiv C-H$ |
| " | " | " | " | $-C\equiv C-CH_3$ |
| " | " | " | " | $-CH_2-C\equiv C-H$ |
| " | " | " | $-C\equiv C-H$ | $-OH$ |
| " | " | " | 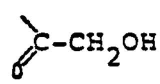 | $-H$ |
| " | 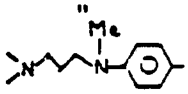 | " | " | " |
| " | " | " | $-C\equiv C-H$ | OH |
| " | " | " | OH | $-C\equiv C-H$ |
| " | " | " | " | $-C\equiv C-CH_3$ |

| 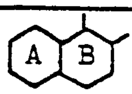 | $R_1$ | $R_2$ | $R_3$ | $R_4$ |
|---|---|---|---|---|
| 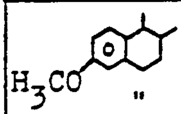 | 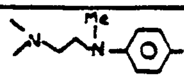 | $CH_2CH_3$ | $-C\equiv C-H$ | -OH |
| " | " | " | 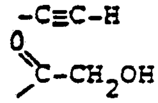 | -H |
| " | 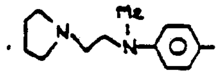 | $CH_3$ | OH | $-C\equiv C-H$ |
| " | " | " | " | $-C\equiv C-CH_3$ |
| " | " | " | " | $-CH_2-C\equiv C-H$ |
| " | " | " | $-C\equiv C-H$ | -OH |
| " | " | " | 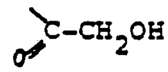 | -H |
| " | 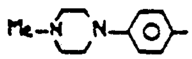 | " | " | " |
| " | " | " | $-C\equiv C-H$ | -OH |
| " | " | " | OH | $-C\equiv C-H$ |
| " | " | " | " | $-CH_2-C\equiv C-H$ |
| " | " | " | " | $-CH_2-CH=CH_2$ |
| " | " | " | " | $-CH_2CN$ |
| " | 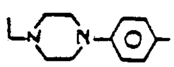 | " | " | $-C\equiv C-H$ |
| " | " | " | " | $-C\equiv C-CH_3$ |
| " | " | " | " | $-CH_2-C\equiv C-H$ |
| " | " | " | " | $-CH_2-CH=CH_2$ |
| " | " | " | " | $-CH_2-CN$ |
| " | " | " | $-C\equiv C-H$ | -OH |
| " | 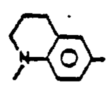 | " | -OH | $-C\equiv C-H$ |
| " | " | " | " | $-C\equiv C-CH_3$ |
| " | " | " | " | $-CH_2-C\equiv C-H$ |
| " | " | " | $-C\equiv C-H$ | -OH |
| " | " | " | 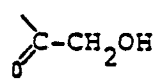 | -H |
| " | 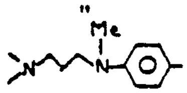 | " | " | " |
| " | " | " | $-C\equiv C-H$ | CH |
| " | " | " | OH | $-C\equiv C-H$ |
| " | " | " | " | $-C\equiv C-CH_3$ |

| 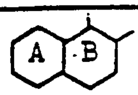 | $R_1$ | $R_2$ | $R_3$ | $R_4$ |
|---|---|---|---|---|
| 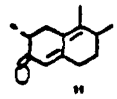 | 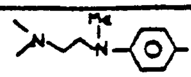 | $CH_2CH_3$ | $-C\equiv C-H$ | -OH |
| " | " | " | 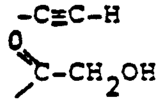 | -H |
| " | 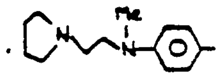 | $CH_3$ | OH | $-C\equiv C-H$ |
| " | " | " | " | $-C\equiv C-CH_3$ |
| " | " | " | " | $-CH_2-C\equiv C-H$ |
| " | " | " | $-C\equiv C-H$ | -OH |
| " | " | " | 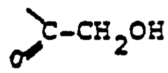 | -H |
| " | 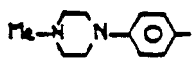 | " | " | " |
| " | " | " | $-C\equiv C-H$ | -OH |
| " | " | " | OH | $-C\equiv C-H$ |
| " | " | " | " | $-CH_2-C\equiv C-H$ |
| " | " | " | " | $-CH_2-CH=CH_2$ |
| " | " | " | " | $-CH_2CN$ |
| " | 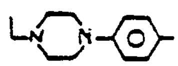 | " | " | $-C\equiv C-H$ |
| " | " | " | " | $-C\equiv C-CH_3$ |
| " | " | " | " | $-CH_2-C\equiv C-H$ |
| " | " | " | " | $-CH_2-CH=CH_2$ |
| " | " | " | " | $-CH_2-CN$ |
| " | " | " | $-C\equiv C-H$ | -OH |
| " | 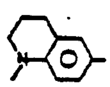 | " | -OH | $-C\equiv C-H$ |
| " | " | " | " | $-C\equiv C-CH_3$ |
| " | " | " | " | $-CH_2-C\equiv C-H$ |
| " | " | " | $-C\equiv C-H$ | -OH |
| " | " | " | 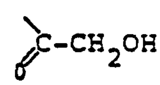 | -H |
| " | 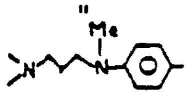 | " | " | " |
| " | " | " | $-C\equiv C-H$ | OH |
| " | " | " | OH | $-C\equiv C-H$ |
| " | " | " | " | $-C\equiv C-CH_3$ |

| 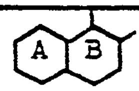 | $R_1$ | $R_2$ | $R_3$ | $R_4$ |
|---|---|---|---|---|
| 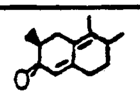 | 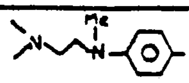 | $CH_2CH_3$ | $-C\equiv C-H$ | $-OH$ |
| " | " | " | 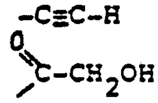 | $-H$ |
| " | 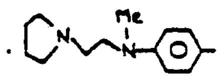 | $CH_3$ | OH | $-C\equiv C-H$ |
| " | " | " | " | $-C\equiv C-CH_3$ |
| " | " | " | " | $-CH_2-C\equiv C-H$ |
| " | " | " | $-C\equiv C-H$ | $-OH$ |
| " | " | " | 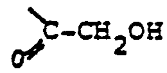 | $-H$ |
| " | 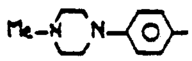 | " | " | " |
| " | " | " | $-C\equiv C-H$ | $-OH$ |
| " | " | " | OH | $-C\equiv C-H$ |
| " | " | " | " | $-CH_2-C\equiv C-H$ |
| " | " | " | " | $-CH_2-CH=CH_2$ |
| " | " | " | " | $-CH_2CN$ |
| " | 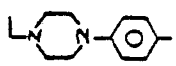 | " | " | $-C\equiv C-H$ |
| " | " | " | " | $-C\equiv C-CH_3$ |
| " | " | " | " | $-CH_2-C\equiv C-H$ |
| " | " | " | " | $-CH_2-CH=CH_2$ |
| " | " | " | " | $-CH_2-CN$ |
| " | " | " | $-C\equiv C-H$ | $-OH$ |
| " | 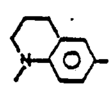 | " | $-OH$ | $-C\equiv C-H$ |
| " | " | " | " | $-C\equiv C-CH_3$ |
| " | " | " | " | $-CH_2-C\equiv C-H$ |
| " | " | " | $-C\equiv C-H$ | $-OH$ |
| " | " | " | 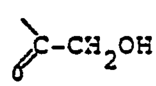 | $-H$ |
| " | 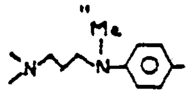 | " | " | " |
| " | " | " | $-C\equiv C-H$ | OH |
| " | " | " | OH | $-C\equiv C-H$ |
| " | " | " | " | $-C\equiv C-CH_3$ |

| 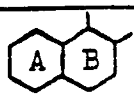 | $R_1$ | $R_2$ | $R_3$ | $R_4$ |
|---|---|---|---|---|
| 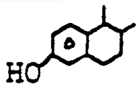 | 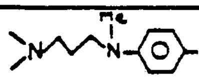 | $CH_3$ | OH | $-CH_2-C\equiv C-H$ |
| " | " | " | " | -H |
| " | " | " | " | $-CH_3$ |
| " | 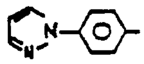 | " | " | $-C\equiv C-H$ |
| " | " | " | " | $-C\equiv C-CH_3$ |
| " | " | " | " | $-C\equiv C-CF_3$ |
| " | " | " | " | $-CH_2-C\equiv C-H$ |
| " | " | " | $-C\equiv C-H$ | -OH |
| " | 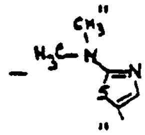 | " | OH | $-C\equiv C-H$ |
| " | " | " | " | $-C\equiv C-CH_3$ |
| " | " | " | " | $-C\equiv C-CF_3$ |
| " | " | " | " | $-CH_2-C\equiv C-H$ |
| " | " | " | $-C\equiv C-H$ | -OH |
| " | " | " | $-C\equiv C-CH_3$ | -OH |
| 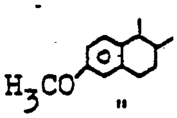 | 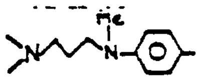 | $CH_3$ | OH | $-CH_2-C\equiv C-H$ |
| " | " | " | " | -H |
| " | " | " | " | $-CH_3$ |
| " | 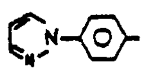 | " | " | $-C\equiv C-H$ |
| " | " | " | " | $-C\equiv C-CH_3$ |
| " | " | " | " | $-C\equiv C-CF_3$ |
| " | " | " | " | $-CH_2-C\equiv C-H$ |
| " | " | " | $-C\equiv C-H$ | -OH |
| " | 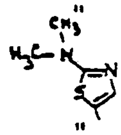 | " | OH | $-C\equiv C-H$ |
| " | " | " | " | $-C\equiv C-CH_3$ |
| " | " | " | " | $-C\equiv C-CF_3$ |
| " | " | " | " | $-CH_2-C\equiv C-H$ |
| " | " | " | $-C\equiv C-H$ | -OH |
| " | " | " | $-C\equiv C-CH_3$ | -OH |

| 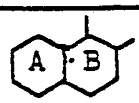 | $R_1$ | $R_2$ | $R_3$ | $R_4$ |
|---|---|---|---|---|
| 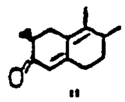 | 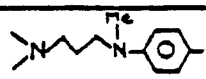 | $CH_3$ | .OH | $-CH_2-C\equiv C-H$ |
| " | " | " | " | -H |
| " | " | " | " | $-CH_3$ |
| " | 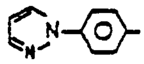 | " | " | $-C\equiv C-H$ |
| " | " | " | " | $-C\equiv C-CH_3$ |
| " | " | " | " | $-C\equiv C-CF_3$ |
| " | " | " | " | $-CH_2-C\equiv C-H$ |
| " | " | " | $-C\equiv C-H$ | -OH |
| " | 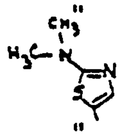 | " | OH | $-C\equiv C-H$ |
| " | " | " | " | $-C\equiv C-CH_3$ |
| " | " | " | " | $-C\equiv C-CF_3$ |
| " | " | " | " | $-CH_2-C\equiv C-H$ |
| " | " | " | $-C\equiv C-H$ | -OH |
| " | " | " | $-C\equiv C-CH_3$ | -OH |
| 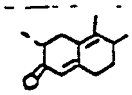 | 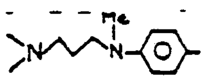 | $CH_3$ | OH | $-CH_2-C\equiv C-H$ |
| " | " | " | " | -H |
| " | " | " | " | $-CH_3$ |
| " | 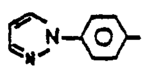 | " | " | $-C\equiv C-H$ |
| " | " | " | " | $-C\equiv C-CH_3$ |
| " | " | " | " | $-C\equiv C-CF_3$ |
| " | " | " | " | $-CH_2-C\equiv C-H$ |
| " | " | " | $-C\equiv C-H$ | -OH |
| " | 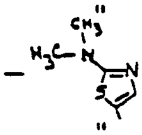 | " | OH | $-C\equiv C-H$ |
| " | " | " | " | $-C\equiv C-CH_3$ |
| " | " | " | " | $-C\equiv C-CF_3$ |
| " | " | " | " | $-CH_2-C\equiv C-H$ |
| " | " | " | $-C\equiv C-H$ | -OH |
| " | " | " | $-C\equiv C-CH_3$ | -OH |

**EXEMPLE 1**

: 2,2-diméthyl 17β-hydroxy 11β-(4-diméthylamino phényl) 17α-(prop-1-ynyl) estra-4,9-dien-3-one

**Stade A**

: 11β-(4-diméthylamino phényl) 17α-(prop-1-ynyl) 17β-/(2RS-tétrahydropyrannyl) oxy/ estra 4,9-dièn-3-one.

On dissout 1,075 g de 17β-hydroxy 11β-(4-diméthylamino phényl) 17α-(prop-1-ynyl) estra-4,9-dièn-3-one dans 10 ml de tétrahydrofuranne et 2 $cm^3$ de 3,4-dihydro 2H-pyranne, ajoute 570 mg d'acide paratoluène sulfonique et agite une heure à température ambiante. On ajoute 1 ml de triéthylamine, dilue à l'acétate d'éthyle et lave avec une solution saturée de carbonate acide de sodium. On sèche, distille à sec sous pression réduite et obtient 2 g de produit brut sous forme d'huile jaune.

**Stade B**

: 2,2-diméthyl 11β-(4-diméthylamino phényl) 17α-(prop-1-ynyl) 17β-/(2RS-tétrahydro pyrannyl)oxy/ estra-4,9-dièn-3-one.

Le produit brut obtenu ci-dessus au stade A est dissous dans 15 ml de tétrahydrofuranne anhydre. On refroidit vers - 60° C et ajoute sous agitation et en 5 à 6 minutes, alternativement par fractions de 1 ml, 3 ml d'une solution 2M de tertbutylate de potassium dans le tétrahydrofuranne et une solution ajustée à 3 ml de 0,38 ml d'iodure de méthyle dans le tétrahydrofuranne. On agite 5 minutes après la fin de l'introduction, ajoute 1 ml de triéthylamine, laisse revenir à température ambiante puis distille à sec.

**Stade C**

: 2,2-diméthyl 17β-hydroxy 11β-(4-diméthylamino phényl) 17α-(prop-1-ynyl) estra-4,9-dièn-3-one

Le produit obtenu ci-dessus au stade A est dissous dans 10 ml de méthanol. On ajoute 2,5 ml d'acide chlorhydrique 5N. On laisse reposer 15 minutes à température ambiante puis dilue à l'eau et chasse le méthanol sous pression réduite.

On ajoute 2,5 ml d'ammoniaque concentrée, essore le produit précipité, et le lave à l'eau. On le redissout dans l'acétate d'éthyle, sèche la solution et distille à sec.

Le produit obtenu (1,5 g) est purifié par chromatographie sur silice (éluant: benzène-acétate d'ethyle 9/1). On obtient 990 mg de produit attendu cristallisé que l'on reprend dans l'heptane. On essore, lave, sèche et obtient 980 mg de produit purifié, F = 170 - 172° C.

On obtient un produit pur en opérant comme suit: on joint au produit ci-dessus un échantillon de même qualité et dissout l'ensemble (2,2 g) dans du chlorure de méthylène. On filtre et distille à sec le filtrat. On dissout le filtrat au reflux de 10 ml d'éther isopropylique. On refroidit, amorce la cristallisation par grattage et abandonne vers + 5° C. On essore les cristaux obtenus, les lave, les sèche et obtient 1,88 g de produit attendu, F = 171° C.

$(\alpha)_D$ = + 183,5° ± 3,5(c=1 % dans le chloroforme)

Analyse: $C_{31}H_{39}NO_2$

Calculé: C % 81,36 H % 8,59 N % 3,06

Trouvé: 81,1 8,8 3,2

Le 17β-hydroxy 11β-(4-diméthylamino phényl) 17α-(prop-1-ynyl) estra-4,9-dièn-3-one utilisé au départ de l'exemple 1 a été préparé dans la demande européenne n° 57115.

**EXEMPLE 2**

: 11β-(4-diméthylamino phényl) 17α-(prop-1-ynyl) estra 1,3,5 (10)-triène 3,17-diol et 17β-acétoxy 11β-(4-diméthylamino phényl) 17α-(prop-1-ynyl) estra 1,3,5 (10)-trièn 3-ol

1°) Aromatisation:

On introduit goutte à goutte à + 5° C 2 ml d'anhydride acétique à 98 % et 1 ml de bromure d'acétyle dans une solution sous azote de 2 g de 17β-hydroxy 11β-(4-diméthyl amino phényl) 17α-(prop-1-ynyl) estra 4,9-dièn-3-one dans 20 ml de chlorure de méthylène sec. On agite une heure à température ambiante puis ajoute 100 ml de solution de carbonate acide de sodium. On agite 30 minutes, décante, extrait au chlorure de méthylène sèche et amène à sec. On obtient 2,65 g de produit.

2°) Saponification:

Le produit précédent (2,65 g) est dissous dans 25 ml de méthanol puis on ajoute à température ambiante sous azote 2,6 ml de lessive de soude. On agite 45 minutes, acidifie par 14 ml d'acide chlorhydrique 2N puis alcalinise à l'ammoniaque. On extrait au chlorure de méthylène, lave à l'eau, séche et amène à sec sous pression réduite. On obtient 2,3 g de produit que l'on chromatographie sur silice en éluant par un mélange chlorure de méthylène-acétate d'éthyle 8:2.

On obtient dans l'ordre 430 mg de 17β-acétoxy 11β-(4-diméthylamino phényl) 17α-(prop-1-ynyl) estra 1,3,5 (10) trièn-3-ol (Rf = 0,58) et 800 mg de 11β-(4-diméthylamino phényl) 17α-(prop-1-ynyl) estra 1,3,5 (10) trièn 3,17-diol (Rf = 0,36).

Le produit 17β acétoxy (430 mg) est purifié comme suit: On le dissout dans 4 ml de chlorure de méthylène au reflux, filtre, ajoute 4 ml d'éther isopropylique, concentre à petit volume, refroidit, essore, lave à l'éther isopropylique puis sèche à 60° C sous vide.

On obtient 370 mg de produit F = 236° C,

$(\alpha)_D$ = - 194,5° ± 3°

Analyse: $C_{31}H_{37}NO_3$

Calculé: C % 78,95 H % 7,91 N % 2,97
Trouvé: 78,7 7,9 3,0

Le produit 3,17-diol est purifié comme suit:

On dissout 880 mg provenant de deux préparations similaires dans 12 ml de chlorure de méthylène au reflux, filtre, ajoute 12 ml d'éther isopropylique, concentre à 5 ml glace, essore lave à l'éther isopropylique glacé puis sèche et obtient 770 mg de produit F = 246° C, $(\alpha)_D$ = -188,5° ± 2,5°

Analyse: $C_{29}H_{35}NO_2$
Calculé: C % 81,08 H % 8,21 N % 3,26
Trouvé: 81,1 8,2 3,1

**EXEMPLE 3**

: 2-/(acétyloxy)méthylène/ 11β-(4-diméthylamino phényl) 17β-hydroxy 17α-(prop-1-ynyl) estra 4,9-dièn-3-one.

**Stade A**

: 17β-hydroxy 2-hydroxyméthylène 11β-(4-diméthyl amino phényl) 17α-(prop-1-ynyl) estra 4,9-dièn 3-one.

On lave trois fois 5 g d'hydrure de sodium en suspension à 55,5% dans l'huile par 50 ml d'oxyde de diéthyle en éliminant le surnageant puis ajoute 100 ml de benzène sec et 5 g de 17β-hydroxy 11β-(4-diméthylamino phényl) 17α-(prop-1-ynyl)estra 4,9-dièn-3-one. On ajoute ensuite goutte à goutte 9,4 ml de formiate d'éthyle.

On agite 20 heures à température ambiante puis verse dans 200 ml d'eau. On lave par 3 fois 50 ml d'oxyde de diéthyle et réextrait par 2 fois 50 ml d'eau. La phase organique est éliminée, les phases aqueuses rassemblées sont amenées à pH 3, 2 par addition de 70 ml d'acid e chlorhydrique 2N. On revient à pH basique en rajoutant 40 ml de solution saturée de carbonate acide de sodium.

On extrait par 100 ml puis 3 fois 50 ml d'oxyde de diéthyle, lave par 50 ml de solution saturée de chlorure de sodium, sèche et porte à sec sous pression réduite. On obtient 5,2 g de produit brut utilisé tel quel pour le reste de la synthèse.

**Stade B:**

2-/(acétyloxy)méthylène/ 11β-(4-diméthylamino phényl) 17β-hydroxy 17α-(prop-1-ynyl) estra 4,9 dièn-3-one.

On ajoute à + 5° C 0,2 ml de pyridine puis 0,16 ml de chlorure d'acétyle à une solution de 460 mg de produit obtenu au stade A dans 20 ml de chloroforme sec. On laisse une heure au bain de glace, ajoute 10 ml de solution aqueuse de carbonate acide de sodium, agite 5 minutes, décante, lave à l'eau, sèche puis amène à sec sous pression réduite.

On obtient 510 mg de produit brut que l'on dissout dans 10 ml de chlorure de méthylène, ajoute 50 mg de charbon actif, filtre puis amène à sec. On obtient 490 mg de produit que l'on empâte dans l'éther de pétrole, lave ensuite à l'éther de pétrole puis a l'éther isopropylique (2 fois 5 ml). On sèche à 50° C sous vide. On obtient 380 mg de produit attendu. $(\alpha)_D$ = + 182° ± 3° (c = 1 % dans le chloroforme).

Analyse: $C_{32}H_{37}NO_4$
Calculé: C % 76,92 H % 7,46 N % 2,8
Trouvé: 77,0 7,6 2,6

**EXEMPLE 4**

: 11β-(4-diméthylamino phényl) 17β-hydroxy 2-(4-morpholinyl méthylène) 17α-(prop-1-ynyl) estra 4,9-dièn-3-one.

On ajoute 0,24 ml de morpholine à une solution de 1,065 g de 17β-hydroxy 2-hydroxy méthylène 11β-(4-diméthylamino phényl) 17α-(prop-1-ynyl) estra 4,9 dièn-3-one obtenue comme à l'exemple 3 stade A, dans 10 ml de méthanol. On chauffe une heure à 55° C puis amène à sec à 50° C sous pression réduite.

On obtient 1,25 g de produit brut que l'on chromatograghie sur alumine (éluant: cyclohexane-acétone-triéthylamine 70/30/1) Rf = 0,4. On obtient 1,06 g de produit pur $(\alpha)_D$ = +263° ± 4,5° (c = 0,7 % dans le chloroforme).

**EXEMPLE 5**

: 11β-(4-diméthylamino phényl) 17β-hydroxy 2α-méthyl 17α-(prop-1-ynyl) estra 4,9-dièn-3-one, et 11β-(4-diméthylamino phényl) 17β-hydroxy 2β-méthyl 17α-(prop-1-ynyl) estra 4,9-dièn-3- one.

On introduit lentement à -70° C sous argon une solution de 0,82 ml de cyclohexyl isopropylamine dans 5 ml de tétrahydrofurane dans un mélange de 3 ml de solution 1,6 M de butyllithium dans l'hexane et de 5 ml de tétrahydrofurane.

On laisse réagir dix minutes puis introduit 2,2 g de 11β-(4-diméthylamino phényl) 17α-(prop-1-ynyl) 17β-/(2RS-tétrahydropyrannyl)oxy/ estra 4,9-dièn-3-one préparé comme à l'exemple 1, stade A, en solution dans 8 ml de tétrahydrofurane anhydre. On laisse réagir 10 minutes à - 70° C puis ajoute 0,5 ml d'iodure de méthyle.

On laisse 30 minutes à - 70° C puis amène à température ambiante. On ajoute 1 ml de triéthylamine puis dilue avec de l'acétate d'éthyle et lave à l'eau. Après séchage et évaporation du solvant on obtient 2,5 g de produit protégé en 17β par un radical tétrahydropyrannyle.

Ce produit est dissous dans 25 ml de méthanol. On ajoute 4 ml d'acide chlorhydrique à 50 %. On laisse 2 heures à température ambiante puis dilue à l'eau et extrait au chlorure de méthylène. On

lave à l'eau, sèche, évapore la phase organique sous pression réduite et obtient 2,04 g de produit attendu sous forme de mélange d'épimères 2-méthylés.

On chromatographie le mélange obtenu sur 300 g de silice avec un mélange éther-éther de pétrole (2/3).

On sépare 600 mg d'isomère 2β et 345 mg d'isomère 2α (et environ 280 mg de mélange).

On recristallise 1,1 g d'isomère 2β dans un mélange chlorure de méthylène-éther isopropylique et obtient 1,04 g de produit F = 211° C.

On recristallise 703 mg d'isomère 2α dans le même mélange chlorure de méthylène-éther isopropylique et obtient 614 mg de produit purifié F = 204° C. Une nouvelle recristallisation donne 560 mg de produit pur F = 205° C.

Analyse: $C_{30}H_{37}NO_2$
Calculé: C % 81,22 H % 8,40 N % 3,15
Trouve
isomère 2α: 81,2 8,5 3,2
isomère 2β: 80,8 8,6 3,2

**EXEMPLE 6**

: 17β-hydroxy 11β-(3-méthoxyphényl) 17α-(prop-1-ynyl) estr-5(10) en-3-one et(E)17β-hydroxy 11β-(3-méthoxyphényl) 17α-(prop-1-eny-1) estr 5(10) en-3-one.

On ajoute 20 ml de tétrahydrofurane, 5 ml de tert-butanol et 4,17 g de 11β-(3-méthoxy phényl) 17β-hydroxy 17α-(prop-1-ynyl) estra 4,9-dièn-3-one à 100 ml d'ammoniac liquide à - 55° C. On ajoute à la solution obtenue 170 mg de lithium coupé en petits morceaux. Après deux heures à - 55° C on introduit lentement 50 ml de solution aqueuse de chlorure d'ammonium, laisse revenir à température ambiante, extrait à l'acétate d'éthyle, lave à l'eau salée, sèche et amène à sec. On obtient 4,3 g d'une résine que l'on chromatographie sur 430 g de gel de silice en éluant par un mélange cyclohexaneacetate d'éthyle 7/3.

On obtient 380 mg de produit 17α prop-1-ényl, Rf = 0,27 et 2,65 g de produit 17α prop-1-ynyle, Rf = 0,25.

Constantes physique:
Produit 17α prop-1-ényle
$(\alpha)_D$ = + 10,5° ± 1°(c=1,2 % dans le chloroforme).
Analyse: $C_{28}H_{36}O_3$
Calculé: C % 79,96 H % 8,63
Trouvé: 79,8 8,9
Produit 17α- prop-1-ynyle
$(\alpha)_D$ = - 30° ± 1,5(c=1 % dans le chloroforme);
Analyse: $C_{28}H_{34}O_3$
Calculé: C % 80,34 H % 8,19
Trouvé: 80,4 8,3

La 11β;(3-méthoxyphényl) 17β-hydroxy 17α(prop-1-ynyl)estra 4,9-dièn-3-one utilisée au départ de l'exemple 6 a été préparé comme suit:

a) 3,3-éthylène dioxy 11β-(3-méthyloxy phényl) l7α(prop-1-ynyl) estra 9-èn 5α,17β-diol.

On agite à - 20° C pendant 15 minutes un mélange sous azote de 33,8 ml de solution à 0,8 M de bromure de 3-méthyl-oxy phényl magnésium dans le tétrahydrofurane et de 285 mg de chlorure cuivreux puis ajoute en 15 minutes 3,3 g de 3,3-éthylène dioxy 5α,10α-époxy l7α(prop-1-ynyl) estr 9(11) en 17β-ol dans 33 ml de tétrahydrofurane. Après quelques minutes on rajoute 33 ml de tétrahydrofurane. Après une heure d'agitation à -20° C on verse dans un mélange de 15 g de chlorure d'ammonium dans 200 ml d'eau glacée. On agite une demi-heure, extrait trois fois à l'éther et lave à l'eau. On sèche et porte à sec sous pression réduite. On obtient 5,3 g de produit brut que l'on chromatographie sur--------silice (éluant: chlorure de méthylène-acétone 95/5 à 1 % de triéthylamine).

On isole 2,7 g de produit attendu, Rf = 0,30.

On recristallise 200 mg de produit dans le mélange chlorure de méthyléne-éther isopropylique at obtient 165 mg de produit sous forme de cristaux blancs, F = 228° C.

Analyse: $C_{30}H_{38}O_5$
Calculé: C % 75,28 H % 8,0
Trouvé: 75,3 8,1

b) 17β-hydroxy 11β-(3-méthyloxy phényl) 17α-(prop-1-ynyl) estra 4,9-dièn 3-one.

On porte à reflux sous agitation pendant une heure trente minutes un mélange de 2,5 g de 3,3-éthylène dioxy 11β-(3-méthyloxy phényl) 17α-(prop-1-ynyl) estra 9-èn 5α, 17β-diol dans 125 ml d'éthanol à 95° et 2,5 g de résine Redex®.

Après refroidissement on filtre la résine et rince abondamment avec de l'éthanol à 95°.

On porte le filtrat à sec sous pression réduite et obtient 2,38 g de produit brut que l'on chromatographie sur silice (éluant: éther de pétrole-acétate d'éthyle 3/2). On isole 1,75 g de produit amorphe que l'on recristallise dans un mélange éther-cyclohéxane. On essore, rince au cyclohexane, sèche sous vide et obtient 1,42 g de produit attendu, F = 164° C.

Analyse: $C_{28}H_{32}O_3$
Calculé: C % 80,73 H % 7,74
Trouvé: 80,7 7,9

**EXEMPLE 7**

: 11β-(4-diméthylamino phényl) 17α-(prop-1-ynyl) isoxazolo /4,5-b/ estra 4,9-dièn-17-ol.

On ajoute 350 mg de chlorhydrate d'hydroxylamine à une solution de 1,2 g de 17β-hydroxy 2-hydroxyméthylène 11β-(4-diméthylamino phényl) 17α-(prop-1-ynyl) estra 4,9-dièn 3-one dans 6 ml de tert-butanol et porte 10 minutes au reflux.

On refroidit, dilue le mélange à l'eau et extrait au chlorure de méthylène. La phase organique lavée, séchée est évaporée sous pression réduite. On obtient 1,23 g de produit brut que l'on chromatographie sur 90 g de silice (éluant: éther

de pétrole-éther 4/6). On obtient 755 mg de produit pur.

**EXEMPLE 8**

: 2-cyano 11β-(4-diméthylamino phényl) 17β-hydroxy 17α-(prop-1-ynyl) estra 4,9-dièn 3-one.

On fait barboter de l'azote à température ambiante pendant 15 minutes dans une solution de 1,2 g de produit préparé à l'exemple 7 dans 10 ml de méthanol puis ajoute 3 ml de potasse N.

On abandonne la solution sous azote à température ambiante pendant 3 heures. On dilue à l'eau, extrait au chlorure de méthylène, lave la phase organique à l'eau, sèche puis évapore sous pression réduite. On obtient 1,19 g de produit attendu que l'on chromatographie sur ----- silice (éluant: étheréther de pétrole 7/3). On obtient 685 mg de produit attendu.

Analyse: $C_{30}H_{34}O_2N_2$, 0,25 $H_2O$
Calculé: C % 78,48 H % 7,57 N % 6,10 $H_2O$ % ∶ 1 %
Trouvé: 78,2 7,6 5,9 $H_2O$ ∶ 0,8 %

**EXEMPLE 9**

: 2,2-dicyano 11β-(4-diméthylamino phényl) 17β-hydroxy 17α-(prop-1-ynyl) estra 4,9-dièn-3-one.

On introduit sous argon 4,4 ml d'une solution 1,6 M de butylithium dans l'hexane dans 10 ml de tétrahydrofurane anhydre. On refroidit à -70° C et ajoute une solution de 1,15 ml de N-cyclohexyl isopropylamine dans 15 ml de tétrahydrofurane anhydre.

On refroidit la solution ainsi obtenue à -70° C et ajoute 1,290 g de 17β-hydroxy 11β-(4-diméthyl aminophényl) 17α-(prop-1-ynyl) estra 4,9-dièn-3-one en solution dans 12 ml de tétrahydrofurane. On obtient la solution A.

On refroidit à -60° C une solution de 2,55 g de cyanure de tosyle dans 15 ml de tétrahydrofurane et ajoute goutte à goutte la solution A obtenue ci-dessus. On laisse réagir 40 minutes à -60° C puis laisse revenir à température ambiante. On verse dans l'eau et extrait à l'acétate d'éthyle. La phase organique est lavée, séchée et évaporée sous vide. On obtient 2,85 g de produit que l'on chromatographie sur 80 g de silice (éluant: benzène-acétate d'éthyle 4/1). On obtient 1,06 g de produit attendu que l'on recristallise dans un mélange chlorure de méthylène-éther isopropylique. On obtient 774 mg de produit pur. F = 265° C.

Analyse: $C_{31}H_{33}N_3O_2$
Calculé: C % 77,63 H % 6,93 N % 8,76
Trouvé: 77,6 7,0 8,7

**EXEMPLE 10**

: 17β-hydroxy 11β-(4-diméthylamino phényl) 17α-/prop-1-ynyl) estr 5(10) en-3-one.

On ajoute 15 ml de tétrahydrofurane anhydre et 2,5 ml de tert-butanol dans 40 ml d'ammoniac liquide refroidi à -60° C. On ajoute ensuite 2,15 g de 17β-hydroxy 11β-(4-diméthylamino phényl) 17α-(prop-1-ynyl) estra 4,9-dien-3-one. Aprés dissolution, on introduit 80 mg de lithium en 6 fractions en l'espace de 30 minutes. Après 30 minutes supplémentaires on retire le bain réfrigérant et fait couler lentement 40 ml de solution aqueuse à 100 g/l de chlorure d'ammonium. On agite 30 minutes à température ambiante puis extrait à l'acétate d'éthyle. La phase organique est lavée avec une solution de chlorure d'ammonium, séchée et évaporée à sec. Les produits obtenus de 2,3 g sont chromatographiés sur 90 g de gel de silice (éluant: benzène-acétate d'éthyle 4/1). On obtient 1,67 g de produit attendu. Un échantillon analytique est préparé par nouvelle chromatographie sur silice (éluant: éther éthylique-heptane 2/1)

$(\alpha)_D$ = - 68,5° ± 2° (c = 1 % dans le chloroforme).

Analyse: $C_{29}H_{37}NO_2$
Calculé: C % 80,7 H % 8,64 N % 3,25
Trouvé: 80,6 8,90 3,05

**EXEMPLE 11**

: 11β-(4-diméthylamino phényl) 3-méthoxy 17α-prop-1-ynyl estra 1,3,5(10) trien-17β-ol.

On ajoute en une seule fois 0,06 ml de sulfate de méthyle à une solution maintenue à la température d'un bain de glace et constituée de 215 mg de 11β-(4-diméthylamino phényl) 17α-(prop-1-ynyl) estra 1,3,5(10) trien 3,17-diol préparé à l'exemple 2 dans 6 ml de soude 0,1 N et 6 ml d'acétone. On supprime le bain de glace et agite 2 heures 30. On dilue par 30 ml d'acétate d'éthyle, décante, lave à l'eau salée, seche puis élimine les solvants et obtient 155 mg de produit que l'on chromatographie sur gel de silice (éluant: éther de pétrole-acétate d'éthyle 3/2) après dissolution dans le chlorure de méthylène.

On obtient 120 mg de produit attendu, Rf = 0,4

**EXEMPLE 12**

: 17β-acétoxy 11β-(4-diméthylamino phényl) 3-méthoxy 17α-(prop-1-ynyl) estra 1,3,5(10) triène.

On ajoute sous agitation 0,08 ml de sulfate de méthyle à une solution maintenue sous azote de 330 mg de 17β-acétoxy 11β-(4-diméthylamino phényl) 17α-(prop-1-ynyl) estra 1,3,5(10) trien-3-ol préparé à l'exemple 2 dans 1,65 ml d'acétone; 0,33 ml d'eau et 0,42 ml de soude 2N. On agite une heure puis dilue le milieu réactionnel avec de

l'eau et extrait à l'acétate d'éthyle. On sèche la phase organique et évapore le solvant sous pression réduite.

On purifie le produit ainsi obtenu par chromatographie sur gel de silice (éluant: cyclohexane-acétate d'éthyle 4/1) On obtient 220 mg de produit attendu.

**EXEMPLE 13**

: 11β-/4-(2-diméthylaminoéthoxy) phenyl/ estra 1,3, 5(10)trièn 3, 17β-diol.

**Stade A**

: 11β-/4-(2-diméthylaminoéthoxy) phényl/ 3-hydroxy estra 1,3,5(10)trièn-17-one.

A 4 g de 11β-/4-(2-diméthylaminoéthoxy) phényl/ estra 4,9-dien 3,17-dione dans 40 ml de chlorure de méthylène à 0, +5° C, on ajoute en 10 minutes, un mélange de 4 ml d'anhydride acétique et 2 ml de bromure d'acétyle, agite 2 heures en laissant revenir à température ambiante. On verse le mélange réactionnel dans 200 ml d'une solution aqueuse saturée de bicarbonate de sodium et agite un quart d'heure. On extrait au chlorure de méthylène, lave les extraits organiques à l'eau, sèche et concentre à sec sous pression réduite. On obtient 4,33 g de produit que l'on dissout dans 40 ml de méthanol, ajoute 4 ml de lessive de soude, agite 1 heure 30 min à temperature ambiante. On verse le mélange réactionnel dans 200 ml d'eau, acidifie à pH ≅ 2 --par addition de 30 ml d'acide chlorhydrique 2N, puis amène à pH ≅ 9 par addition de 5 ml d'ammoniaque. On extrait au chlorure de méthylène, lave à l'eau, sèche et concentre à sec sous pression réduite. On chromatographie le résidu sur silice, élue par un mélange chlorure de méthylène-méthanol (9-1) et obtient 2,6 g de produit attendu.

**Stade B**

: 11β-/4-(2-diméthylaminoéthoxy) phényl/ estra 1,3,5 (10)trièn 3,17β-diol.

On mélange 2,1 g du produit obtenu ci-dessus et 21 ml de méthanol, ajoute par petites fractions 545 mg de borohydrure de sodium et agite à température ambiante pendant 1 heure. On verse dans 210 ml d'un mélange glace et eau, extrait au chlorure de méthylène, lave à l'eau saturée en chlorure de sodium sèche et concentre à sec sous pression réduite. On chromatographie le résidu sur silice en éluant par un mélange acétone-méthanol (8-2) et obtient 1,37 g de produit que l'on recristallise dans un mélange chlorure de méthylène-éther isopropylique. On isole 1,27 g de produit attendu. F = 130° C.

$/\alpha/_D$ = -46,5° ± 1,5° (0,8% $CHCl_3$).

Le produit de départ du stade A a été préparé comme suit: Stade a: 11β/4-(2-diméthylaminoéthoxy) phényl/ 3,3-éthylène dioxy 5α-hydroxy estr-9-ène 17-one.

Dans un mélange de 11,5 g de tournures de magnésium et 20 ml de tétrahydrofuranne, on introduit, en 1 heure un quart et en maintenant la température à environ 35° C, 97,4 g de 4-bromo 4-(2-diméthylaminoéthoxy) benzène dans 480 ml de tétrahydrofuranne. On agite encore 1 heure et verse 380 ml de cette solution dans une suspension contenant 23,5 g de complexe Cu Br,$(CH_3)_2S$ et 235 ml de tétrahydrofuranne. Après un quart d'heure d'agitation à température ambiante, on ajoute en 20 minutes, 30 g de 3,3-éthylène dioxy 5α,10α-époxy estr-9(11)èn 17-one(décrite dans la demande européenne n° 57115) dans 150 ml de tétrahydrofuranne et laisse sous agitation à température ambiante pendant 16 heures. On verse le mélange réactionnel dans 3 litres de solution aqueuse saturée en chlorure d'ammonium. On extrait à l'éther, lave les solutions organiques avec une solution saturée de chlorure d'ammonium puis avec une solution saturée de chlorure de sodium, sèche et concentre à sec sous pression réduite. On obtient 66,7 g de produit brut que l'on chromatographie d'abord sur silice en éluant avec un mélange chloroforme-méthanol (9-1) à 1‰ de triéthylamine puis sur alumine en éluant avec un mélange benzène-acétate d'éthyle (8-2). On obtient 30,65 g de produit attendu.

Stade b: 11β-/4-(2-diméthylaminoéthoxy) phényl/ estra 4,9-dièn 3,17-dione.

On agite 3 heures à température ambiante, 5 g de produit obtenu ci-dessus dans 100 ml de méthanol et 15 ml d'acide chlorhydrique 2N. On verse le mélange réactionnel dans 400 ml d'éther et amène à pH alcalin par addition de 100 ml de bicarbonate de sodium 0,5M. Après un quart d'heure d'agitation, on décante et extrait à l'éther, lave les phases organiques avec une solution saturée de chlorure de sodium, sèche et concentre a sec sous pression réduite. On obtient 3,90 g de produit que l'on empâte 3 fois avec un minimum d'éther isopropylique. On obtient 3,10 g de produit attendu. F = 206° C.

**EXEMPLE 14**

: 11β-/4-(2-diméthylaminoéthoxy) phényl) estra 1,3, 5(10)trièn 17β-ol.

**Stade A**

: 11β-/4-(2-diméthylaminoéthoxy) phényl/ estra 4,9-dièn 3,17β-diol (mélange d'isomère A et d'isomère B).

A 5 g de 11β-/4-(2-diméthylaminoéthoxy) phényl/ estra 4,9-dièn 3,17-dione (obtenu au

stade b de l'exemple 13) dans 100 ml de méthanol, on ajoute 1,74 g de borohydrure de sodium par petites fractions en 20 minutes. On agite encore 1 heure à température ambiante et verse dans 750 ml d'eau et glace et agite 30 minutes. On essore le précipité formé, le lave à l'eau jusqu'à neutralité, le sèche et obtient 4,6 g de produit, mélange des 2 isomères A et B. F ≅ 110° C.

**Stade B**

: 11β-/4-(2-diméthylaminoéthoxy) phényl/ estra 1,3,5 (10)trién 17β-ol.

On agite à température ambiante pendant 3 heures, 1 g de produit obtenu au stade A, 20 ml de tétrahydrofuranne et 1 ml d'acide chlorhydrique 6N. On verse le mélange réactionnel dans 200 ml de solution aqueuse saturée de bicarbonate de sodium et extrait à l'acétate d'éthyle, puis lave avec une solution aqueuse saturée de chlorure de sodium, sèche et concentre à sec sous pression réduite. on chromatographie le résidu sur alumine en éluant par un mélange éther de pétrole (eb 60-80° C)-acétone (8-2) et obtient 325 mg de produit que l'on dissout dans un mélange chlorure de méthylène-éther isopropylique (1-l),concentre lentement au demi et recueille 300,5 mg de produit attendu. F = 155° C. /α/$_D$ = -38,5° ± 2° (c = 0,8% $CHCl_3$).

**EXEMPLE 15**

: 3-(2-diméthylamino) éthoxy 11β-phényl estra 1,3,5 (10)trién 17β-ol.

**Stade A**

: 3-hydroxy 11β-phényl estra 1,3,5(10)trién 17-one.

On refroidit à 0° C, 3,76 g de 11β-phényl estra 4,9-dièn 3,17-dione dans 26,3 ml de chlorure de méthylène et introduit goutte à goutte, sous agitation, un mélange contenant 3,75 ml d'anhydride acétique et 1,9 ml de bromure d'acétyle. On agite pendant 1 heure 15 minutes à température ambiante. On verse le milieu réactionnel, goutte à goutte, et sous agitation, dans 90 ml d'une solution aqueuse saturée de bicarbonate. Après 15 minutes, on extrait au chlorure de méthylène, lave à l'eau, sèche, concentre à sec sous pression réduite. Au résidu, on ajoute 26,3 ml de méthanol puis 18,8 ml de lessive de soude et maintient l'agitation pendant 16 heures. On acidifie à pH ~ 1, en maintenant la température à ~ 20° C, par addition de 40 ml d'acide sulfurique dilué au 1/5ème et agite 20 minutes le produit cristallisé formé. On essore, lave par empâtage, avec 4 fois 25 ml d'eau. On obtient 4,030 g de produit que l'on recristallise dans le chlorure de méthylène. On isole 3,01 g de produit pur. F = 290° C.

/α/$_D$ = -9° ± 2° (c = 0,5% $CHCl_3$).

**Stade B**

: 11β-phényl estra 1,3,5(10)trién 3 17β-diol.

On chauffe à 50° C, sous agitation et sous atmosphère inerte 1 g du produit obtenu ci-dessus dans 10 ml de méthanol et introduit en 10 minutes, 144 mg de borohydrure de sodium. Après 1 heure dans les mêmes conditions, on refroidit à +20° C, amène le pH à 5 par addition, goutte à goutte, de 0,4 ml d'acide acétique et agite 10 minutes. On verse le mélange réactionnel dans 30 ml d'eau et glace, agite 30 minutes, essore, lave à l'eau par empêtage, sèche et obtient 896 mg de produit. F = 228° C. /α/$_D$ = -34° ± 2° (c = 0,5% $CHCl_3$).

**Stade C**

: 3-(2-diméthylaminoéthoxy) 11β-phényl estra 1,3,5(10) trièn 17β-ol.

On mélange 1,220 g de produit obtenu ci-dessus et 12,2 ml d'éthanol à 95°. On ajoute en une seule fois 3,5 ml d'une solution N de soude dans l'éthanol à 95° et chauffe à 60° C. On ajoute en une fois, l'amine préparée à partir de 555 mg de chlorhydrate de diméthylamino 2-chloroéthane dissous dans 1,7 ml d'éthanol à 95° puis neutralise par 3,85 ml d'une solution N de soude dans l'éthanol à 95° fraichement préparée. On chauffe au reflux sous agitation et atmosphère inerte pendant 1 heure et 30 minutes. On refroidit à 20° C, filtre le chlorure de sodium formé, l'empâte avec 10 ml d'éthanol à 95°. On concentre à sec le filtrat. On agite le résidu avec 20 ml de chlorure de méthylène et 20 ml d'eau pendant 10 minutes, décante et extrait la phase aqueuse au chlorure de méthylène, lave les phases organiques à l'eau, sèche, concentre à sec sous pression réduite et obtient 1,261 g de produit brut. Après chromatographie sur silice élution par un mélange chloroforme-méthanol (6-4), on recueille 939 mg de produit attendu. /α/$_D$ = -32° ± 2° (c = 0,7% $CHCl_3$). Le produit de départ du stade A a été préparé comme suit:

**Stade a**

: 3,3-éthylène dioxy 5α-hydroxy 11β-phényl estr-9-èn 17-one.

On introduit en 1 heure 10 minutes et en laissant la température s'élever jusqu'au reflux, 80 g de bromobenzène dans 400 ml de tétrahydrofuranne, dans un mélange de 13 g de tournures de magnésium et 30 ml de

tétrahydrofuranne. On maintient l'agitation et laisse la température revenir à +20° C. On refroidit à -25° C, 330 ml de la solution de bromure de phényl magnésien obtenue (0,85M), ajoute en une fois 4,165 g de chlorure cuivreux et agite 10 minutes à -25° C. On introduit, goutte à goutte, en 20 minutes à -25° C, 11,5 g de 3,3-éthylàne dioxy 5α, 10α-époxy estr 9(11)èn 17-one dans 60 ml de tétrahydrofuranne et maintient les conditions pendant 2 heures. On verse le mélange réactionnel dans 600 ml d'eau et glace et 45 g de chlorure d'ammonium, agite 30 minutes et extrait à l'éther, lave à l'eau, sàche, concentre à sec sous pression réduite. On chromatographie le résidu sur silice, élue par un mélange benzène-acétate d'éthyle (6-4) à 1‰ de triéthylamine et obtient 7,86 de produit attendu. F = 173° C.

$/\alpha/_D = +54{,}5° \pm 1{,}5°$ (c = 1% $CHCl_3$).

**Stade b**

: 11β-phényl estra 4,9-dièn 3,17-dione.

On chauffe à 40° C, 7,4 g de produit obtenu au stade a dans 225 ml d'éthanol à 95° C et introduit en une fois 7,4 g de Résine Redex® CF. On chauffe au reflux pendant une heure sous agitation et sous gaz inerte, filtre la résine et la lave par empâtage 4 fois avec 20 ml d'éthanol à 95°. On concentre à sec le filtrat sous pression réduite et obtient 6,5 g de résidu que l'on chromatographie sur silice, élue par un mélange de chloroforme- acétate d'éthyle (9-1) et obtient 486 mg de produit brut que l'on dissout au reflux dans un mélange de 12,5 ml d'éther isopropylique et 3 ml de chlorure de méthylène, filtre à chaud, concentre à faible volume et laisse cristalliser. On essore, lave à l'éther isopropylique et obtient 369,4 mg de produit attendu. F = 197° C.

$/\alpha/_D = +223° \pm 3°$ (c = 0,5% $CHCl_3$).

**Etude pharmacologique des produits de l'invention.**

**I/ Etude de l'activité des produits de l'invention sur les récepteurs hormonaux**

**Récepteur minéralocorticoide du rein du rat**

Des rats mâles Sprague-Dawley EOPS, pesant 140 à 160 g, surrénalectomisés depuis 4 à 8 jours sont sacrifiés et leurs reins sont perfusés in situ avec 50 ml d'un tampon Tris 10mM Saccharose 0,25 M, HCl pH 7,4. Les reins sont ensuite prélevés, décapsulés et homogénéisés à 0° C à l'aide d'un Potter teflon-verre (1 g de tissu pour 3 ml de tampon). L'homogénat est centrifugé pendant 10 minutes à 800 g à 0° C.

Afin d'éliminer la fixation de l'aldostérone tritiée sur le récepteur glucocorticoïde, le 11β, 17β dihydroxy 21-méthyl pregna 1,4,6-trien 20-yn 3-one stéroide se fixant uniquement sur le récepteur glucocorticoïde est additionné au surnageant à la concentration finale de $10^{-6}$M. Ce surnageant est ultracentrifugé à 105 000 g pendant 60 minutes à 0° C. Des aliquotes du surnageant ainsi obtenu sont incubées à O° C avec une concentration constante (T) d'aldostérone tritiée en présence de concentrations croiasantes (0-2500. $10^{-9}$M) d'aldostérone froide ou du produit froid à étudier. Après un temps (t) d'incubation, la concentration d'aldostérone tritiée liée (B) est mesurée par la technique d'adsorption au charbon-dextran.

**Récepteur androgène de la prostate de rat**

Des rats mâles Sprague Dawley EOPS de 160 à 200 g sont castrés. 24 heures après la castration, les animaux sont sacrifiés, les prostates sont prélevées, pesées et homogénéisées à 0° C à l'aide d'un Potter teflon-verre dans une solution tamponnée TS (Tria 10 mM, saccharose 0,25 M, HCl pH 7,4) (1 g de tissu pour 5 ml de TS). L'homogénat est ensuite ultracentrifugé (105 000 g pendant 60 minutes) à 0° C. Des aliquotes du surnageant ainsi obtenu, sont incubées à 0° C pendant un temps E d'incubation avec une concentration constante (T) de testostérone tritiée en présence de concentrations croissantes (0 - 1000. $10^{-9}$ M), soit de testostérone froide, soit du produit à tester. La concentration de testostérone tritiée liée (B) est ensuite mesurée dans chaque incubat par la technique d'adsorption au charbon-dextran.

**Récepteur Progestogène de l'utérus de lapine**

Des lapines impubères d'environ 1 kg reçoivent une application cutanée de 25 µg d'estradiol. 5 jours après ce traitement, lea animaux sont sacrifiés, les utérus sont prélevés, pesés et homogénéisés à 0° C, à l'aide d'un Potter teflon-verre dans une solution tamponnée TS (Tris 10mM, saccharose 0,25 M, HCl pH 7,4) (1 g de tissu pour 50 ml de TS). L'homogénat est ensuite ultracentrifugé (105 000 g x 90 mn) à 0° C. Des aliquotes du surnageant ainsi obtenu, sont incubées à 0° C pendant un temps t, avec une concentration constante (T) de produit R tritié (17,21-diméthyl 19-nor-4,9-pregnadiène-3,20-dione) en présence de concentrations croissantes (0 - 2 500. $10^{-9}$M) soit de R froid, soit de progestérone froide, soit du produit froid à tester. La concentration de R tritié lié (B) est ensuite mesurée dans chaque incubat par la technique d'adsorption au charbon dextran.

**Récepteur glucocorticoïde du thymus de rat**

Des rats mâles Sprague Dawley EOPS de 160 à 200 g sont surrénalectomisés. 4 à 8 jours après cette ablation, les animaux sont sacrifiés, et les thymus sont prélevés et homogénéisés à 0° C dans un tampon Tris 10mM, saccharose 0,25M, dithiothreitol 2mM, HCl pH 7,4, à l'aide d'un Potter polytétrafluoroétbylène-verre (1 g de tissu Pour 10 ml de TS). L'homogénat est ensuite ultracentrifugé (105 000 g x 90 mn) à 0° C. Des aliquotes du surnageant ainsi obtenu, sont incubées à 0° C pendant un temps (t) avec une concentration constante (T) de dexaméthasone tritiée en présence de concentrations croissantes (0 - 2 500.$10^{-9}$M) soit de dexaméthasone froide, soit du produit froid à tester. La concentration de la dexaméthasone tritiée liée (B) est ensuite mesurée dans chaque incubat par la technique d'adsorption au charbondextran.

**Récepteur estrogène de l'utérus de souris**

Des souris femelles impubères âgées de 18 à 21 jours sont sacrifiées, les utérus sont prélevés puis homogénéisés à 0° C à l'aide d'un Potter teflon-verre dans une solution tamponnée TS (Tris 10mM, saccharose 0,25 M, HCl pH 7,4 (1 g de tissu pour 25 ml de TS). L'homogénat est ensuite ultracentrifugé (105 000 g x 90 mn) à 0° C. Des aliquotes du surnageant ainsi obtenu, sont incubées à 0° C ou à 25° C pendant un temps (t) avec une concentration constante (T) d'estradiol tritié en présence de concentrations croissantes (0 - 1 000. $10^{-9}$M) soit d'estradiol froid, soit du produit froid à tester.

La concentration d'estradiol tritié lié (B) est ensuite mesurée dans chaque incubat par la technique d'adsorption au charbon-dextran.

**Calcul de l'affinité relative de liaison**

Le calcul de l'affinité relative de liaison (ARL) est identique pour tous les récepteurs.

On trace les 2 courbes suivantea: le pourcentage de l'hormone tritiée liée

$$\frac{B}{T}$$

en fonction du logarithme de la concentration de l'hormone de référence froide et

$$\frac{B}{T}$$

en fonction du logarithme de la concentration du produit froid testé. On determine la droite d equation $I_{50}$ = (

$$(\frac{B}{T} \text{ max} + \frac{B}{T} \text{ min})/2.$$

$$\frac{B}{T}$$

max = Pourcentrage de l'hormone tritiée liée pour une incubation de cette hormone tritiée à la concentration (T).

$$\frac{B}{T}$$

min = Pourcentage de l'hormone tritiée liée pour une incubation de cette hormone tritiée à la concentration (T) en présence d'un grand excès d'hormone froide (2500.$10^{-9}$M).

Les intersections de la droite $I_{50}$ et des courbes permettent d'évaluer les concentrations de l'hormone de référence froide (CH) et du produit froid testé (CX) qui inhibent de 50 % la liaison de l'hormone tritiée sur le récepteur.

L'affinité relative de liaison (ARL) du produit testé est déterminée par l'équation

$$ARL = 100 \frac{(CH)}{(CX)}$$

**Conclusion**

Les produits étudiés, particulièrement les produits des éxemples 2 et 5 présentent une affinité très marquée pour les récepteurs glucocorticoïde et progestogène, ainsi qu'une affinité modérée pour le récepteur androgène.

Des résultats obtenus on peut conclure que les produits peuvent présenter des activités agonistes ou antagonistes des glucocorticoïdes, des progestogènes et des androgènes. On peut conclure en outre que les produits des exemples 13 et 14 présentent des activités agonistes ou antagonistes des estrogènes.

Les résultats obtenus sont les suivants:

| Temps d'incubation à 0°C / Produits des exemples | Minéralo corticoïde | | Androgène | | Pro-gestogène | | Gluco-corticoïde | | Estrogène | |
|---|---|---|---|---|---|---|---|---|---|---|
| | 1 H | 24 H | 1/2H | 24 H | 2 H | 24 H | 4 H | 24 H | 2 H | 5 H (25°C) |
| 1 | <0,1 | <0,1 | - | - | 4 | 4 | 97 | 38 | <0,1 | <0,1 |
| 2 (17 OH) | 0,2 | <0,1 | * | * | 39 | 130 | 153 | 222 | 5 | <0,1 |
| 2 (17 OAc) | <0,1 | <0,1 | <0,1 | <0,1 | 5 | 52 | 58 | 100 | <0,1 | 0,3 |
| 5 (2α) | <0,1 | <0,1 | 0,5 | 0,4 | 40 | 39 | 214 | 255 | <0,1 | <0,1 |
| 5 (2β) | <0,1 | <0,1 | 0,7 | 0,5 | 44 | 44 | 224 | 299 | <0,1 | <0,1 |
| 9 | <0,1 | <0,1 | <0,1 | <0,1 | <0,1 | <0,1 | 14 | 7 | <0,1 | <0,1 |
| 10 | 1,5 | <0,1 | - | - | 27 | 62 | 83 | 115 | <0,1 | <0,1 |
| 13 | - | - | 5 | 6 | - | - | 3,9 | - | 78 | 239 |
| 14 | - | - | 10,6 | 0,5 | - | - | 0,4 | - | 4,1 | 1,4 |
| 15 | - | - | 1,3 | 0,2 | - | - | 2 | - | 0,1 | 0,1 |

*: Le produit présente une affinité pour le récepteur.

**II/ Activite antiglucocorticoïde**

La technique utilisée découle de la méthode décrite par Dausse et Coll dans Molecular pharmacology 13, 948-955 (1977) ("the relationship beetween glucocorticoïd structure and effects upon Thymocytes"), pour des thymocytes de souris.

Des thymocytes de rats surrénalectomisés sont incubés à 37° C pendant 3 heures, dans un milieu nutritif renfermant $5.10^{-8}$M de dexaméthasone en présence ou non d'un produit à étudier à différentes concentrations. On ajoute l'uridine tritiée, et poursuit l'incubation pendant une heure. On refroidit les incubats, les traite avec une solution d'acide trichloroacétique à 5%, les filtre sur papier Whatman GF/A, les lave trois fois à l'aide d'une solution d'acide trichloroacétique à 5%. On détermine la radioactivité retenue par le filtre.

Les glucocorticoïdes et en particulier la dexaméthasone provoquent une diminution de l'incorporation d'uridine tritiée. Les produits des exemples 1, 2, 5 et 10 s'opposent à cet effet.

| Produit de l'exemple : | $5.10^{-8}$ Dexaméthasone + produit à tester à la concentration de : | % d'inhibition de l'effet de la Dexaméthasone |
|---|---|---|
| 1 | $10^{-8}$M<br>$10^{-7}$M<br>$10^{-6}$M | 8<br>18<br>* |
| 2 (17 OH | $10^{-8}$M<br>$10^{-7}$M<br>$10^{-6}$M | 41<br>91<br>* |
| 2 (17 OAc) | $10^{-8}$M<br>$10^{-7}$M<br>$10^{-6}$M | 24<br>76<br>* |
| 3 | $10^{-8}$M<br>$10^{-7}$M<br>$10^{-6}$M | 0<br>0<br>60 |
| 4 | $10^{-8}$M<br>$10^{-7}$M<br>$10^{-6}$M | 0<br>0<br>51 |
| 5 (2α) | $10^{-8}$M<br>$10^{-7}$M<br>$10^{-6}$M | 19<br>57<br>100 |
| 5 (2β) | $10^{-8}$M<br>$10^{-7}$M<br>$10^{-6}$M | 10<br>57<br>* |
| 7 | $10^{-8}$M<br>$10^{-7}$M<br>$10^{-6}$M | 0<br>27<br>* |
| 9 | $10^{-8}$M<br>$10^{-7}$M<br>$10^{-6}$M | 3<br>1<br>2 |
| 10 | $10^{-8}$M<br>$10^{-7}$M<br>$10^{-6}$M | 0<br>23<br>* |
| 6 | $10^{-8}$M<br>$10^{-7}$M<br>$10^{-6}$M | 0<br>0<br>68 |
| 6 (17α propényle) | $10^{-8}$M<br>$10^{-7}$M<br>$10^{-6}$M | 0<br>0<br>56 |

*: A la dose de $10^{-6}$ M l'inhibition de l'effet de la déxaméthasone a été totale.

Il a par ailleurs été constaté qu'utilisés seuls les produits testés ne provoquent aucun effet du type glucocorticoïde aux doses provoquant l'éffet antagoniste.

**Conclusion**

Les produits étudiés présentent une activité anti-glucocorticoïde très marquée tout en étant dépourvus d'activité glucocorticoïde.

**Compositions pharmaceutiques:**

On a préparé des comprimés répondant à la formule suivante:

- Produit de l'exemple 5 (2β).............. 50 mg

Excipient (talc, amidon, stéarate de magnésium) q.s. pour un comprime termine à............... 120 mg

**Revendications**

pour les Etats contractants BE CH DE FR GB IT LI LU NL SE

1°) Les produits de formule générale I:

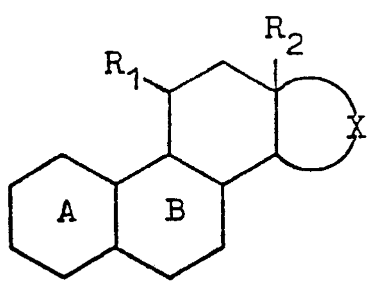

(I)

dans laquelle $R_1$ représente un radical organique renfermant de 1 à 18 atomes de carbone et éventuellement un ou plusieurs hétéroatomes, l'atome immédiatement adjacent au carbone en 11 étant un atome de carbone, $R_2$ représente un radical hydrocarboné renfermant de 1 à 8 atomes de carbone, X représente le reste d'un cycle pentagonal ou hexagonal éventuellement substitué et éventuellement porteur d'une insaturation, les cycles A et B ayant l'une des structures suivantes:

a) - Soit A et B représentent le groupement:

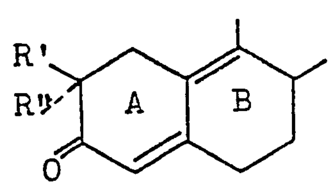

dans lequel R' et R" identiques ou différents représentent un atome d'hydrogène, un radical nitrile ou un radical alkyle ayant de 1 à 4 atomes de carbone étant entendu que l'un au moins des radicaux R' ou R" ne représente pas un atome d'hydrogène;

b) - Soit A et B représentent le groupement:

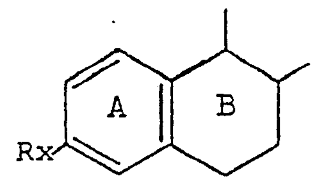

Rx représentant un atome d'hydrogène ou un groupement -ORe, dans lequel Re représente un atome d'hydrogène, un radical alkyle ayant de 1 à 6 atomes de carbone éventuellement substitue ou un radical acyle;

c) - Soit A et B représentent le groupement:

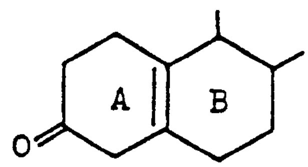

d) - Soit A et B représentent le groupement

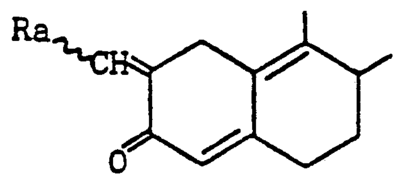

dans lequel Ra représente un radical

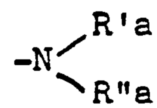

dans lequel R'a et R"a représentent soit un radical alkyle ayant de 1 à 4 atomes de carbone, soit R'a et R"a représentent avec l'atome d'azote auquel ils sont liés, un hétérocycle à 5 ou 6 chaînons comportant éventuellement un autre hétéro atome, ou Ra représente un radical acyloxy, le trait ondulé signifiant que Ra peut se trouver dans la position E ou Z,

e) - Soit A et B représentent le groupement

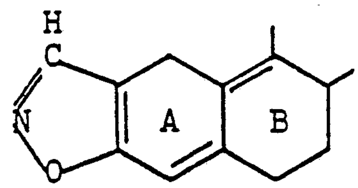

étant entendu que lorsque A et B représentent le groupement

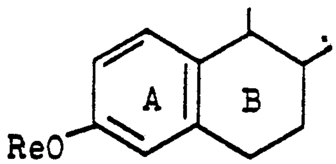

le radical $R_1$ contient au moins un atome d'azote, de phosphore ou de silicium, et que lorsque A et B représentent le groupement

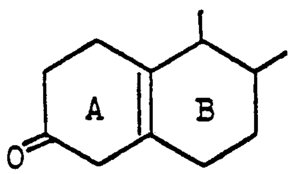

le radical $R_1$ ne représente pas un radical alkyle saturé linéaire, ainsi que les sels d'addition des produits de formule I avec les acides.

2°) Les produits de formule I telle que définie à la revendication 1 dans laquelle X représente le reste d'un cycle de formule

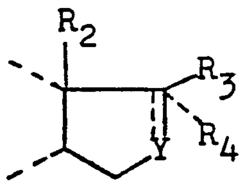

dans lequel $R_2$ conserve la même signification que dans la revendication 1, le trait pointillé en 16-17 symbolise la présence éventuelle d'une double liaison, Y représente un radical

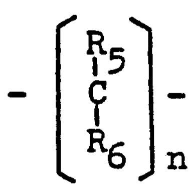

dans lequel n représente le nombre 1 ou 2, $R_5$ représente un atome d'hydrogène, un radical alkyle renfermant de 1 à 8 atomes de carbone, un radical alkényle ou alkynyle renfermant de 2 à 8 atomes de carbone, un radical aryle renfermant de 6 à 14 atomes de carbone, ou un radical aralkyle renfermant de 7 à 15 atomes de carbone, $R_6$, identique ou différent de $R_5$, peut prendre l'une des valeurs indiquées pour $R_5$ et peut également représenter un radical hydroxyle, $R_3$ et $R_4$ identiques ou différents, représentent

soit un atome d'hydrogène,

soit un radical choisi dans le groupe formé par les radicaux OH, $Oalc_4$, $0\text{-}CO\text{-}alc_5$ dans lesquels $alc_4$ et $alc_5$ représentent un radical alkyle renfermant de 1 à 8 atomes de carbone ou aralkyle renfermant de 7 à 15 atomes de carbone,

soit un radical alkényle ou alkynyle renfermant de 2 à 8 atomes de carbone,

soit un radical

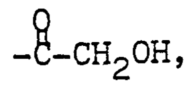

soit un radical $-COCH_2OCOalc_6$, dans lequel $alc_6$ représente un radical alkyle renfermant de 1 à 8 atomes de carbone éventuellement substitué ou un radical aralkyle renfermant de 7 à 15 atomes de carbone,

soit un radical $CO\text{-}CO_2H$,

soit un radical $CO\text{-}CO_2\text{-}alc_7$ dans lequel $alc_7$ représente un radical alkyle renfermant de 1 à 8 atomes de carbone,

soit un radical

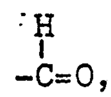

soit un radical

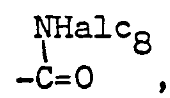

dans lequel $alc_8$ représente un radical alkyle renfermant de 1 à 8 atomes de carbone ou un radical aralkyle renfermant de 7 à 15 atomes de carbone,

soit un radical $-C\equiv N$,

soit $R_3$ et $R_4$ forment ensemble un radical

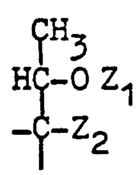

dans lequel $Z_1$ représente un atome d'hydrogène, un radical alkyle ou un radical acyle renfermant de 1 à 8 atomes de carbone et $Z_2$ un radical alkyle renfermant de 1 à 8 atomes de carbone,

soit $R_3$ et $R_4$ forment ensemble un radical

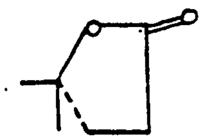

3°) Les produits de formule I telle que définie à la revendication 2 dans laquelle le cycle D ne porte pas d'insaturation, $R_5$ et $R_6$ représentent un atome d'hydrogène et n est égal à 1.

4°) Les produits de formule I telle que définie à l'une quelconque des revendications 1 ou 2 dans laquelle $R_3$ représente un radical OH ou OCO alc$_5$ et $R_4$ représente un radical alkényle ou alkynyle ayant au plus 4 atomes de carbone, alc$_5$ gardant la même signification que dans la revendication 2.

5°) Les produits de formule I telle que définie à l'une quelconque des revendications 1 à 4 dans laquelle $R_1$ représente un radical aryle ou aralkyle portant une fonction amine

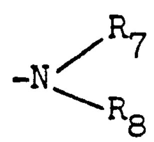

dans laquelle $R_7$ et $R_8$ représentent un radical alkyle renfermant de 1 à 8 atomes de carbone ou un radical alkyle primaire, secondaire ou tertiaire renfermant de 1 à 8 atomes de carbone, comportant un ou plusieurs hétéroatomes choisi dans le groupe constitué par l'oxygène, l'azote, le soufre et le silicium dont au moins un atome d'azote, ou substitué par un hétérocycle comportant au moins un atome d'azote.

6°) Les produits de formule I telle que définie à l'une quelconque des revendications 1 à 4 dans laquelle $R_1$ représente un radical 2,3 ou 4-pyridyle,

un radical

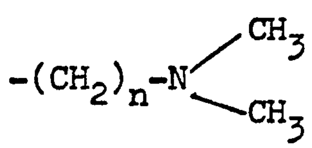 (n ≥3),

un radical:

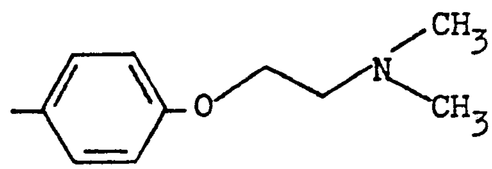 ,

un radical

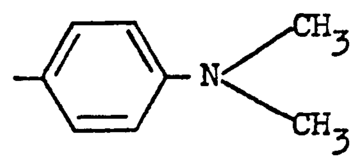

un radical

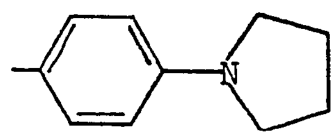

ou un radical

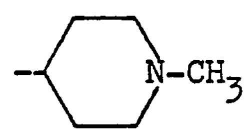

7°, Les produits de formule I telle que définie à l'une quelconque des revendications 1 à 4 dans laquelle $R_1$ représente un radical thényle, furyle, cycloalkyle ayant de 3 à 6 atomes de carbone ou phényle éventuellement substitué par un ou plusieurs radicaux choisis parmi les radicaux hydroxy, halogène, trifluorométhyle, alkyle, alkoxy, alkylthio éventuellement oxydé sous forme de sulfoxyde ou de sulfone, étant entendu que les cycles A et B ne représentent pas le groupement

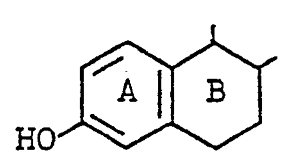

8°) Les produits de formule I telle que définie à l'une quelconque des revendications 1 à 4 et 7 dans laquelle $R_1$ représente un radical phényle substitué par un radical choisi dans le groupe formé par les radicaux chloro, fluoro, méthylthio, méthylsulfonyle, méthoxy, hydroxy et allyloxy.

9°) Les produits de formule générale I telle que définie à l'une quelconque des revendications 1 à 8 dans laquelle les cycles A et B représentent le

groupement

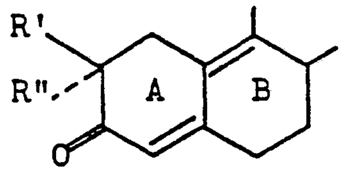

dans lequel R' et R'' sont tels que:
- soit R' et R'' identiques représentent chacun un radical méthyle ou un radical nitrile
- soit l'un représente un atome d'hydrogéne et l'autre représente un radical méthyle ou nitrile.

10°) Les produits de formule générale I telle que définie à l'une quelconque des revendications 1 à 8 dans laquelle les cycles A et B représentent le groupement

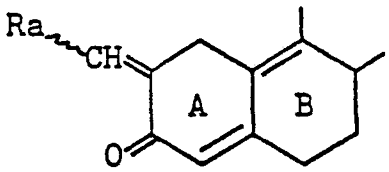

dans lequel Ra représente un radical morpholino ou un radical acétyloxy.

11°) Les produits de formule générale I telle que définie à l'une quelconque des revendications 1 à 9 répondant aux formules suivantes:
- la 2,2-diméthyl 17β-hydroxy 11β-(4-diméthylamino phényl) 17α-(prop-1-ynyl) estra 4,9-dièn-3-one,
- la 2,2-dicyano 11β-(4-diméthylamino phényl) 17β-hydroxy 1817α-(prop-1-ynyl) estra 4,9-dièn-3-one,
- la 2α-méthyl 11β-(4-diméthylamino phényl) 17β-hydroxy 17α-(prop-1-ynyl) estra 4,9-dièn-3-one,
- la 2β-méthyl 11β-(4-diméthylamino phényl) 17β-hydroxy 17α-(prop-1-ynyl) estra 4,9-dièn-3-one,
- la 2-cyano 11β-(4-diméthylamino phényl) 17β-hydroxy 17α-(prop-1-ynyl) estra 4,9-dién-3-one, et leurs sels.

12°) Les produits de formule générale I telle que définie à l'une quelconque des revendications 1 à 8 répondant aux formules suivantes:
- le 11β-(4-diméthylamino phényl) 17α-(prop-1-ynyl)-estra 1,3,5(10) triène 3,17-diol,
- le 17β-acétoxy 11β-(4-diméthylamino phényl) 17α-(prop-1-ynyl estra 1,3,5(10) trièn-3-ol,
- le 11β-(4-diméthylamino phényl) 3-méthoxy 17α-(prop-1-ynyl) estra 1,3,5(10) trièn-17β-ol,
- le 17β-acétoxy 11β-(4-diméthylamino phényl) 3-méthoxy 17α-(prop-1-ynyl) estra 1,3,5(10) triéne et leurs sels.

13°) Les produits de formule générale I telle que définie à l'une quelconque des revendications 1 à 8 répondant aux formules suivantes:
- la 17β-hydroxy 11β-(3-méthoxy phényl) 17α-(prop-1-ynyl) estra 5(10) en-3-one,
- la E 17β-hydroxy 11β-(3-méthoxyphényl) 17α-(prop-1-ényl) estra 5(10) en-3-one
- la 17β-hydroxy 11β-(4-diméthylamino phényl) 17α-(prop-1-ynyl) estr 5(10) en-3-one,
et leurs sels.

14°) Les produits de formule générale I telle que définie à l'une quelconque des revendications 1 à 8 et 10, répondant aux formules suivantes:
- la 2/(acétyloxy)méthylène/ 11β-(4-diméthylamino phényl) 17β-hydroxy 17α-(prop-1-ynyl) estra 4,9-dièn-3-one,
- la 11β-(4-diméthylamino phényl) 17β-hydroxy-2-(4-morpholi-nylméthylène) 17α-(prop-1-ynyl) estra 4,9-dièn-3-one
et leurs sels.

15°) Le produit de formule générale I telle que définie à l'une quelconque des revendications 1 à 8 répondant à la formule:
- le 11β-(4-diméthylaminophényl) 17α-(prop-1-ynyl) isoxazolo /4,5-b/ estra 4,9-dièn-17-ol,
et ses sels.

16°) Les produits de formule générale I telle que définie à l'une quelconque des revendications 1 à 3 répondant aux formules suivantes:
- le 11β-/4-(2-diméthylaminoéthoxy) phényl/ estra 1,3,5(10)triène 3,17β-diol,
- le 11β-/4-(2-diméthylaminoéthoxy) phényl/ estra 1,3,5(10)trièn-17β-ol,
- le 3-(2-diméthylamino) éthoxy 11β-phényl estra 1,3,5(10)trièn-17β-ol,
et leurs sels.

17°) Procédé de préparation des produits de formule générale I telle que définie à la revendication 1 caractérisé en ce que:

a) pour préparer les produits de formule $I_A$

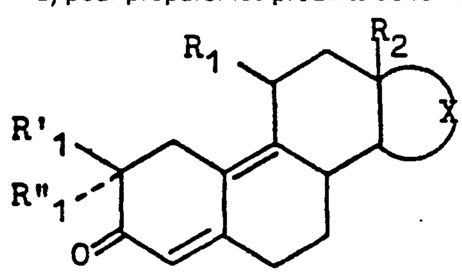

($I_A$)

dans laquelle $R_1$, $R_2$ et X conservent la même signification qu'à la revendication 1 et $R'_1$ et $R''_1$ sont tels que, ou bien $R'_1$ et $R''_1$ représentent chacun un radical alkyle ou bien l'un représente un atome d'hydrogène et l'autre représente un radical alkyle ou bien $R'_1$ et $R''_1$ représentent chacun un radical nitrile ou bien l'un représente un radical alkyle et l'autre un radical nitrile, on soumet un produit de formule II:

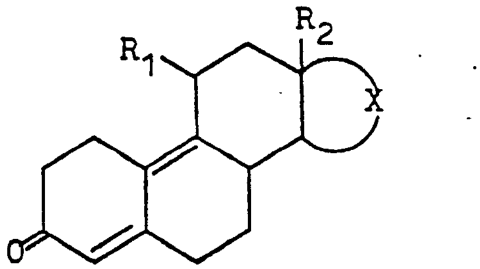

(II)

dans laquelle $R_1$, $R_2$ et X conservent la même signification qu'à la revendication 1, d'abord, éventuellement à l'action d'un réactif de protection des groupements fonctionnels puis à l'action d'une base forte et ou bien d'un halogénure d'alkyle ou bien du cyanure de tosyle, ou bien d'abord à l'action d'un halogénure d'alkyle puis du cyanure de tosyle, puis élimine si nécessaire le groupement protecteur;

b) [1]) pour préparer les produits de formule $I_B$:

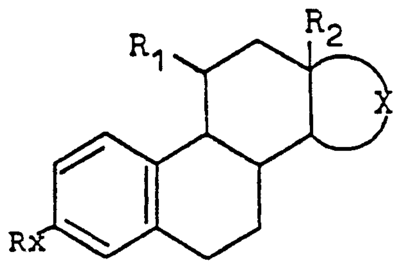

($I_B$)

répondant à la formule $IB_1$:

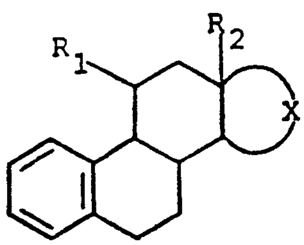

($IB_1$)

dans laquelle $R_1$, $R_2$ et X conservent la même signification qu'a la revendication 1, on soumet un produit de formule II à l'action d'un agent réducteur, pour obtenir un produit de formule $II_1$:

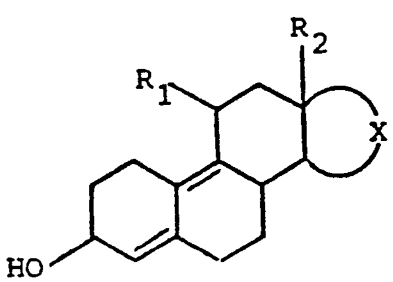

($II_1$)

dans laquelle $R_1$, $R_2$ et X conservent la même signification que ci-dessus, que l'on soumet à l'action d'un agent d'aromatisation acide;

b) 2) pour préparer les produits de formule $I_B$ répondant à la formule $IB_2$:

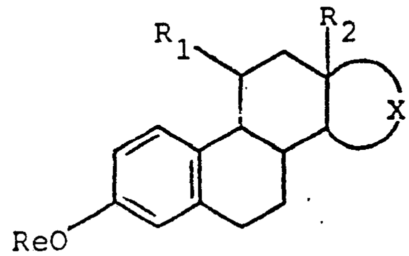

($IB_2$)

dans laquelle $R_1$, $R_2$, Re et X conservent la même signification que ci-dessus, on soumet un produit de formule II à l'action d'un agent d'aromatisation puis de saponification, puis éventuellement à un réactif d'alkylation ou d'acylation;

c) pour préparer les produits de formule $I_C$:

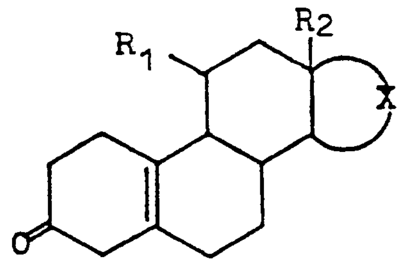

($I_C$)

dans laquelle $R_1$, $R_2$ et X conservent la même signification qu'à la revendication 1, on soumet un produit de formule II à l'action d'un agent réducteur;

d) pour préparer les produits de formule $I_D$:

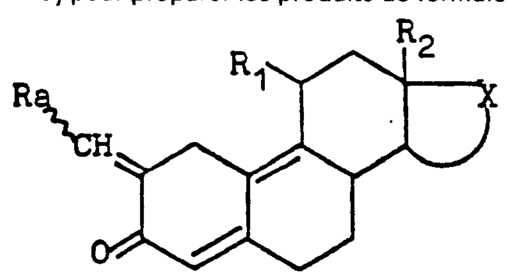

($I_D$)

dans laquelle $R_1$, $R_2$, X et Ra conservent la même signification qu'à la revendication 1, on soumet un produit de formule II à l'action d'un réactif de formylation pour obtenir un produit de formule III

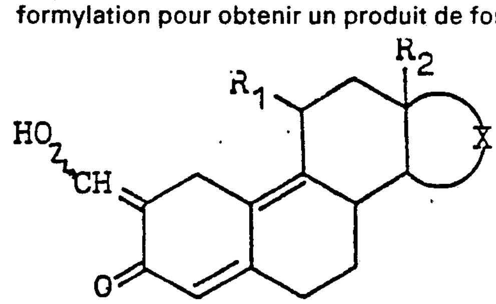

(III)

produit de formule III que, ou bien l'on fait réagir avec un réactif d'acylation, ou bien l'on fait réagir avec une amine de formule

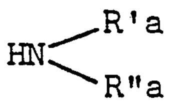

dans laquelle R'a et R''a conservent la même signification qu'à la revendication 1;

e) pour préparer les produits de formule $I_E$:

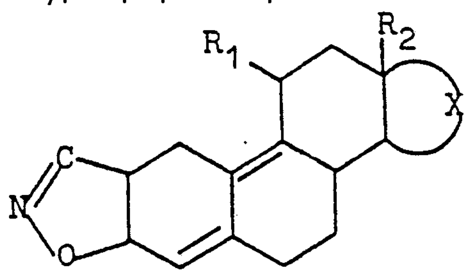

($I_E$)

dans laquelle $R_1$, $R_2$ et X conservent la même signification qu,à la revendication 1, on fait agir l'hydroxylamine sur un produit de formule III;

f) pour préparer les produits de formule $I,_A$

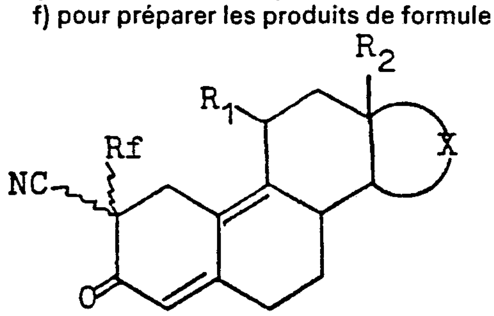

($I'_A$)

dans laquelle Rf représente un atome d'hydrogène ou un radical alkyle et $R_1$, $R_2$ et X conservent la même signification qu'à la revendication 1, on fait agir une base sur les produits de formule $I_E$ pour obtenir les produits de formule $I'_A$ dans laquelle Rf représente un atome d'hydrogène et si désiré l'on soumet les produits ainsi obtenus à l'action d'une base forte et d'un halogénure d'alkyle pour obtenir les produits de formule $I'_A$ dans laquelle Rf représente un radical alkyle.

18°) Procédé selon la revendication 17 de préparation des produits de formule I telle que définie aux revendications 4 et 6 caractérisé en ce que l'on met en oeuvre le procédé décrit à la revendication 17 au départ de produits de formule II dans laquelle le substituant

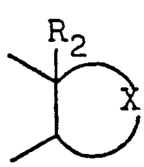

représente un radical

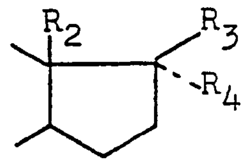

dans lequel $R_2$ représente un radical méthyle, $R_3$ représente un radical OH ou

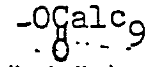

$alc_9$ étant un radical alkyle ayant de 1 à 4 atomes de carbone, $R_4$ représente un radical alkényle ou alkynyle ayant de 2 à 4 atomes de carbone et $R_1$ représente un radical phényle substitué par un radical-$OAlc_{10}$, $Alc_{10}$ étant un radical alkyle ayant de 1 à 4 atomes de carbone, ou

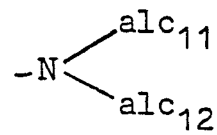

$alc_{11}$ et $alc_{12}$ étant des radicaux alkyles ayant de 1 à 4 atomes de carbone.

19°) A titre de médicaments, les produits de formule I telle que définie à l'une des revendications 1 ou 2, pharmaceutiquement acceptables, ainsi que leurs sels d'addition avec les acides pharmaceutiquement acceptables.

20°) A titre de médicaments, les produits de formule I telle que définie à l'une quelconque des revendications 3 à 15, pharmaceutiquement acceptables, ainsi que leurs sels d'addition pharmaceutiquement acceptables.

21°) A titre de médicaments, les produits de formule I telle que définie à la revendication 16, pharmaceutiquement acceptables, ainsi que leurs sels d'addition avec les acides pharmaceutiquement acceptables.

22°) Compositions pharmaceutiques renfermant comme principe actif, un au moins des médicaments selon l'une quelconque des revendications 19 à 21.

23°) A titre de produits industriels nouveaux, les produits de formule générale III:

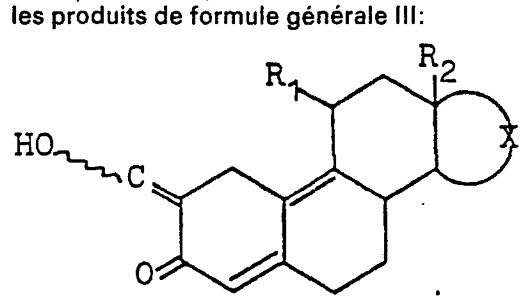

(III)

dans laquelle $R_1$, $R_2$ et X conservent la même signification qu'à la revendication 1.

**Revendications**

pour l'Etat contractant AT

1.- Procédé pour préparer les produits de formule générale (I):

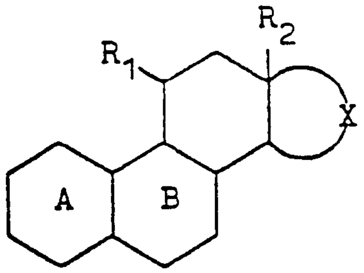

(I)

dans laquelle $R_1$ représente un radical organique renfermant de 1 à 18 atomes de carbone et éventuellement un ou plusieurs hétéroatomes, l'atome immédiatement adjacent au carbone en 11 étant un atome de carbone, $R_2$ représente un radical hydrocarboné renfermant de 1 à 8 atomes de carbone, X représente le reste d'un cycle pentagonal ou hexagonal éventuellement substitué et éventuellement porteur d'une insaturation, les cycles A et B ayant l'une des structures suivantes: a) - Soit A et B représentent le groupement:

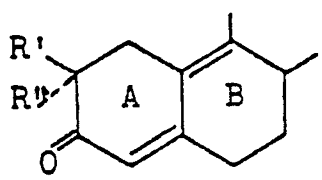

dans lequel R' et R'' identiques ou différents représentent un atome d'hydrogène, un radical nitrile ou un radical alkyle ayant de 1 à 4 atomes de carbone étant entendu que l'un au moins des radicaux R' ou R'' ne représente pas un atome d'hydrogène;

b) - Soit A et B représentent le groupement:

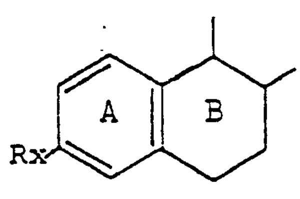

Rx représentant un atome d'hydrogène ou un groupement -ORe, dans lequel Re représente un atome d'hydrogène, un radical alkyle ayant de 1 à 6 atomes de carbone éventuellement substitué ou un radical acyle;

c) - Soit A et B représentent le groupement :

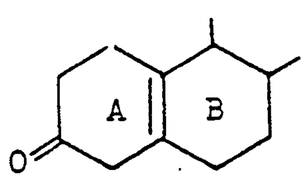

d) - Soit A et B représentent le groupement

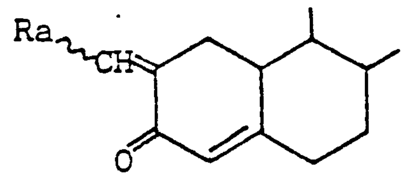

dans lequel Ra représente un radical

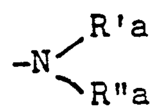

dans lequel R'a et R''a représentent soit un radical alkyle ayant de 1 à 4 atomes de carbone, soit R'a et R''a représentent avec l'atome d'azote auquel ils sont liés, un hétérocycle à 5 ou 6 chaînons comportant éventuellement un autre hétéro atome, ou Ra représente un radical acyloxy, le trait ondulé signifiant que Ra peut se trouver dans la position E ou Z,

e) - Soit A et B représentent le groupement

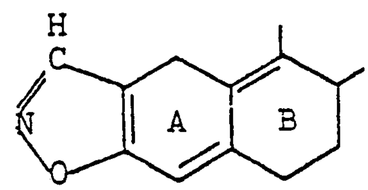

étant entendu que lorsque A et B représentent le groupement

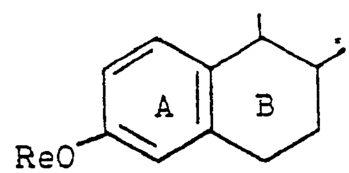

le radical $R_1$ contient au moins un atome d'azote, de phosphore ou de silicium, et que lorsque A et B représentent le groupement

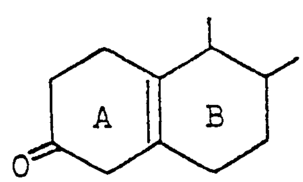

le radical $R_1$ ne représente pas un radical alkyle saturé linéaire, ainsi que les sels d'addition des produits de formule I avec les acides, caractérisé en ce que:

a) pour préparer les produits de formule $I_A$

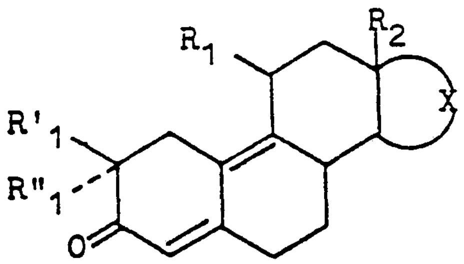

$(I_A)$

dans laquelle $R_1$, $R_2$ et X conservent la même signification que précédemment et $R'_1$ et $R''_1$ sont tels que, ou bien $R'_1$ et $R''_1$ représentent chacun un radical alkyle ou bien l'un représente un atome d'hydrogène et l'autre représente un radical alkyle ou bien $R'_1$ et $R''_1$ représentent chacun un radical nitrile ou bien l'un représente un radical alkyle et l'autre un radical nitrile, on soumet un produit de formule

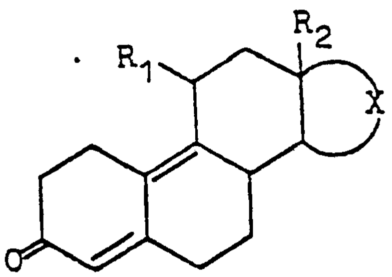

(II)

dans laquelle $R_1$, $R_2$ et X conservent la même signification que précédemment, d'abord, éventuellement à l'action d'un réactif de protection des groupements fonctionnels puis à l'action d'une base forte et ou bien d'un halogénure d'alkyle ou bien du cyanure de tosyle, ou bien d'abord à l'action d'un halogénure d'alkyle puis du cyanure de tosyle, puis élimine si nécessaire le groupement protecteur;

b) 1) pour préparer les produits de formule $I_B$:

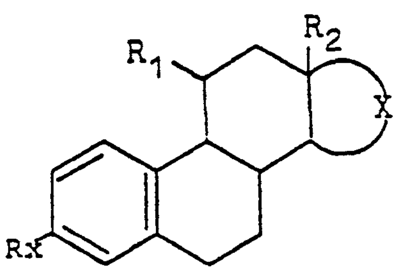

$(I_B)$

répondant à la formule $IB_1$:

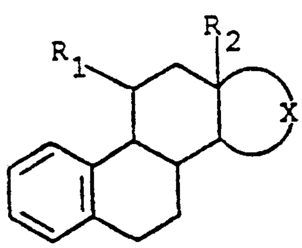

$(IB_1)$

dans laquelle $R_1$, $R_2$ et x conservent la même signification que précédemment on soumet un produit de formule II à l'action d'un agent réducteur, pour obtenir un produit de formule $II_1$:

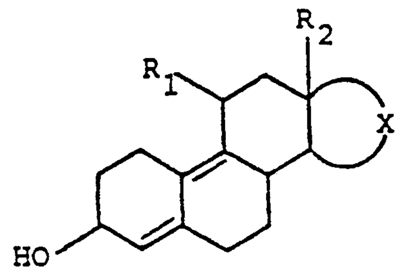

$(II_1)$

dans laquelle $R_1$, $R_2$ et X conservent la même signification que ci-dessus, que l'on soumet à l'action d'un agent d'aromatisation acide;

b) 2) pour préparer les produits de formule $I_B$ répondant à la formule $IB_2$:

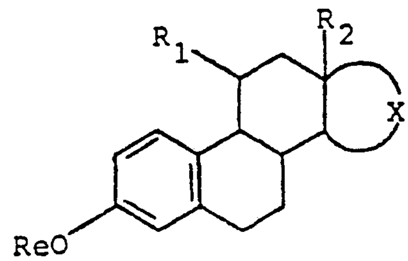

$(IB_2)$

dans laquelle $R_1$, $R_2$, Re et X conservent la même signification que ci-dessus, on soumet un produit de formule II à l'action d'un agent d'aromatisation puis de saponification, puis éventuellement à un réactif d'alkylation ou d'acylation;

c) pour préparer les produits de formule $I_C$:

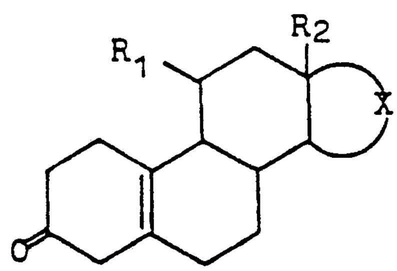

$(I_C)$

dans laquelle $R_1$, $R_2$ et X conservent la même signification que précédemment on soumet un produit de formule II à l'action d'un agent réducteur;

d) pour préparer les produits de formule $I_D$:

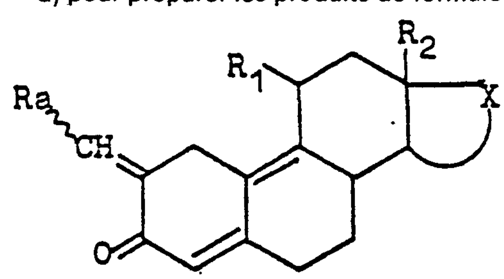

$(I_D)$

dans laquelle $R_1$, $R_2$, X et Ra conservent la même signification que précédemment on soumet un produit de formule II à l'action d'un reactif de formylation pour obtenir un produit de formule III

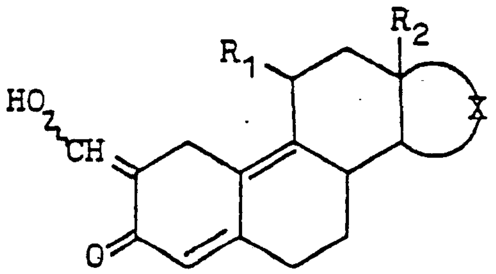

(III)

produit de formule III que, ou bien l'on fait réagir avec un réactif d'acylation, ou bien l'on fait réagir avec une amine de formule

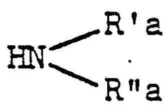

dans laquelle R'a et R''a conservent la même signification que précédemment;

e) pour préparer les produits de formule $I_E$:

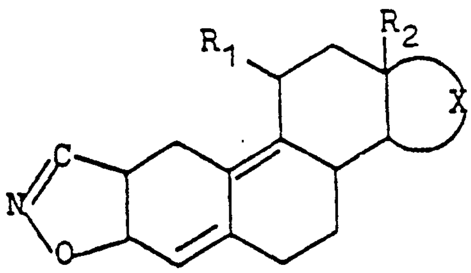

$(I_E)$

dans laquelle $R_1$, $R_2$ et X conservent la même signification que précédemment on fait agir l'hydroxylamine sur un produit de formule III;

f) pour préparer les produits de formule $I,_A$

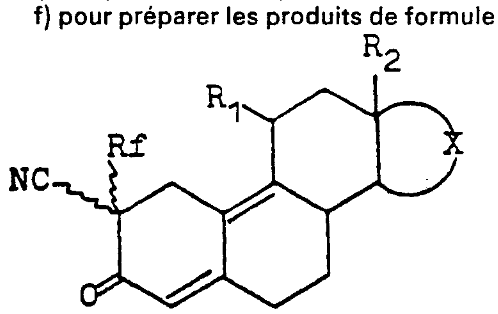

$(I'_A)$

dans laquelle Rf représente un atome d'hydrogène ou un radical alkyle et $R_1$, $R_2$ et X conservent la même signification que précédemment, on fait agir une base sur les produits de formule $I_E$ pour obtenir les produits de formule $I'_A$ dans laquelle Rf représente un atome d'hydrogène et si désiré l'on soumet les produits ainsi obtenus à l'action d'une base forte et d'un halogénure d'alkyle pour obtenir les produits de formule $I'_A$ dans laquelle Rf représente un radical alkyle.

2.- Procédé selon la revendication 1, caractérisé en ce que l'on met en oeuvre ledit procédé, au départ de produits de formule (II) dans laquelle X représente le reste d'un cycle de formule

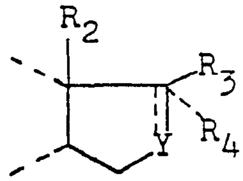

dans lequel $R_2$ conserve la même signification que dans la revendication 1, le trait pointillé en 16-17 symbolise la présence éventuelle d'une double liaison, Y représente un radical

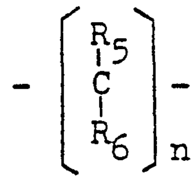

dans lequel n représente le nombre 1 ou 2, $R_5$ représente un atome d'hydrogène, un radical alkyle renfermant de 1 à 8 atomes de carbone, un radical alkényle ou alkynyle renfermant de 2 à 8 atomes de carbone, un radical aryle renfermant de 6 à 14 atomes de carbone, ou un radical aralkyle renfermant de 7 à 15 atomes de carbone, $R_6$, identique ou différent de $R_5$ peut prendre l'une des valeurs indiquées pour $R_5$ et peut également représenter un radical hydroxyle, $R_3$ et $R_4$ identiques ou différents, représentent

soit un atome d'hydrogène,

soit un radical choisi dans le groupe formé par les radicaux OH, $Oalc_4$, $O-CO-alc_5$ dans lesquels $alc_4$ et $alc_5$ représentent un radical alkyle renfermant de 1 à 8 atomes de carbone ou aralkyle renfermant de 7 à 15 atomes de carbone,

soit un radical alkényle ou alkynyle renfermant de 2 à 8 atomes de carbone,

soit un radical

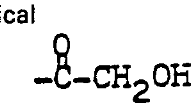

soit un radical $-COCH_2OCOalc_6$, dans lequel $alc_6$ représente un radical alkyle renfermant de 1 à 8 atomes de carbone éventuellement substitué ou un radical aralkyle renfermant de 7 à 15 atomes de carbone,

soit un radical $CO-CO_2H$,

soit un radical $CO-CO_2-alc_7$ dans lequel $alc_7$ représente un radical alkyle renfermant de 1 à 8 atomes de carbone,

soit un radical 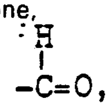

soit un radical 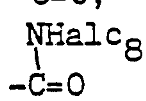 , dans lequel $alc_8$ représente un radical alkyle renfermant de 1 à 8 atomes de carbone ou un radical aralkyle renfermant de 7 à 15 atomes de carbone,

soit un radical $-C\equiv N$,

soit $R_3$ et $R_4$ forment ensemble un radical

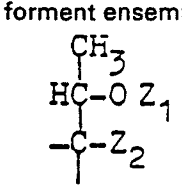

dans lequel $Z_1$ représente un atome d'hydrogène, un radical alkyle ou un radical acyle renfermant de 1 à 8 atomes de carbone et $Z_2$ un radical alkyle renfermant de 1 à 8 atomes de carbone,
soit $R_3$ et $R_4$ forment ensemble un radical

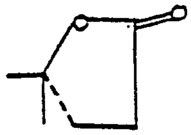

3.- Procédé selon la revendication 2, caractérisé en ce que l'on met en oeuvre ledit procédé, au départ de produits de formule (II) dans laquelle le cycle D ne porte pas d'insaturation, $R_5$ et $R_6$ représentent un atome d'hydrogène et n est égal à 1.

4.- Procédé selon la revendication 2, caractérisé en ce que l'on met en oeuvre ledit procédé, au départ de produits de formule (II) dans laquelle $R_3$ représente un radical OH ou OCO $alc_5$ et $R_4$ représente un radical alkényle ou alkynyle ayant au plus 4 atomes de carbone, $alc_5$ gardant la même signification que dans la revendication 2.

5.- Procédé selon l'une quelconque des revendications 1 à 4, caractérisé en ce que l'on met en oeuvre ledit procédé, au départ de produits de formule (II) dans laquelle $R_1$ représente un radical aryle ou aralkyle portant une fonction amine

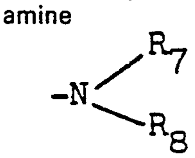

dans laquelle $R_7$ et $R_8$ représentent un radical alkyle renfermant de 1 à 8 atomes de carbone ou un radical alkyle primaire, secondaire ou tertiaire renfermant de 1 à 8 atomes de carbone, comportant un ou plusieurs hétéroatomes choisi dans le groupe constitué par l'oxygène, l'azote et le soufre, dont au moins un atome d'azote, ou substitué par un hétérocycle comportant au moins un atome d'azote.

6.- Procédé selon l'une quelconque des revendications 1 à 4, caractérisé en ce que l'on met en oeuvre ledit procédé, au départ de produits de formule (II) dans laquelle $R_1$ représente un radical 2,3 ou 4-pyridyle,
un radical

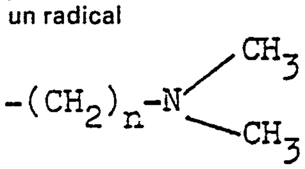 $(n \geq 3)$,

un radical:

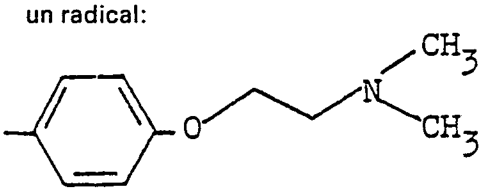 ,

un radical

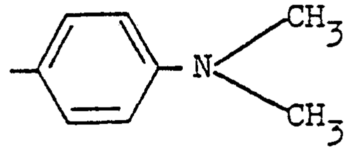

un radical

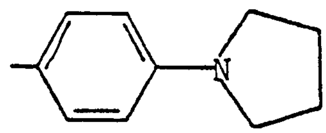

ou un radical

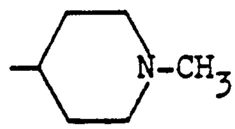

7.- Procédé selon l'une quelconque des revendications 1 à 4, caractérisé en ce que l'on met en oeuvre ledit procédé, au départ de produits de formule (II) dans laquelle $R_1$ représente un radical thiényle, furyle, cycloalkyle ayant de 3 à 6 atomes de carbone ou phényle éventuellement substitué par un ou plusieurs radicaux choisis parmi les radicaux hydroxy, halogène, trifluorométhyle, alkyle, alkoxy, alkylthio éventuellement oxydé sous forme de sulfoxyde ou de sulfone, étant entendu que les cycles A et B ne représentent pas le groupement

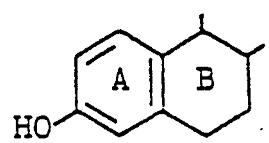

8.- Procédé selon l'une quelconque des revendications 1 à 4, caractérisé en ce que l'on met en oeuvre ledit procédé, au départ de produits de formule (II) dans laquelle $R_1$ représente un radical phényle substitué par un radical choisi dans le groupe formé par les radicaux chloro, fluoro, méthylthio, méthylsulfonyle, méthoxy, hydroxy et allyloxy.

9.- Procédé selon l'une quelconque des revendications 1 à 8, caractérisé en ce que l'on met en oeuvre ledit procédé, au départ de produits de formule (II) dans laquelle les cycles A et B représentent le groupement

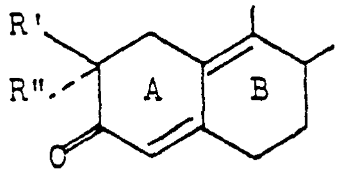

dans lequel R' et R'' sont tels que:

- soit R' et R'' identiques représentent chacun un radical méthyle ou un radical nitrile
- soit l'un représente un atome d'hydrogène et l'autre représente un radical méthyle ou nitrile.

10.- Procédé selon l'une quelconque des revendications 1 à 8, caractérisé en ce que l'on met en oeuvre ledit procédé, au départ de produits de formule (II) dans laquelle les cycles A et B représentent le groupement

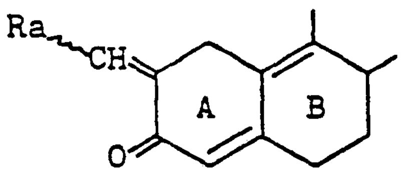

dans lequel Ra représente un radical morpholino ou un radical acétyloxy.

11.- Procédé selon la revendication 1, caractérisé en ce que l'on met en oeuvre ledit procédé, au départ de produits de formule (II) dans laquelle le substituant

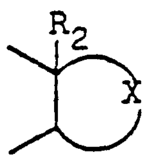

représente un radical

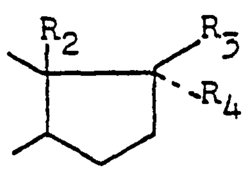

dans lequel $R_2$ représente un radical méthyle, $R_3$ représente un radical OH ou

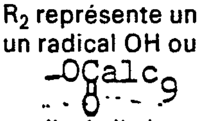 ,

$alc_9$ étant un radical alkyle ayant de 1 à 4 atomes de carbone, $R_4$ représente un radical alkényle ou alkynyle ayant de 2 à 4 atomes de carbone et $R_1$ représente un radical phényle substitué par un radical-$OAlc_{10}$, $Alc_{10}$ étant un radical alkyle ayant de 1 à 4 atomes de carbone,

ou 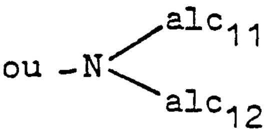

$alc_{11}$ et $alc_{12}$ étant des radicaux alkyles ayant de 1 à 4 atomes de carbone.

12.- Procédé selon l'une----- quelconque des revendications 1 à 9, caractérisé en ce que l'on prépare l'un quelconque des produits répondant aux formules suivantes:

- la 2,2-diméthyl 17β-hydroxy 11β-(4-diméthylamino phényl) 17α-(prop-1-ynyl) estra 4,9-dièn-3-one,
- la 2,2-dicyano 11β-(4-diméthylamino phényl) 17β-hydroxy 17α-(prop-1-ynyl) estra 4,9-dièn-3-one,
- la 2α-méthyl 11β-(4-diméthylamino phényl) 17β-hydroxy 17α-(prop-1-ynyl) estra 4,9-dièn-3-one,
- la 2β-méthyl 11β-(4-diméthylamino phényl) 17β-hydroxy 17α-(prop-1-ynyl) estra 4,9-dièn-3-one,
- la 2-cyano 11β-(4-diméthylamino phényl) 17β-hydroxy 17α-(prop-1-ynyl) estra 4,9-dièn-3-one, et leurs sels.

13.- Procédé selon l'une quelconque des revendications 1 à 8, caractérisé en ce que l'on prépare l'un quelconque des produits répondant aux formules suivantes:

- le 11β-(4-diméthylamino phényl) 17α-(prop-1-ynyl) estra 1,3,5(10) triène 3,17-diol,
- le 17β-acétoxy 11β-(4-diméthylamino phényl) 17α-(prop-1-ynyl estra 1,3,5(10) trièn-3-ol,
- le 11β-(4-diméthylamino phényl) 3-méthoxy 17α-(prop-1-ynyl) estra 1,3,5(10) trièn-17β-ol,
- le 17β-acétoxy 11β-(4-diméthylamino phényl) 3-méthoxy 17α-(prop-1-ynyl) estra 1,3,5(10) triène

et leurs sels.

14.- Procédé selon l'une quelconque des revendications 1 à 8, caractérisé en ce que l'on prépare l'un quelconque des produits répondant aux formules suivantes:

- la 17β-hydroxy 11β-(3-méthoxy phényl) 17α-(prop-1-ynyl) estra 5(10) en-3-one,
- la E 17β-hydroxy 11β-(3-méthoxyphényl) 17α-(prop-1-ényl) estra 5(10) en-3-one,
- la 17β-hydroxy 11β-(4-diméthylamino phényl) 17α-(prop-1-ynyl) estr 5(10) en-3-one,

et leurs sels.

15.- Procédé selon l'une quelconque des revendications 1 à 8 et 10, caractérisé en ce que l'on prépare l'un quelconque des produits répondant aux formules suivantes:

- la 2/(acétyloxy)méthylène/ 17β-(4-diméthylamino phényl) 17β-hydroxy 17α-(prop-1-ynyl) estra 4,9-dièn-3-one,
- la 11β-(4-diméthylamino phényl) 17β-hydroxy-2-(4-morpholi-nylméthylène) 17α-(prop-1-ynyl) estra 4,9-dièn-3-one

et leurs sels.

16.- Procédé selon l'une quelconque des revendications 1 à 8, caractérisé en ce que l'on prépare le 11β-(4-diméthylaminophényl) 17α-

(prop-1-ynyl) isoxazolo /4,5-b/ estra 4,9-dièn-17-ol et ses sels.

17.- Procédé selon l'une quelconque des revendications 1 è 3, caractérisé en ce que l'on prépare l'un quelconque des produits répondant aux formules suivantes:

- le 11β-/4-(2-diméthylaminoéthoxy) phényl/ estra 1,3,5(10)triène 3,17β-diol,
- le 11β-/4-(2-diméthylaminoéthoxy) phényl/ estra 1,3,5(10)trièn-17β-ol,
- le 3-(2-diméthylamino) éthoxy 1β-phényl estra 1,3,5(10)trien-17β-ol,

et leurs sels.

**Patentansprüche**

für die Vertragsstaaten: BE, CH, DE, FR, GB, IT, LI, LU, NL, SE

1. Produkte der allgemeinen Formel I

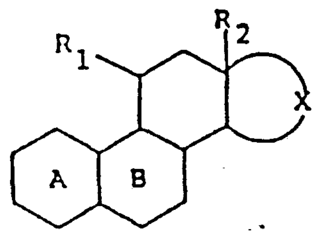

(I)

worin $R_1$ einen organischen Rest mit 1 bis 18 Kohlenstoffatomen und gegebenenfalls einem oder mehreren Heteroatomen bedeutet, wobei das dem Kohlenstoffatom in 11-Stellung unmittelbar benachbarte Atom ein Kohlenstoffatom ist, $R_2$ einen Kohlenwasserstoffrest mit 1 bis 8 Kohlenstoffatomen bedeutet, X den Rest eines pentagonalen oder hexagonalen Ringes, der gegebenenfalls substituiert ist und gegebenenfalls eine Unsättigung enthält, bedeutet, wobei die Ringe A und B eine der folgenden Strukturen besitzen:

(a) entweder bedeuten A und B die Gruppe

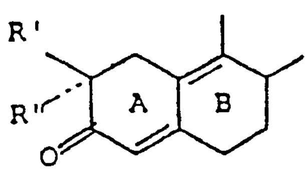

worin R' und R'', die gleich oder verschieden sind, ein Wasserstoffatom, einen Nitrilrest oder einen Alkylrest mit 1 bis 4 Kohlenstoffatomen bedeuten, wobei zumindest einer der Reste R' oder R'' kein Wasserstoffatom bedeutet;

(b) oder A und B bedeuten die Gruppe

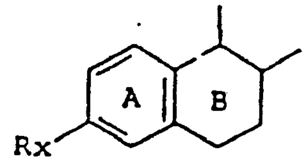

wobei Rx ein Wasserstoffatom oder eine Gruppe -ORe darstellt, worin Re ein Wasserstoffatom, einen Alkylrest mit 1 bis 6 Kohlenstoffatomen, der gegebenenfalls substituiert ist, oder einen Acylrest bedeutet;

(c) oder A und B stellen die Gruppe

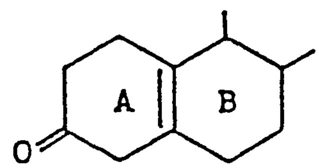

dar;

(d) oder A und B bedeuten die Gruppe

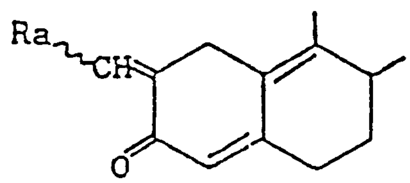

worin Ra einen Rest

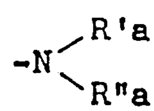

bedeutet, worin R'a und R''a entweder einen Alkylrest mit 1 bis 4 Kohlenstoffatomen bedeuten oder R'a und R''a mit dem Stickstoffatom, an das sie gebunden sind, einen Heterocyclus mit 5 oder 6 Kettengliedern bilden, der gegebenenfalls ein weiteres Heteroatom enthält, oder Ra einen Acyloxyrest bedeutet, wobei die gewellte Linie anzeigt, daß sich Ra in der Eoder Z-Position befinden kann;

(e) oder A und B bedeuten die Gruppe

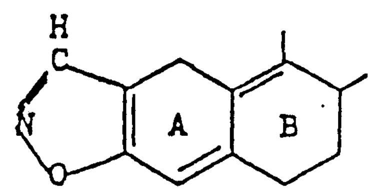

wobei, wenn A und B die Gruppe

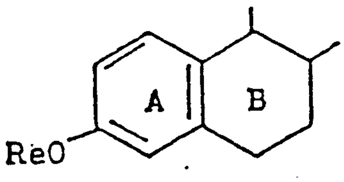

darstellen, der Rest $R_1$ zumindest ein Stickstoff-, Phosphor- oder Siliciumatom enthält, und wenn A und B die Gruppe

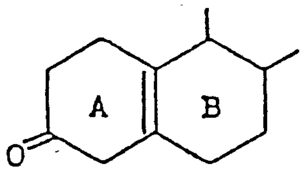

darstellen, der Rest $R_1$ keinen linearen, gesättigten Alkylrest bedeutet,

sowie die Additionssalze der Produkte der Formel I mit Säuren.

2. Produkte der Formel I gemäß Anspruch 1, worin X den Rest eines Rings der Formel

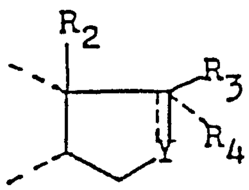

darstellt, worin $R_2$ die in Anspruch 1 angegebene Bedeutung besitzt, die gestrichelte Linie in 16-17-Stellung die etwaige Anwesenheit einer Doppelbindung anzeigt, Y einen Rest

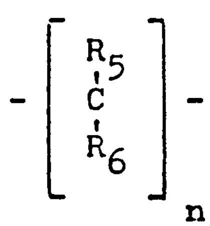

bedeutet, worin n die Zahl 1 oder 2 darstellt, $R_5$ ein Wasserstoffatom, einen Alkylrest mit 1 bis 8 Kohlenstoffatomen, einen Alkenylrest oder Alkinylrest mit 2 bis 8 Kohlenstoffatomen, einen Arylrest mit 6 bis 14 Kohlenstoffatomen oder einen Aralkylrest mit 7 bis 15 Kohlenstoffatomen darstellt, $R_6$, das mit $R_5$ identisch oder von diesem verschieden ist, eine der für $R_5$ angegebenen Bedeutungen besitzen kann und auch einen Hydroxylrest darstellen kann, $R_3$ und $R_4$ die gleich oder verschieden sind,

entweder ein Wasserstoffatom

oder einen Rest, ausgewählt unter den Resten OH, $Oalc_4$, O-CO-$alc_5$, worin $alc_4$ und $alc_5$ einen Alkylrest mit 1 bis 8 Kohlenstoffatomen oder einen Aralkylrest mit 7 bis 15 Kohlenstoffatomen darstellen,

oder einen Alkenyl- oder Alkinylrest mit 2 bis 8 Kohlenstoffatomen

oder einen Rest 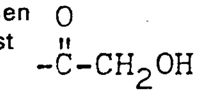

oder einen Rest $-COCH_2OCOalc_6$, worin $alc_6$ einen Alkylrest mit 1 bis 8 Kohlenstoffatomen, der gegebenenfalls substituiert ist, oder einen Aralkylrest mit 7 bis 15 Kohlenstoffatomen bedeutet,

oder einen Rest $CO-CO_2H$

oder einen Rest $CO-CO_2-alc_7$, worin $alc_7$ einen Alkylrest mit 1 bis 8 Kohlenstoffatomen bedeutet,

oder einen Rest 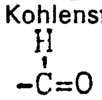

oder einen Rest 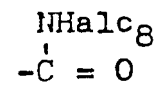,

worin $alc_8$ einen Alkylrest mit 1 bis 8 Kohlenstoffatomen oder einen Aralkylrest mit 7 bis 15 Kohlenstoffatomen bedeutet,

oder einen Rest $-C\equiv N$ darstellen

oder $R_3$ und $R_4$ gemeinsam einen Rest

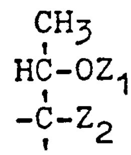

bilden, worin $Z_1$ ein Wasserstoffatom, einen Alkylrest oder einen Acylrest mit 1 bis 8 Kohlenstoffatomen darstellt und $Z_2$ einen Alkylrest mit 1 bis 8 Kohlenstoffatomen bedeutet,

oder $R_3$ und $R_4$ gemeinsam einen Rest

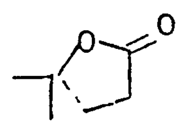

bilden.

3. Die Produkte der Formel I gemäß Anspruch 2, worin der Ring D keine Unsättigung enthält, $R_5$ und $R_6$ ein Wasserstoffatom bedeuten und n für 1 steht.

4. Die Produkte der Formel I gemäß einem der Ansprüche 1 oder 2, worin $R_3$ einen Rest OH oder $OCOalc_5$ bedeutet und $R_4$ einen Alkenyl- oder Alkinylrest mit höchstens 4 Kohlenstoffatomen darstellt, wobei $alc_5$ die in Anspruch 2 angegebene Bedeutung besitzt.

5. Die Produkte der Formel I gemäß einem der Ansprüche 1 bis 4, worin $R_1$ einen Aryl- oder Aralkylrest mit einer Aminfunktion

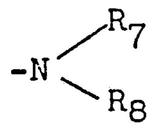

darstellt, worin $R_7$ und $R_8$ einen Alkylrest mit 1 bis 8 Kohlenstoffatomen oder einen primären, sekundären oder tertiären Alkylrest mit 1 bis 8 Kohlenstoffatomen darstellen, der ein oder mehrere Heteroatome, ausgewählt unter Sauerstoff, Stickstoff, Schwefel und Silicium, enthält, von denen zumindest eines ein Stickstoffatom ist, oder der substituiert ist durch einen Heterocyclus, der zumindest ein Stickstoffatom enthält.

6. Die Produkte der Formel I gemäß einem der Ansprüche 1 bis 4, worin $R_1$ einen 2-, 3- oder 4-Pyridylrest,

einen Rest

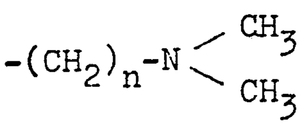 $(n \geqslant 3)$

einen Rest

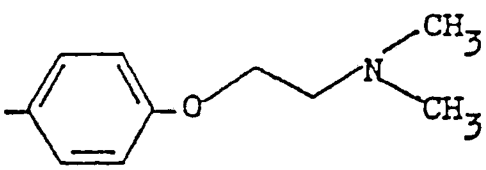

einen Rest

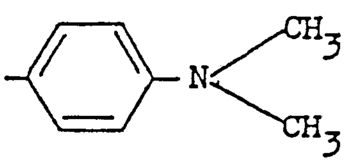

einen Rest

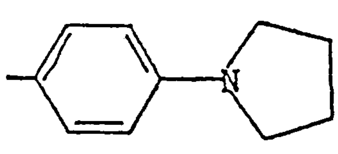

oder einen Rest

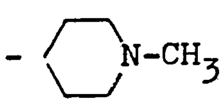

bedeutet.

7. Die Produkte der Formel I gemäß einem der Ansprüche 1 bis 4, worin $R_1$ einen Thienyl-, Furyl-, Cycloalkylrest mit 3 bis 6 Kohlenstoffatomen oder einen Phenylrest bedeutet, der gegebenenfalls durch einen oder mehrere Reste, ausgewählt unter den Hydroxy-, Halogen-,Trifluormethyl-, Alkyl-, Alkoxy-, in Form des Sulfoxids oder Sulfons gegebenenfalls oxidierten Alkylthioresten, substituiert ist, wobei die Ringe A und B nicht die Gruppe

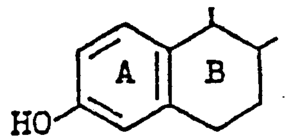

darstellen.

8. Die Produkte der Formel I gemäß einem der Ansprüche 1 bis 4 und 7, worin $R_1$ einen Phenylrest bedeutet, der durch einen Rest substituiert ist, ausgewählt unter den Chlor-, Fluor-, Methylthio-, Methylsulfonyl-, Methoxy-, Hydroxy- und Allyloxyresten.

9. Die Produkte der allgemeinen Formel I gemäß einem der Ansprüche 1 bis 8, worin die Ringe A und B die Gruppe

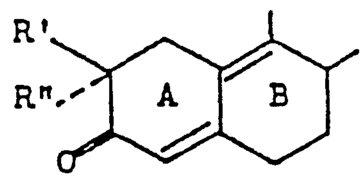

darstellen, worin R' und R" wie folgt sind:

entweder bedeuten R' und R", die identisch sind, jeweils einen Methyl- oder einen Nitrilrest

oder einer von ihnen bedeutet ein Wasserstoffatom und der andere bedeutet einen Methyl- oder Nitrilrest.

10. Die Produkte der allgemeinen Formel I gemäß einem der Ansprüche 1 bis 8, worin die Ringe A und B die Gruppe

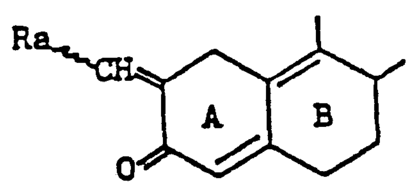

bedeuten, worin Ra einen Morpholino- oder einen Acetyloxyrest darstellt.

11. Die Produkte der allgemeinen Formel I gemäß einem der Ansprüche 1 bis 9 mit den folgenden Bezeichnungen:

2,2-Dimethyl-17β-hydroxy-11β-(4-dimethylamino-phenyl)-17α-(prop-1-inyl)-östra-4,9-dien-3-on,

2,2-Dicyano-11β-(4-dimethylaminophenyl)-17β-hydro-xy-17α-(prop-1-inyl)-östra-4,9-dien-3-on,

2α-Methyl-11β-(4-dimethylaminophenyl)-17β-hydro-xy-17α-(prop-1-inyl)-östra-4,9-dien-3-on,
2β-Methyl-11β-(4-dimethylaminophenyl)-17β-hydro-xy-17α-(prop-1-inyl)-östra-4,9-dien-3-on,
2-Cyano-11β-(4-dimethylaminophenyl)-17β-hydroxy-17α-(prop-1-inyl)-östra-4,9-dien-3-on
sowie ihre Salze.

12. Die Produkte der allgemeinen Formel I gemäß einem der Ansprüche 1 bis 8 mit den folgenden Bezeichnungen:
11β-(4-Dimethylaminophenyl)-17α-(prop-1-inyl)-östra-1,3,5(10)-trien-3,17-diol,
l7β-Acetoxy-11ß-(4-dimethylaminophenyl)-17α-(prop-1-inyl)-östra-1,3,5(10)-trien-3-ol,
11β-(4-Dimethylaminophenyl)-3-methoxy-17α-(prop-1-inyl)-östra-1,3,5(10)-trien-17β-ol,
17β-Acetoxy-11β-(4-dimethylaminophenyl)-3-meth-oxy-17α-(prop-1-inyl)-östra-1,3,5(10)-trien
und ihre Salze.

13. Die Produkte der allgemeinen Formel I gemäß einem der Ansprüche 1 bis 8 mit den folgenden Bezeichnungen:
17β-Hydroxy-11β-(3-methoxyphenyl)-17α-(prop-1-inyl)-östra-5(10)-en-3-on,
E-17β-Hydroxy-11β-(3-methoxyphenyl)-17α-(prop-1-enyl)-östra-5(10)-en-3-on,
17β-Hydroxy-11β-(4-dimethylaminophenyl)-17α-(prop-1-inyl)-östr-5(10)-en-3-on
und ihre Salze.

14. Die Produkte der allgemeinen Formel I gemäß einem der Ansprüche 1 bis 8 und 10 mit den folgenden Bezeichnungen:
2-[(Acetyloxy)-methylen]-11β-(4-dimethylaminophe-nyl)-17β-hydroxy-17α-(prop-1-inyl)-östra-4,9-dien-3-on,
11β-(4-Dimethylaminophenyl)-17β-hydroxy-2-(4-morpholinylmethylen)-17α-(prop-1-inyl)-östra-4,9-dien-3-on
und ihre Salze.

15. Produkt der allgemeinen Formel I gemäß einem der Ansprüche 1 bis 8 mit der folgenden Bezeichnung:
11β-(4-Dimethylaminophenyl)-17α-(prop-1-inyl)-isoxazolo [4.5-b] östra-4,9-dien-17-ol
und seine Salze.

16. Die Produkte der allgemeinen Formel I gemäß einem der Ansprüche 1 bis 3 mit den folgenden Bezeichnungen:
11β-[4-(2-Dimethylaminoethoxy)-phenyl]-östra-1,3,5(10)-trien-3,17β-diol,
11β-[4-(2-Dimethylaminoethoxy)-phenyl]-östra-1,3,5(10)-trien-17β-ol,
3-(2-Dimethylamino)-ethoxy-11β-phenyl-östra-1,3, 5(10)-trien-17β-ol
und ihre Salze.

17. Verfahren zur Herstellung der Produkte der allgemeinen Formel I gemäß Anspruch 1, dadurch gekennzeichnet, daß
(a) zur Herstellung der Produkte der Formel $I_A$

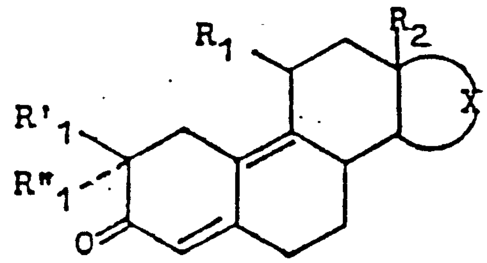

$(I_A)$

worin $R_1$, $R_2$ und X die in Anspruch 1 angegebene Bedeutung besitzen und $R'_1$ und $R''_1$ derart sind, daß entweder $R'_1$ und $R''_1$ jeweils einen Alkylrest bedeuten oder einer der Reste ein Wasserstoffatom und der andere einen Alkylrest bedeutet oder $R'_1$ und $R''_1$ jeweils einen Nitrilrest bedeuten oder einer der Reste einen Alkylrest und der andere einen Nitrilrest bedeutet, man ein Produkt der Formel II

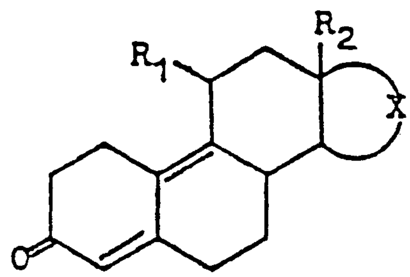

(II)

worin $R_1$, $R_2$ und X die in Anspruch 1 angegebene Bedeutung besitzen, zunächst gegebenenfalls der Einwirkung eines Mittels zum Schutz der funktionellen Gruppen und dann der Einwirkung einer starken Base und entweder derjenigen eines Alkylhalogenids oder von Tosylcyanid oder zunächst der Einwirkung eines Alkylhalogenids und dann derjenigen von Tosylcyanid unterzieht und dann erforderlichenfalls die Schutzgruppe entfernt;

(b)(1) zur Herstellung der Produkte der Formel $I_B$

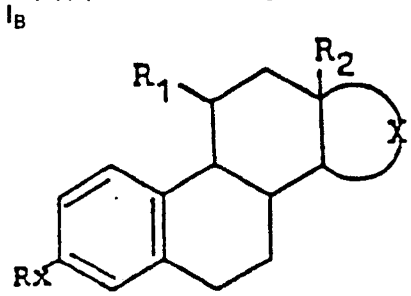

$(I_B)$

entsprechend der Formel $IB_1$

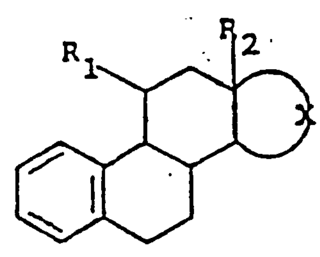

$(IB_1)$

worin $R_1$, $R_2$ und X die in Anspruch 1 angegebene Bedeutung besitzen, man ein Produkt der Formel II der Einwirkung eines

Reduktionsmittels unterzieht, um ein Produkt der Formel $II_1$

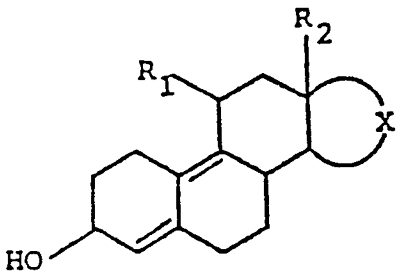

$(II_1)$

zu erhalten, worin $R_1$, $R_2$ und X die vorstehend angegebene Bedeutung besitzen, welches man der Einwirkung eines sauren Aromatisierungsmittels unterzieht;

(b)(2) zur Herstellung der Produkte der Formel $I_B$ entsprechend der Formel $IB_2$

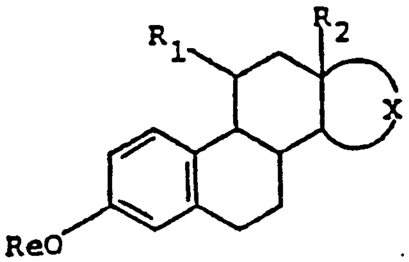

$(IB_2)$

worin $R_1$, $R_2$, Re und X die vorstehend angegebene Bedeutung besitzen, man ein Produkt der Formel II der Einwirkung eines Aromatisierungsmittels und danach derjenigen eines Verseifungsmittels und anschließend gegebenenfalls derjenigen eines Alkylierungs- oder Acylierungsmittels unterzieht;

(c) zur Herstellung der Produkte der Formel $I_C$

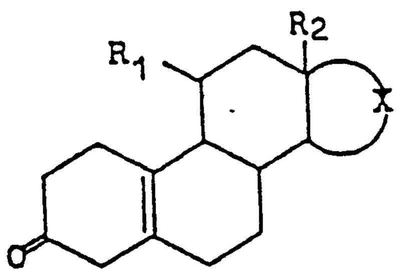

$(I_C)$

worin $R_1$, $R_2$ und X die in Anspruch 1 angegebene Bedeutung besitzen, man ein Produkt der Formel II der Einwirkung eines Reduktionsmittels unterzieht;

(d) zur Herstellung der Produkte der Formel $I_D$

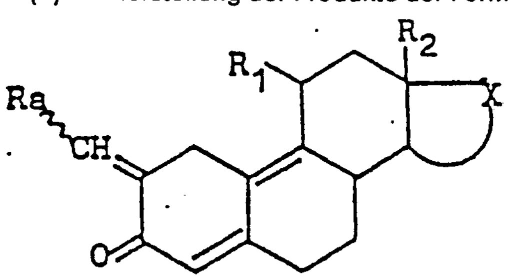

$(I_D)$

worin $R_1$, $R_2$, X und Ra die in Anspruch 1 angegebene Bedeutung besitzen, man ein Produkt der Formel II der Einwirkung eines Formylierungsmittels unterzieht, um ein Produkt der Formel III

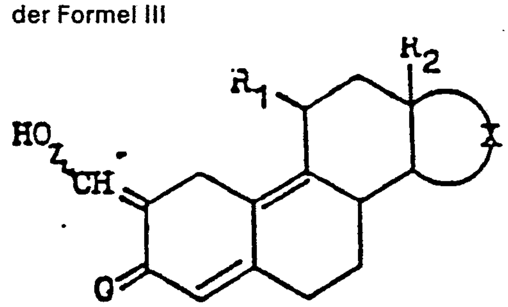

(III)

zu erhalten, welches Produkt der Formel III man entweder mit einem Acylierungsmittel umsetzt oder mit einem Amin der Formel

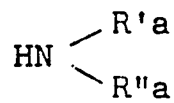

umsetzt, worin R'a und R''a die in Anspruch 1 angegebene Bedeutung besitzen;

(e) zur Herstellung der Produkte der Formel $I_E$

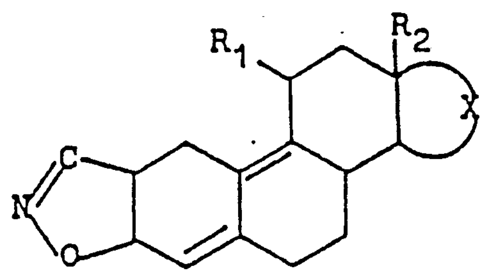

$(I_E)$

worin $R_1$, $R_2$ und X die in Anspruch 1 angegebene Bedeutung besitzen, man Hydroxylamin mit einem Produkt der Formel III umsetzt;

(f) zur Herstellung der Produkte der Formel $I'_A$

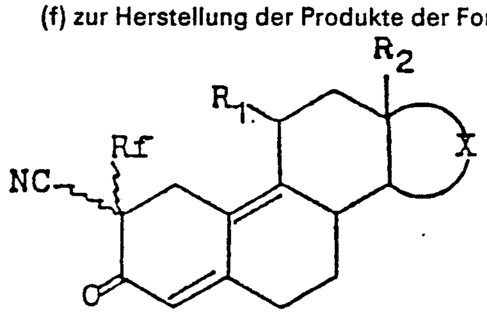

$(I'_A)$

worin Rf ein Wasserstoffatom oder einen Alkylrest bedeutet und $R_1$, $R_2$ und X die in Anspruch 1 angegebene Bedeutung besitzen, man eine Base mit den Produkten der Formel $I_E$ umsetzt, um die Produkte der Formel $I'_A$ zu erhalten, worin Rf ein Wasserstoffatom bedeutet, und gewünschtenfalls die so erhaltenen Produkte der Einwirkung einer starken Base und derjenigen eines Alkylhalogenids unterzieht, um die Produkte der Formel $I'_A$ zu erhalten, worin Rf einen Alkylrest bedeutet.

18. Verfahren gemäß Anspruch 17 zur Herstellung der Produkte der Formel I gemäß den Ansprüchen 4 und 6, dadurch gekennzeichnet, daß man das in Anspruch 17 beschriebene Verfahren, ausgehend von Produkten der Formel II, durchführt, worin der Substituent

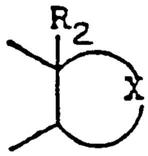

einen Rest

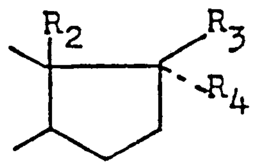

darstellt, worin $R_2$ einen Methylrest bedeutet, $R_3$ einen Rest -OH oder

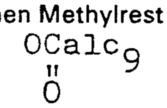

darstellt, worin $alc_9$ einen Alkylrest mit 1 bis 4 Kohlenstoffatomen bedeutet, $R_4$ einen Alkenyl- oder Alkinylrest mit 2 bis 4 Kohlenstoffatomen darstellt und $R_1$ einen Phenylrest darstellt, der durch einen Rest $-OAlc_{10}$, wobei $Alc_{10}$ einen Alkylrest mit 1 bis 4 Kohlenstoffatomen bedeutet, oder

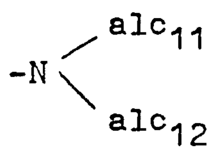

substituiert ist, worin $alc_{11}$ und $alc_{12}$ Alkylreste mit 1 bis 4 Kohlenstoffatomen darstellen.

19. Als Arzneimittel die pharmazeutisch verträglichen Produkte der Formel I gemäß einem der Ansprüche 1 oder 2 sowie deren Additionssalze mit pharmazeutisch verträglichen Säuren.

20. Als Arzneimittel die pharmazeutisch verträglichen Produkte der Formel I gemäß einem der Ansprüche 3 bis 15 sowie deren pharmazeutisch verträgliche Additionssalze.

21. Als Arzneimittel die pharmazeutisch verträglichen Produkte der Formel I gemäß Anspruch 16 sowie deren pharmazeutisch verträgliche Additionssalze mit Säuren.

22. Pharmazeutische Zusammensetzungen, enthaltend als Wirkstoff zumindest eines der Arzneimittel gemäß einem der Ansprüche 19 bis 21.

23. Als neue, industrielle Produkte die Produkte der allgemeinen Formel III

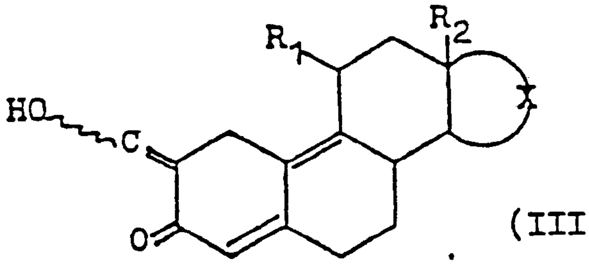

(III)

worin $R_1$, $R_2$ und X die in Anspruch 1 angegebene Bedeutung besitzen.

**Patentansprüche**

für den Vertragsstaat: AT

1. Verfahren zur Herstellung der Produkte der allgemeinen Formel (I):

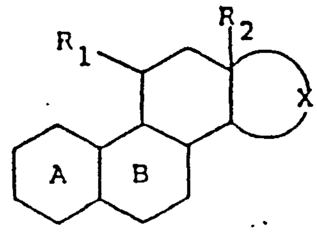

(I)

worin $R_1$ einen organischen Rest mit 1 bis 18 Kohlenstoffatomen und gegebenenfalls einem oder mehreren Heteroatomen bedeutet, wobei das dem Kohlenstoffatom in 11-Stellung unmittelbar benachbarte Atom ein Kohlenstoffatom ist, $R_2$ einen Kohlenwasserstoffrest mit 1 bis 8 Kohlenstoffatomen bedeutet, X den Rest eines pentagonalen oder hexagonalen Ringes, der gegebenenfalls substituiert ist und gegebenenfalls eine Unsättigung enthält, bedeutet, wobei die Ringe A und B eine der folgenden Strukturen besitzen:

(a) entweder bedeuten A und B die Gruppe

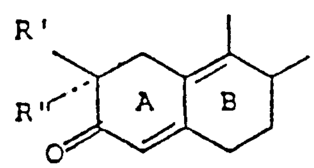

worin R' und R'', die gleich oder verschieden sind, ein Wasserstoffatom, einen Nitrilrest oder einen Alkylrest mit 1 bis 4 Kohlenstoffatomen bedeuten, wobei zumindest einer der Reste R' oder R'' kein Wasserstoffatom bedeutet;

(b) oder A und B bedeuten die Gruppe

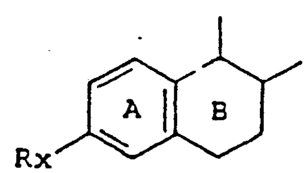

wobei Rx ein Wasserstoffatom oder eine Gruppe -ORe darstellt, worin Re ein Wasserstoffatom, einen Alkylrest mit 1 bis 6 Kohlenstoffatomen, der gegebenenfalls substituiert ist, oder einen Acylrest bedeutet;

(c) oder A und B stellen die Gruppe

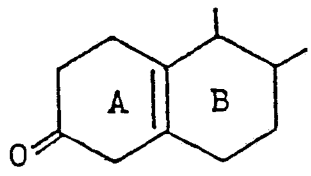

dar;

(d) oder A und B bedeuten die Gruppe

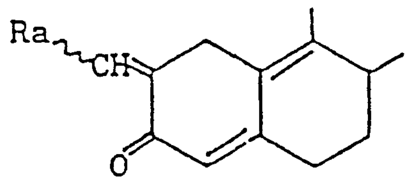

worin Ra einen Rest

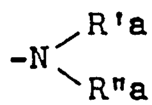

bedeutet, worin R'a und R"a entweder einen Alkylrest mit 1 bis 4 Kohlenstoffatomen bedeuten oder R'a und R"a mit dem Stickstoffatom, an das sie gebunden sind, einen Heterocyclus mit 5 oder 6 Kettengliedern bilden, der gegebenenfalls ein weiteres Heteroatom enthält, oder Ra einen Acyloxyrest bedeutet, wobei die gewellte Linie anzeigt, daß sich Ra in der Eoder Z-Position befinden kann;

(e) oder A und B bedeuten die Gruppe

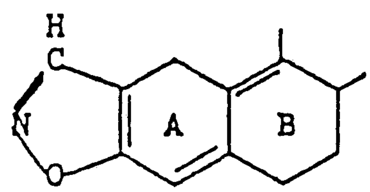

wobei, wenn A und B die Gruppe

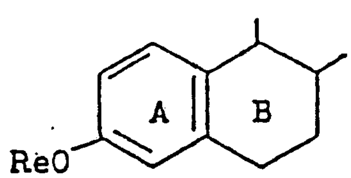

darstellen, der Rest $R_1$ zumindest ein Stickstoff-, Phosphor- oder Siliciumatom enthält, und wenn A und B die Gruppe

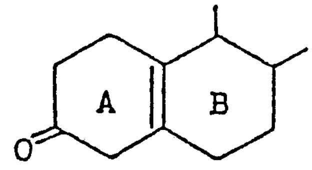

darstellen, der Rest $R_1$ keinen linearen, gesättigten Alkylrest bedeutet,

sowie von den Additionssalzen der Produkte der Formel I mit Säuren,

dadurch gekennzeichnet, daß

(a) man zur Herstellung der Produkte der Formel $I_A$

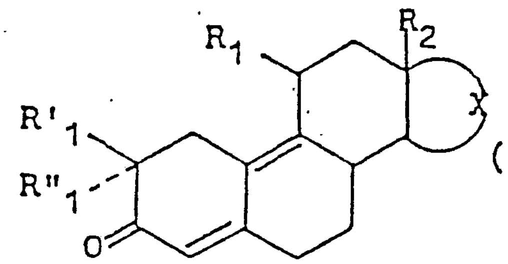

($I_A$)

worin $R_1$, $R_2$ und X die vorstehend angegebene Bedeutung besitzen und $R'_1$ und $R''_1$ derart sind, daß entweder $R'_1$ und $R''_1$ jeweils einen Alkylrest darstellen oder einer der Reste ein Wasserstoffatom bedeutet und der andere einen Alkylrest darstellt oder $R'_1$ und $R''_1$ jeweils einen Nitrilrest bedeuten oder einer der Reste einen Alkylrest und der andere einen Nitrilrest bedeutet, ein Produkt der Formel II

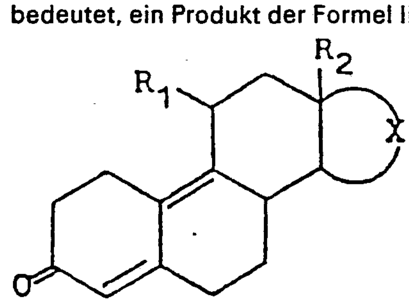

(II)

worin $R_1$, $R_2$ und X die vorstehend angegebene Bedeutung besitzen, zunächst gegebenenfalls der Einwirkung eines Reagens zum Schutz der funktionellen Gruppen, dann der Einwirkung einer starken Base und entweder eines Alkylhalogenids oder von Tosylcyanid oder zunächst der Einwirkung eines Alkylhalogenids, danach derjenigen von Tosylcyanid unterzieht und dann erforderlichenfalls die Schutzgruppe entfernt;

(b)(1) man zur Herstellung der Produkte der Formel $I_B$

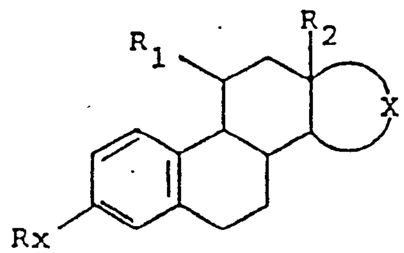

($I_B$)

entsprechend der Formel $IB_1$

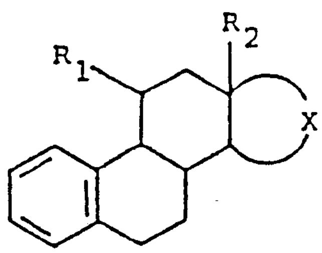

($IB_1$)

worin $R_1$, $R_2$ und X die vorstehend angegebene Bedeutung besitzen, ein Produkt der Formel II der Einwirkung eines Reduktionsmittels unterzieht, um ein Produkt der Formel $II_1$

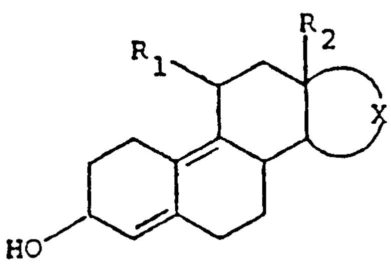

($II_1$)

zu erhalten, worin $R_1$, $R_2$ und X die vorstehend angegebene Bedeutung besitzen, welches man der Einwirkung eines sauren Aromatisierungsmittels unterzieht;

(b)(2) man zur Herstellung der Produkte der Formel $I_B$ entsprechend der Formel $IB_2$

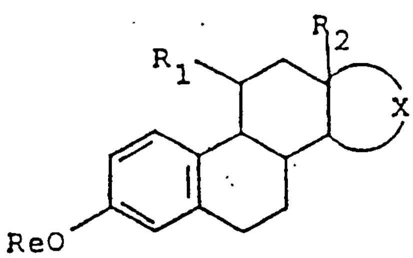

($IB_2$)

worin $R_1$, $R_2$, Re und X die vorstehend angegebene Bedeutung besitzen, ein Produkt der Formel II der Einwirkung eines Aromatisierungsmittels, danach eines Verseifungsmittels und anschließend gegebenenfalls derjenigen eines Alkylierungs- oder Acylierungsmittels unterzieht;

(c) man zur Herstellung der Produkte der Formel $I_C$

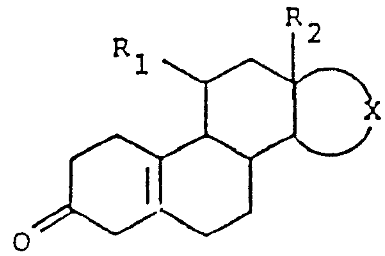

($I_C$)

worin $R_1$, $R_2$ und X die vorstehend angegebene Bedeutung besitzen, ein Produkt der Formel II der Einwirkung eines Reduktionsmittels unterzieht;

(d) man zur Herstellung der Produkte der Formel $I_D$

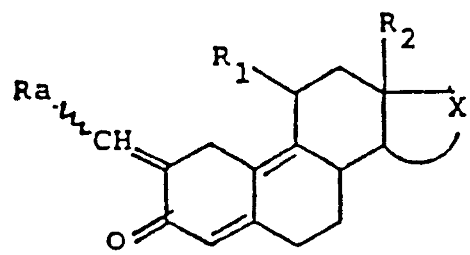

($I_D$)

worin $R_1$, $R_2$, X und Ra die vorstehend angegebene Bedeutung besitzen, ein Produkt der Formel II der Einwirkung eines Formylierungsreagens unterzieht, um ein Produkt der Formel III

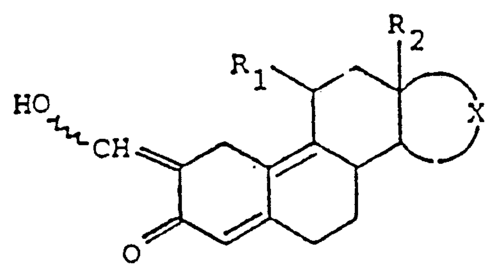

(III)

zu erhalten, das Produkt der Formel III entweder mit einem Acylierungsmittel reagieren läßt oder mit einem Amin der Formel

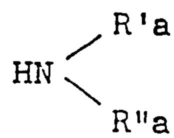

reagieren läßt, worin R'a und R"a die vorstehend angegebene Bedeutung besitzen; (e) man zur Herstellung der Produkte der Formel $I_E$

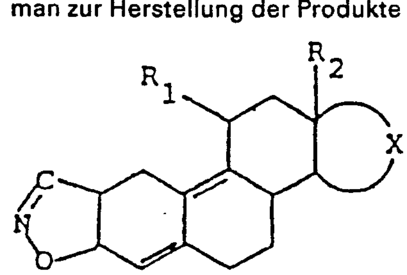

($I_E$)

worin $R_1$, $R_2$ und X die vorstehend angegebene Bedeutung besitzen, Hydroxylamin mit einem Produkt der Formel III umsetzt;

(f) man zur Herstellung der Produkte der Formel $I'_A$

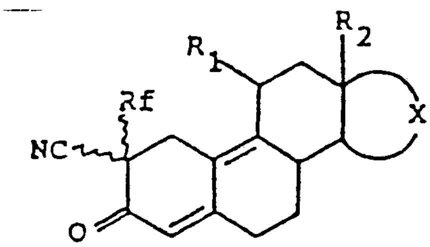

$(I'_A)$

worin Rf ein Wasserstoffatom oder einen Alkylrest bedeutet und $R_1$, $R_2$ und X die vorstehend angegebene Bedeutung besitzen, eine Base mit den Produkten der Formel $I_E$ umsetzt, um die Produkte der Formel $I'_A$ zu erhalten, worin Rf ein Wasserstoffatom bedeutet, und gewünschtenfalls die so erhaltenen Produkte der Einwirkung einer starken Base und eines Alkylhalogenids unterzieht, um die Produkte der Formel $I'_A$, worin Rf einen Alkylrest bedeutet, zu erhalten.

2. Verfahren gemäß Anspruch 1, dadurch gekennzeichnet, daß man das Verfahren, ausgehend von den Produkten der Formel (II), durchführt, worin X den Rest eines Rings der Formel

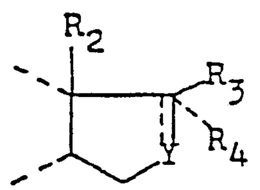

darstellt, worin $R_2$ die in Anspruch 1 angegebene Bedeutung besitzt, die gestrichelte Linie in 16-17-Stellung die etwaige Anwesenheit einer Doppelbindung anzeigt, Y einen Rest

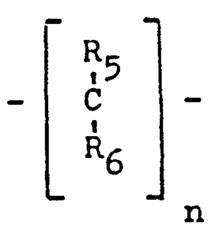

bedeutet, worin n die Zahl 1 oder 2 darstellt, $R_5$ ein Wasserstoffatom, einen Alkylrest mit 1 bis 8 Kohlenstoffatomen, einen Alkenylrest oder Alkinylrest mit 2 bis 8 Kohlenstoffatomen, einen Arylrest mit 6 bis 14 Kohlenstoffatomen oder einen Aralkylrest mit 7 bis 15 Kohlenstoffatomen darstellt, $R_6$, das mit $R_5$ identisch oder von diesem verschieden ist, eine der für $R_5$ angegebenen Bedeutungen besitzen kann und auch einen Hydroxylrest darstellen kann, $R_3$ und $R_4$, die gleich oder verschieden sind,

entweder ein Wasserstoffatom

oder einen Rest, ausgewählt unter den Resten OH, $Oalc_4$, $O-CO-alc_5$, worin $alc_4$ und $alc_5$ einen Alkylrest mit 1 bis 8 Kohlenstoffatomen oder einen Aralkylrest mit 7 bis 15 Kohlenstoffatomen darstellen,

oder einen Alkenyl- oder Alkinylrest mit 2 bis 8 Kohlenstoffatomen

oder einen Rest

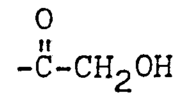

oder einen Rest $-COCH_2OCOalc_6$, worin $alc_6$ einen Alkylrest mit 1 bis 8 Kohlenstoffatomen, der gegebenenfalls substituiert ist, oder einen Aralkylrest mit 7 bis 15 Kohlenstoffatomen bedeutet,

oder einen Rest $CO-CO_2H$

oder einen Rest $CO-CO_2-alc_7$, worin $alc_7$ einen Alkylrest mit 1 bis 8 Kohlenstoffatomen bedeutet,

oder einen Rest 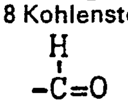

oder einen Rest 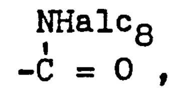

worin $alc_8$ einen Alkylrest mit 1 bis 8 Kohlenstoffatomen oder einen Aralkylrest mit 7 bis 15 Kohlenstoffatomen bedeutet,

oder einen Rest $-C\equiv N$ darstellen

oder $R_3$ und $R_4$ gemeinsam einen Rest

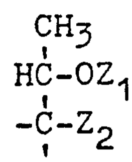

bilden, worin $Z_1$ ein Wasserstoffatom, einen Alkylrest oder einen Acylrest mit 1 bis 8 Kohlenstoffatomen darstellt und $Z_2$ einen Alkylrest mit 1 bis 8 Kohlenstoffatomen bedeutet,

oder $R_3$ und $R_4$ gemeinsam einen Rest

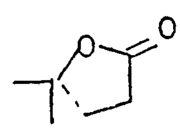

bilden.

3. Verfahren gemäß Anspruch 2, dadurch gekennzeichnet, daß man das Verfahren, ausgehend von den Produkten der Formel II, durchführt, worin der Ring D keine Unsättigung enthält, $R_5$ und $R_6$ ein Wasserstoffatom bedeuten und n für 1 steht.

4. Verfahren gemäß Anspruch 2, dadurch gekennzeichnet, daß man das Verfahren, ausgehend von Produkten der Formel II, durchführt, worin $R_3$ einen Rest OH oder $OCOalc_5$ bedeutet und $R_4$ einen Alkenyl- oder Alkinylrest mit höchstens 4 Kohlenstoffatomen darstellt, wobei $alc_5$ die in Anspruch 2 angegebene Bedeutung besitzt.

5. Verfahren gemäß einem der Ansprüche 1 bis 4, dadurch gekennzeichnet, daß man das Verfahren, ausgehend von Produkten der Formel

(II), durchführt, worin $R_1$ einen Aryl- oder Aralkylrest bedeutet, der eine Aminfunktion

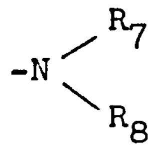

aufweist, worin $R_7$ und $R_8$ einen Alkylrest mit 1 bis 8 Kohlenstoffatomen oder einen primären, sekundären oder tertiären Alkylrest bedeuten, der 1 bis 8 Kohlenstoffatome enthält und ein oder mehrere Heteroatome, ausgewählt unter Sauerstoff, Stickstoff und Schwefel, aufweist, von denen zumindest eines ein Stickstoffatom ist, oder der durch einen Heterocyclus mit zumindest einem Stickstoffatom substituiert ist.

6. Verfahren gemäß einem der Ansprüche 1 bis 4, dadurch gekennzeichnet, daß man das Verfahren, ausgehend von Produkten der Formel (II), durchführt, worin $R_1$ einen 2-, 3- oder 4-Pyridylrest,

einen Rest $-(CH_2)_n$

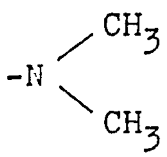

$(n \geqslant 3)$

einen Rest

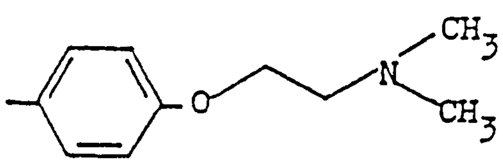

einen Rest

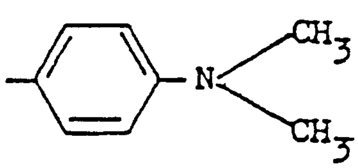

einen Rest

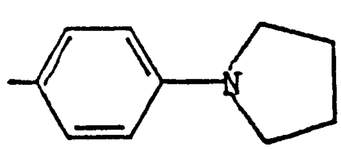

oder einen Rest

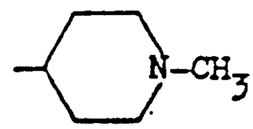

bedeutet.

7. Verfahren gemäß einem der Ansprüche 1 bis 4, dadurch gekennzeichnet, daß man das Verfahren, ausgehend von Produkten der Formel (II), durchführt, worin $R_1$ einen Thienyl-, Furyl-, Cycloalkyl- mit 3 bis 6 Kohlenstoffatomen oder Phenylrest bedeutet, der gegebenenfalls durch einen oder mehrere Reste substituiert ist, ausgewählt unter den Hydroxy-, Halogen-, Trifluormethyl-, Alkyl-, Alkoxy-, gegebenenfalls in Form des Sulfoxids oder Sulfons oxidierten Alkylthioresten, wobei die Ringe A und B nicht die Gruppe

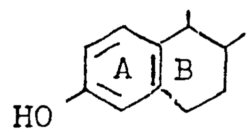

bedeuten.

8. Verfahren gemäß einem der Ansprüche 1 bis 4, dadurch gekennzeichnet, daß man das Verfahren, ausgehend von Produkten der Formel (II), durchführt, worin $R_1$ einen Phenylrest bedeutet, der durch einen Rest substituiert ist, ausgewählt unter den Chlor-, Fluor-, Methylthio-, Methylsulfonyl-, Methoxy-, Hydroxy- und Allyloxyresten.

9. Verfahren gemäß einem der Ansprüche 1 bis 8, dadurch gekennzeichnet, daß man das Verfahren, ausgehend von Produkten der Formel (II), durchführt, worin die Ringe A und B die Gruppe

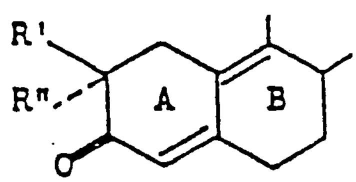

darstellen, worin R' und R'' derart sind, daß entweder R' und R'' gleich sind und jeweils einen Methyl- oder Nitrilrest bedeuten

oder einer der Reste ein Wasserstoffatom und der andere einen Methyl- oder Nitrilrest bedeutet.

10. Verfahren gemäß einem der Ansprüche 1 bis 8, dadurch gekennzeichnet, daß man das Verfahren, ausgehend von Produkten der Formel (II), durchführt, worin die Ringe A und B die Gruppe

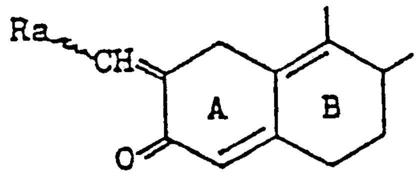

darstellen, worin Ra einen Morpholinorest oder einen Acetyloxyrest bedeutet.

11. Verfahren gemäß Anspruch 1, dadurch gekennzeichnet, daß man das Verfahren, ausgehend von Produkten der Formel (II), durchführt, worin der Substituent

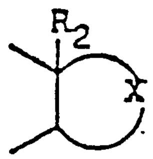

einen Rest

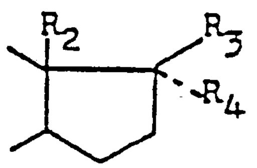

darstellt worin $R_2$ einen Methylrest bedeutet, $R_3$ einen Rest -OH oder $-O\underset{\underset{O}{\|}}{C}alc_9$

darstellt, wobei $alc_9$ einen Alkylrest mit 1 bis 4 Kohlenstoffatomen bedeutet, $R_4$ einen Alkenyl- oder Alkinylrest mit 2 bis 4 Kohlenstoffatomen bedeutet und $R_1$ einen Phenylrest darstellt, der durch einen Rest $-OAlc_{10}$, worin $Alc_{10}$ einen Alkylrest mit 1 bis 4 Kohlenstoffatomen bedeutet, oder

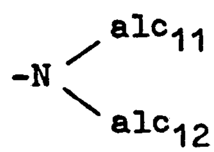

substituiert ist, worin $alc_{11}$ und $alc_{12}$ Alkylreste mit 1 bis 4 Kohlenstoffatomen bedeuten.

12. Verfahren gemäß einem der Ansprüche 1 bis 9, dadurch gekennzeichnet, daß man eines der folgenden Produkte mit den folgenden Bezeichnungen herstellt:

2,2-Dimethyl-17β-hydroxy-11β-(4-dimethylaminophe-nyl)-17α-(prop-1-inyl)-östra-4,9-dien-3-on,

2,2-Dicyano-11β-(4-dimethylaminophenyl)-17β-hydro-xy-17α-(prop-1-inyl)-östra-4,9-dien-3-on,

2α-Methyl-11β-(4-dimethylaminophenyl)-17β-hydroxy-17α-(prop-1-inyl)-östra-4,9-dien-3-on,

2β-Methyl-118-(4-dimethylaminophenyl)-17β-hydroxy-17α-(prop-1-inyl)-östra-4,9-dien-3-on,

2-Cyano-11β-(4-dimethylaminophenyl)-17β-hydroxy-17α-(prop-1-inyl)-östra-4,9-dien-3-on

sowie ihre Salze.

13. Verfahren gemäß einem der Ansprüche 1 bis 8, dadurch gekennzeichnet, daß man eines der Produkte mit den folgenden Bezeichnungen herstellt:

11β-(4-Dimethylaminophenyl)-17α-(prop-1-inyl)-östra-1,3,5(10)-trien-3,17-diol,

17β-Acetoxy-11β-(4-dimethylaminophenyl)-17α-(prop-1-inyl)-östra-1,3,5(10)-trien-3-ol,

11β-(4-Dimethylaminophenyl)-3-methoxy-17α-(prop-1-inyl)-östra-1,3,5(10)-trien-17β-ol,

17β-Acetoxy-11β-(4-dimethylaminophenyl)-3-meth-oxy-17α-(prop-1-inyl)-östra-1,3,5(10)-trien

sowie ihre Salze.

14. Verfahren gemäß einem der Ansprüche 1 bis 8, dadurch gekennzeichnet, daß man eines der Produkte mit den folgenden Bezeichnungen herstellt:

17β-Hydroxy-11β-(3-methoxyphenyl)-17α-(prop-1-inyl)-östra-5(10)-en-3-on,

E-17β-Hydroxy-11β-(3-methoxyphenyl)-17α-(prop-1-enyl)-östra-5(10)-en-3-on,

17β-Hydroxy-11β-(4-dimethylaminophenyl)-17α-(prop-1-inyl)-östr-5(10)-en-3-on

sowie ihre Salze.

15. Verfahren gemäß einem der Ansprüche 1 bis 8 und 10, dadurch gekennzeichnet, daß man eines der Produkte mit den folgenden Bezeichnungen herstellt:

2-[(Acetyloxy)-methylen]-11β-(4-dimethylaminophe-nyl)-17β-hydroxy-17α-(prop-1-inyl)-östra-4,9-dien-3-on,

11β-(4-Dimethylaminophenyl)-17β-hydroxy-2-(4-mor-pholinylmethylen)-17α-(prop-1-inyl)-östra-4,9-dien-3-on

sowie ihre Salze.

16. Verfahren gemäß einem der Ansprüche 1 bis 8, dadurch gekennzeichnet, daß man das 11β-(4-Dimethylaminophenyl)-17α-(prop-1-inyl)-isoxazolo [4.5-b] östra-4,9-dien-17-ol und seine Salze herstellt.

17. Verfahren gemäß einem der Ansprüche 1 bis 3, dadurch gekennzeichnet, daß man eines der Produkte mit den folgenden Bezeichnungen herstellt:

11β-[4-(2-Dimethylaminoethoxy)-phenyl]-östra-1,3, 5(10)-trien-3,17β-diol,

11β-[4-(2-Dimethylaminoethoxy)-phenyl]-östra-1,3, 5(10)-trien-17β-ol,

3-(2-Dimethylamino)-ethoxy-11β-phenyl-östra-1,3, 5(10)-trien-17β-ol

sowie ihre Salze.

## Claims

for the contracting states BE CH DE FR GB IT LI LU NL SE

1) The products with the general formula I:

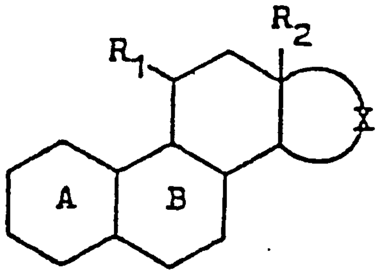

(I)

in which $R_1$ represents an organic radical containing from 1 to 18 carbon atoms and possibly one or more heteroatoms, the atom immediately adjacent to the carbon at position 11 being a carbon atom, $R_2$ represents a hydrocarbon radical containing from 1 to 8 carbon atoms, X represents the residue of a pentagonal or hexagonal ring possibly substituted and possibly carrying an unsaturation, the rings A and B having one of the following structures:

a) - Either A and B represent the group:

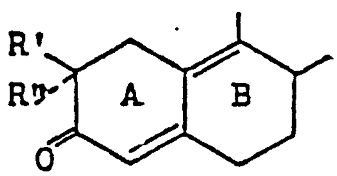

in which R' and R'' being identical or different represent a hydrogen atom, a nitrile radical or an alkyl radical having from 1 to 4 carbon atoms it being understood that one at least of the radicals R' or R'' does not represent a hydrogen atom;

b) - Or A and B represent the group:

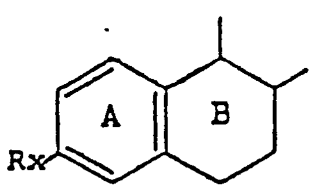

Rx representing a hydrogen atom or a group -ORe, in which Re represents a hydrogen atom, a possibly substituted alkyl radical having from 1 to 6 carbon atoms or an acyl radical;

c) - Or A and B represent the group:

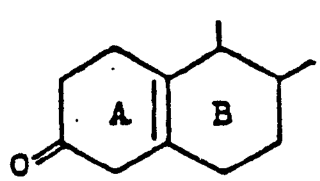

d) - Or A and B represent the group

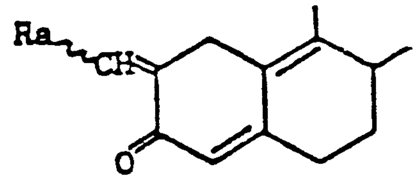

in which Ra represents a radical

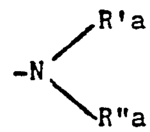

in which R'a and R''a represent either an alkyl radical having from 1 to 4 carbon atoms, or R'a and R''a represent with the nitrogen atom to which they are linked, a heterocycle with 5 or 6 links possibly including another hetero atom, or Ra represents an acyloxy radical, the wavy line signifying that Ra can be found in position E or Z,

e) - Or A and B represent the group

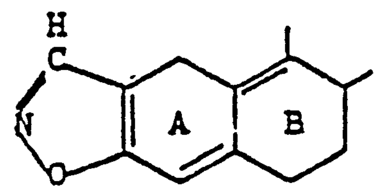

it being understood that when A and B represent the group

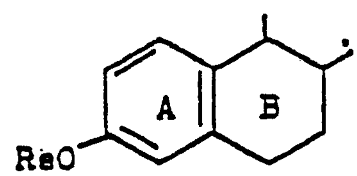

the radical $R_1$ contains at least one nitrogen, phosphorous or silicon atom, and when A and B represent the group

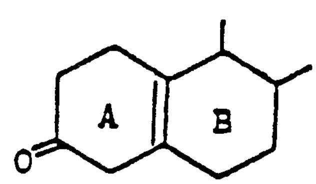

the radical $R_1$ does not represent a saturated linear alkyl radical, as well as the salts of addition of products with the formula I with acids.

2). Products with the formula I as defined in claim 1 in which X represents the residue of a ring with the formula

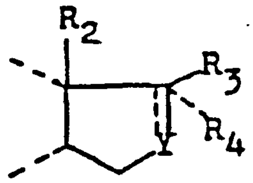

in which $R_2$ retains the same significance as in claim 1, the dotted line at 16-17 symbolizes the possible presence of a double bond, Y represents a radical

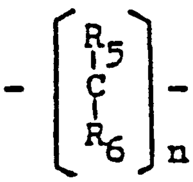

in which n represents the number 1 or 2, $R_5$ represents a hydrogen atom, an alkyl radical containing from 1 to 8 carbon atoms, an alkenyl or alkynyl radical containing from 2 to 8 carbon atoms, an aryl radical containing from 6 to 14 carbon atoms, or an aralkyl radical containing from 7 to 15 carbon atoms, $R_6$, identical to or different from $R_5$, can take one of the values indicated for $R_5$ and can also represent a hydroxyl radical, $R_3$ and $R_4$, identical or different, represent

either a hydrogen atom,

or a radical chosen from the group formed by the radicals OH, $Oalk_4$, $O\text{-}CO\text{-}alk_5$ in which $alk_4$ and $alk_5$ represent an alkyl radical containing from 1 to 8 carbon atoms or an aralkyl radical containing from 7 to 15 carbon atoms,

or an alkenyl or alkynyl radical containing from 2 to 8 carbon atoms,

or a radical 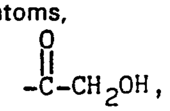,

or a radical $-COCH_2OCOalk_6$, in which $alk_6$ represents an alkyl radical containing from 1 to 8 carbon atoms possibly substituted or an aralkyl radical containing from 7 to 15 carbon atoms,

or a radical $CO\text{-}CO_2H$,

or a radical $CO\text{-}CO_2\text{-}alk_7$ in which $alk_7$ represents an alkyl radical containing from 1 to 8 carbon atoms,

or a radical 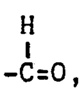,

or a radical 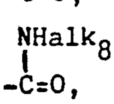,

in which $alk_8$ represents an alkyl radical containing from 1 to 8 carbon atoms or an aralkyl radical containing from 7 to 15 carbon atoms,

or a radical $-C\equiv N$,

or $R_3$ and $R_4$ together form a radical

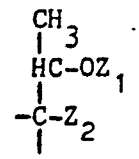

in which $Z_1$ represents a hydrogen atom, an alkyl radical or an acyl radical containing from 1 to 8 carbon atoms and $Z_2$ an alkyl radical containing from 1 to 8 carbon atoms,

or $R_3$ and $R_4$ together form a radical

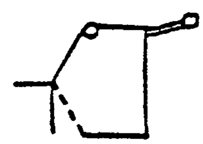

3) Products with the formula I as defined in claim 2 in which the ring D does not carry an unsaturation, $R_5$ and $R_6$ represent a hydrogen atom and n is equal to 1.

4) Products with the formula I as defined in any one of the claims 1 or 2 in which $R_3$ represents a radical OH or $OCOalk_5$ and $R_4$ represents an alkenyl or alkynyl radical having at the most 4 carbon atoms, $alk_5$ keeping the same significance as in claim 2.

5) Products with the formula I as defined in any one of the claims 1 to 4 in which $R_1$ represents an aryl or aralkyl radical carrying an amine function

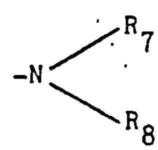

in which $R_7$ and $R_8$ represent an alkyl radical containing from 1 to 8 carbon atoms or a primary, secondary or tertiary alkyl radical containing from 1 to 8 carbon atoms, including one or more heteroatoms chosen from the group composed of oxygen, nitrogen, sulphur, and silicon of which at least one is a nitrogen atom, or substituted by a heterocycle including at least one nitrogen atom.

6) Products with the formula I as defined in any one of the claims 1 to 4 in which $R_1$ represents a 2, 3 or 4-pyridyl radical,

a radical

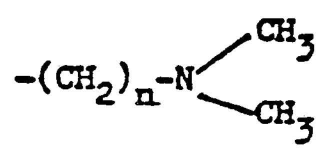 $(n \geq 3)$,

a radical:

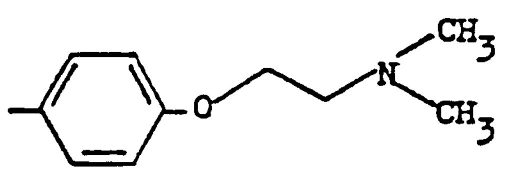

,

a radical

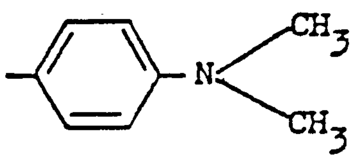

a radical

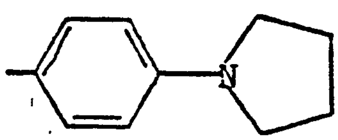

or a radical

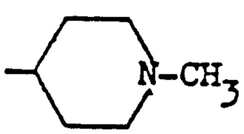

7) Products with the formula I as defined in any one of the claims 1 to 4 in which $R_1$ represents a thienyl or a furyl radical, or a cycloalkyl radical having from 3 to 6 carbon atoms or a phenyl radical possibly substituted by one or more radicals chosen from among the hydroxy, halogen, trifluoromethyl, alkyl, alkoxy or alkylthio radicals possibly oxidized in the form of sulphoxide or sulphone, it being understood that the rings A and B do not represent the group

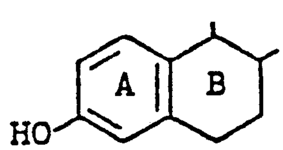

8) Products with the formula I as defined in any one of the claims 1 to 4 and 7 in which $R_1$ represents a phenyl radical substituted by a radical chosen from the group formed by the chloro, fluoro, methylthio, methylsulphonyl, methoxy, hydroxy and allyloxy radicals.

9) Products with the general formula I as defined in any one of the claims 1 to 8 in which the rings A and B represent the group

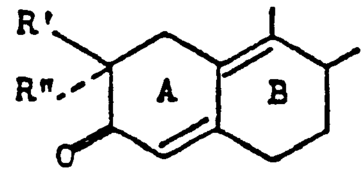

in which R' and R'' are such that:
- either R' and R'' being identical each represent a methyl radical or a nitrile radical
- or one represents a hydrogen atom and the other represents a methyl or nitrile radical.

10) The products with the general formula I as defined in any one of the claims 1 to 8 in which the rings A and B represent the group

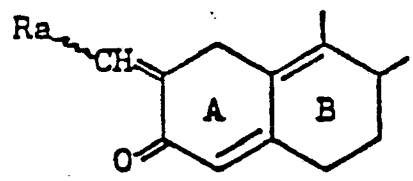

in which Ra represents a morpholine radical or an acetyloxy radical.

11) The products with the general formula I as defined in any one of the claims 1 to 9 answering to the following formulae:
- 2,2-dimethyl-17β-hydroxy-11β-(4-dimethylaminophenyl)-17α-(prop-1-ynyl)estra-4,9-dien-3-one,
- 2,2-dicyano-11β-(4-dimethylaminophenyl)-17β-hydroxy-17α-(prop-1-ynyl)estra-4,9-dien-3-one,
- 2α-methyl-11β-(4-dimethylaminophenyl)-17β-hydroxy-17α-(prop-1-ynyl)estra-4,9-dien-3-one,
-2β-methyl-11β-(4-dimethylaminophenyl)-17β-hydroxy-17α-(prop-1-ynyl)estra-4,9-dien-3-one.
- 2-cyano-11 β-(4-dimethylaminophenyl)-17β-hydroxy-17α-(prop-1-ynyl)estra-4,9-dien-3-one,
and their salts.

12) Products with the general formula I as defined in any one of the claims 1 to 8 answering to the following formulae:
- 11 β -(4-dimethylaminophenyl)-17 α -(prop-1-ynyl)estra-1,3,5(10)-triene-3,17-diol,
- 17β-acetoxy-11β-(4-dimethylaminophenyl)-17α-(prop-1-ynyl)estra-1,3,5(10)-trien-3-ol,
- 11β-(4-dimethylaminophenyl)-3-methoxy-17α-(prop-1-ynyl)estra-1,3,5(10)-trien-17β-ol,
- 17β -acetoxy-11β-(4-dimethylaminophenyl)-3-methoxy-17α -(prop-1-ynyl)estra-1,3,5(10)-triene and their salts.

13) Products with the general formula I as defined in any one of the claims 1 to 8 answering to the following formulae:
- 17β-hydroxy-11β-(3-methoxyphenyl)-17α-(prop-1-ynyl)estra-5(10)-en-3-one,
- E-17β-hydroxy-11 β-(3-methoxyphenyl)-17α-(prop-1-enyl)estra-5(10)-en-one,
- 17β -hydroxy-11β-(4-dimethylaminophenyl)-

17α-(prop-1-ynyl)estr-5(10)-en-3-one, and their salts.

14) Products with the general formula I as defined in any one of the claims 1 to 8 and 10, answering to the following formulae: -
2[(acetyloxy)methylene]-11β-(4-dimethylaminophenyl)-17β-hydroxy-17α-(prop-1-ynyl)estra-4,9-dien-3-one,
- 11β-(4-dimethylaminophenyl)-17β -hydroxy-2-(4-morpholinyl-methylene)-17α-(prop-1-ynyl)estra-4,9-dien-3-one
and their salts.

15) The product with the general formula I as defined in any one of the claims 1 to 8 answering to the formula:
- 11β-(4-dimethylaminophenyl)-17α -(prop-1-ynyl)isoxazolo-[4,5-b]-estra-4,9-dien-17-ol,
and its salts.

16) Products with the general formula I as defined in any one of the claims 1 to 3 answering to the following formulae:
- 11β-[4-(2-dimethylaminoethoxy)phenyl]-estra-1,3,5(10)-triene-3,17β-diol,
- 11β-[4-(2-dimethylaminoethoxy)phenyl]-estra-1,3,5(10)-trien-17β-ol,
- 3-(2-dimethylamino)ethoxy-11 β-phenyl-estra-1,3,5(10)-trien-17β-ol,
and their salts.

17) Preparation process for products with the general formula I as defined in claim 1 characterized in that:

a) to prepare the products with the formula $I_A$:

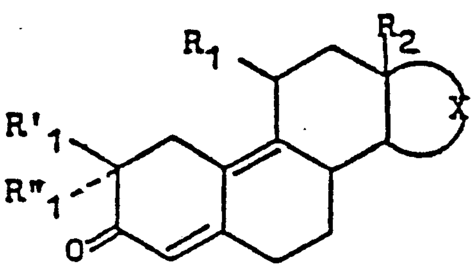

$(I_A)$

in which $R_1$, $R_2$ and X retain the same significance as in claim 1 and $R'_1$ and $R''_1$ are such that, either $R'_1$ and $R''_1$ each represent an alkyl radical or one represents a hydrogen atom and the other represents an alkyl radical or $R'_1$ and $R''_1$ each represent a nitrile radical or one represents an alkyl radical and the other a nitrile radical, a product with the formula II:

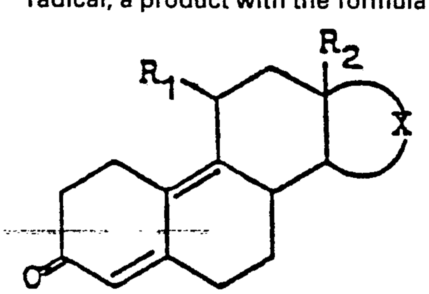

(II)

in which $R_1$, $R_2$ and X retain the same significance as in claim 1, is submitted first, possibly to the action of a protector reagent of the functional groups then to the action of a strong base and either to the action of an alkyl halogenide or of tosyl cyanide, or first to the action of an alkyl halogenide then of tosyl cyanide, then if necessary the protector group is eliminated;

b) 1) to prepare the products with the formula $I_B$:

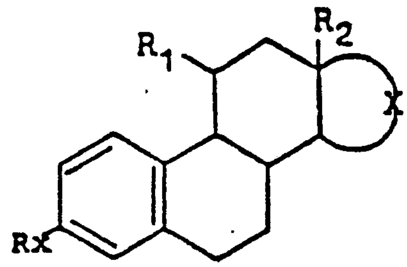

$(I_B)$

answering to the formula $IB_1$:

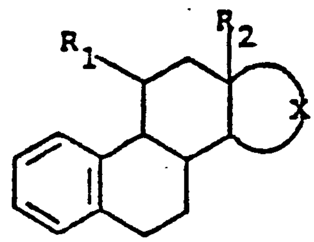

$(IB_1)$

in which $R_1$, $R_2$ and X retain the same significance as in claim 1, a product with the formula II is submitted to the action of a reducing agent, so as to obtain a product with the formula $II_1$:

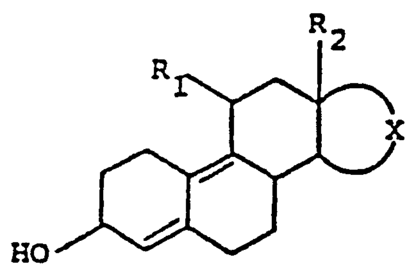

$(II_1)$

in which $R_1$, $R_2$ and X retain the same significance as above, which is submitted to the action of an acid aromatisation agent;

b) 2) to prepare the products with the formula $I_B$ answering to the formula $IB_2$:

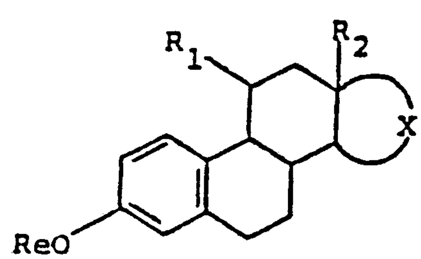

$(IB_2)$

in which $R_1$, $R_2$, Re and X retain the same significance as above, a product with the formula II is submitted to the action of an aromatisation agent then to saponification, then poßsibly to an alkylation or acylation reagent; c) to prepare the products with the formula $I_C$:

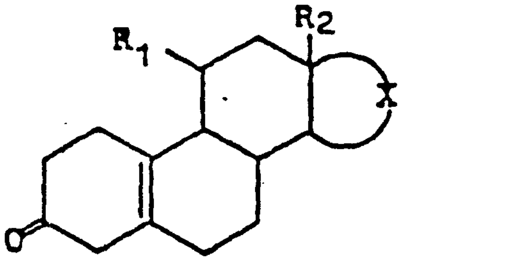

($I_C$)

in which $R_1$, $R_2$ and X retain the same significance as in claim 1, a product with the formula II is submitted to the action of a reducing agent;

d) to prepare the products with the formula $I_D$:

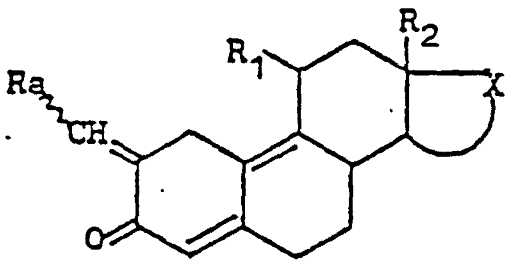

($I_D$)

in which $R_1$, $R_2$, X and Ra retain the same significance as in claim 1, a product with the formula II is submitted to the action of a formylation reagent so as to obtain a product with the formula III

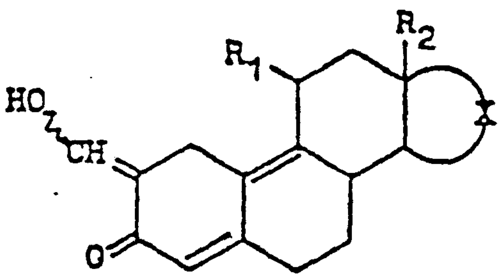

(III)

which product with the formula III, is either made to react with an acylation reagent, or is made to react with an amine with the formula

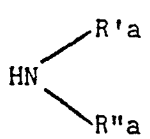

in which R'a and R"a retain the same significance as in claim 1;

e) to prepare the products with the formula $I_E$:

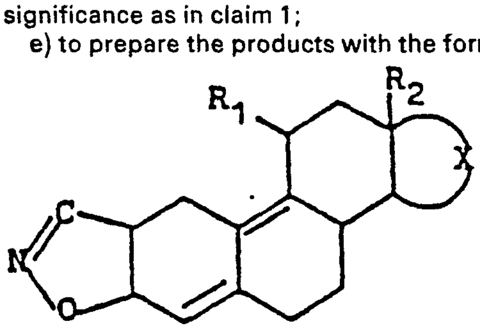

($I_E$)

in which $R_1$, $R_2$ and X retain the same significance as in claim 1, hydroxylamine is made to react on a product with the formula III;

f) to prepare the products with the formula $I'_A$

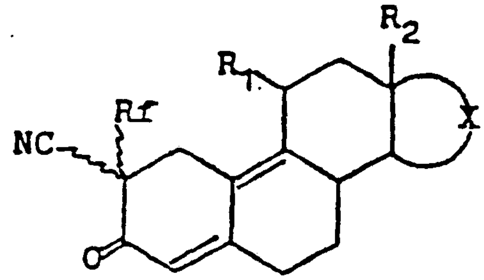

($I'_A$)

in which Rf represents a hydrogen atom or an alkyl radical and $R_1$, $R_2$ and X retain the same significance as in claim 1, a base is made to react on the products with the formula $I_E$ so as to obtain the products with the formula $I'_A$ in which Rf represents a hydrogen atom and if desired the products thus obtained are submitted to the action of a strong base and an alkyl halogenide so as to obtain the products with the formula $I'_A$ in which Rf represents an alkyl radical.

18) Process according to claim 17 for the preparation of products with the formula I as defined in claims 4 and 6, characterized in that the process described in claim 17 is carried out starting with products with the formula II in which the substituent

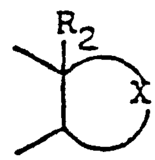

represents a radical

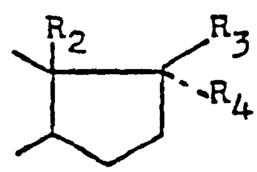

in which $R_2$ represents a methyl radical, $R_3$ represents a radical -OH or $-O\underset{\underset{O}{\|}}{C}alk_9$,

$alk_9$ being an alkyl radical having from 1 to 4 carbon atoms, $R_4$ represents an alkenyl or alkynyl radical having from 2 to 4 carbon atoms and $R_1$ represents a phenyl radical substituted by a radical $-Oalk_{10}$, $alk_{10}$ being an alkyl radical having from 1 to 4 carbon atoms, or

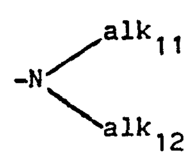

,

$alk_{11}$ and $alk_{12}$ being alkyl radicals having from 1 to 4 carbon atoms.

19) As medicaments, pharmaceutically acceptable products with the formula I as defined in either of the claims 1 or 2, as well as

their salts of addition with pharmaceutically acceptable acids.

20) As medicaments, pharmaceutically acceptable products with the formula I as defined in any one of the claims 3 to 15, as well as their pharmaceutically acceptable salts of addition.

21) As medicaments, pharmaceutically acceptable products with the formula I as defined in claim 16, as well as their salts of addition with pharmaceutically acceptable acids.

22) Pharmaceutical compositions containing as active principle, at least one of the medicaments according to any one of the claims 19 to 21.

23) As new industrial products, the products with the general formula III:

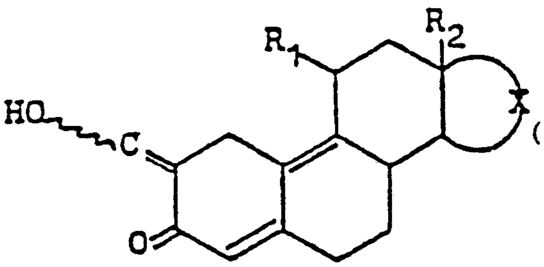

(III)

in which $R_1$, $R_2$ and X retain the same significance as in claim 1.

## Claims

for the contracting state AT

1) Preparation process for products with the general formula (I):

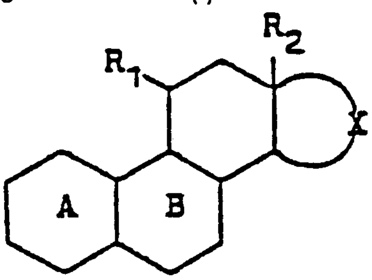

(I)

in which $R_1$ represents an organic radical containing from 1 to 18 carbon atoms and possibly one or more heteroatoms, the atom immediately adjacent to the carbon at position 11 being a carbon atom, $R_2$ represents a hydrocarbon radical containing from 1 to 8 carbon atoms, X represents the residue of a pentagonal or hexagonal ring possibly substituted and possibly carrying an unsaturation, the rings A and B having one of the following structures:

a) - Either A and B represent the group:

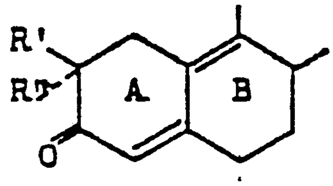

in which R' and R'' being identical or different represent a hydrogen atom, a nitrile radical or an alkyl radical having from 1 to 4 carbon atoms it being understood that one at least of the radicals R' or R'' does not represent a hydrogen atom;

b) - Or A and B represent the group:

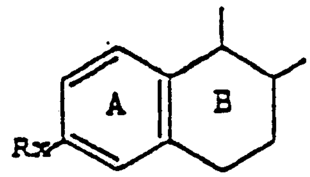

Rx representing a hydrogen atom or a group -ORe, in which Re represents a hydrogen atom, a possibly substituted alkyl radical having from 1 to 6 carbon atoms or an acyl radical;

c) - Or A and B represent the group:

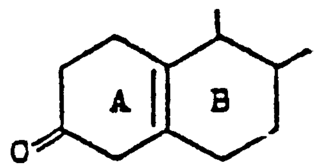

d) - Or A and B represent the group

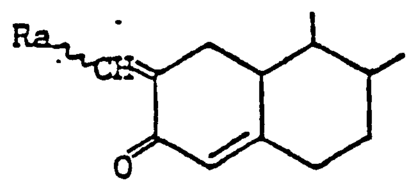

in which Ra represents a radical

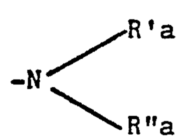

in which R'a and R''a represent either an alkyl radical having from 1 to 4 carbon atoms, or R'a and R''a represent with the nitrogen atom to which they are linked, a heterocycle with 5 or 6 links possibly including another hetero atom, or Ra represents an acyloxy radical, the wavy line signifying that Ra can be found in position E or Z,

e) - Or A and B represent the group

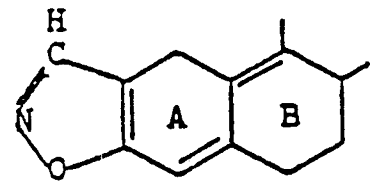

it being understood that when A and B represent the group

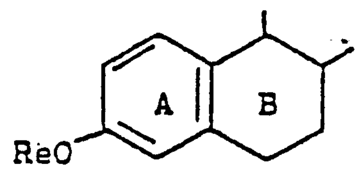

the radical $R_1$ contains at least one nitrogen, phosphorous or silicon atom, and when A and B represent the group

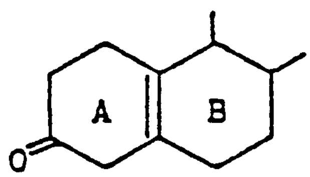

the radical $R_1$ does not represent a saturated linear alkyl radical, as well as the salts of addition of products with the formula I with acids,

characterized in that:

a) to prepare the products with the formula $I_A$:

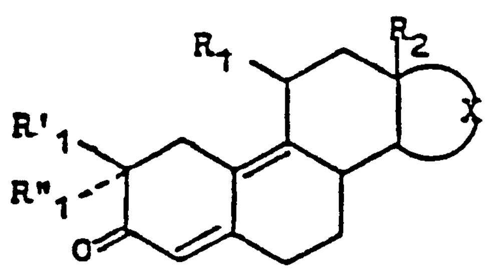
$(I_A)$

in which $R_1$, $R_2$ and X retain the same significance as previously and $R_1$ and $R''_1$ are such that, either $R'_1$ and $R''_1$ each represent an alkyl radical or one represents a hydrogen atom and the other represents an alkyl radical or $R'_1$ and $R''_1$ each represent a nitrile radical or one represents an alkyl radical and the other a nitrile radical, a product with the formula II:

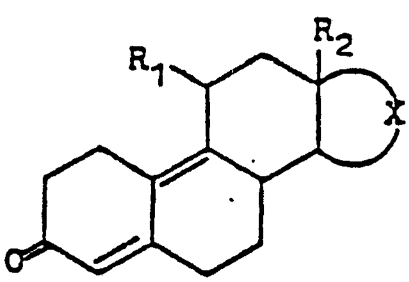
(II)

in which $R_1$, $R_2$ and X retain the same significance as previously, is submitted first, possibly to the action of a protector reagent of the functional groups then to the action of a strong base and either to the action of an alkyl halogenide or of tosyl cyanide, or first to the action of an alkyl halogenide then of tosyl cyanide, then if necessary the protector group is eliminated;

b) 1) to prepare the products with the formula $I_B$:

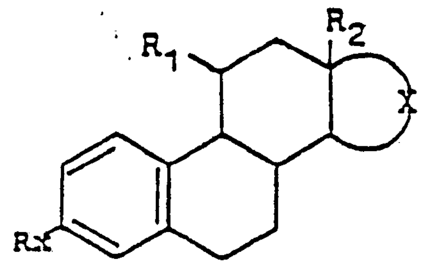
$(I_B)$

answering to the formula $IB_1$:

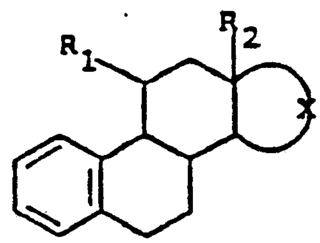
$(IB_1)$

in which $R_1$, $R_2$ and X retain the same significance as previously, a product with the formula II is submitted to the action of a reducing agent, so as to obtain a product with the formula $II_1$:

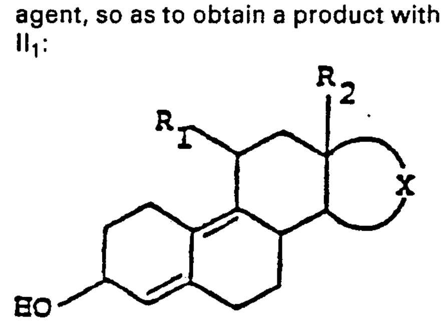
$(II_1)$

in which $R_1$, $R_2$ and X retain the same significance as above, which is submitted to the action of an acid aromatisation agent;

b) 2) to prepare the products with the formula $I_B$ answering to the formula $IB_2$:

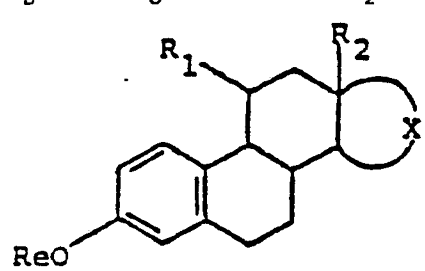
$(IB_2)$

in which $R_1$, $R_2$, Re and X retain the same significance as above, a product with the formula II is submitted to the action of an aromatisation agent then to saponification, then possibly to an alkylation or acylation reagent;

c) to prepare the products with the formulin

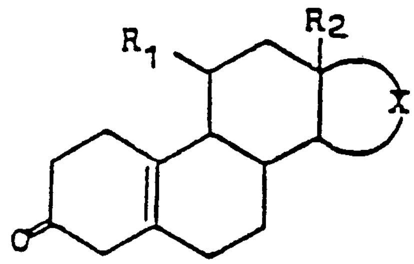

($I_C$)

which $R_1$, $R_2$ and X retain the same significance as previously, a product with the formula II is submitted to the action of a reducing agent;

d) to prepare the products with the formula $I_D$:

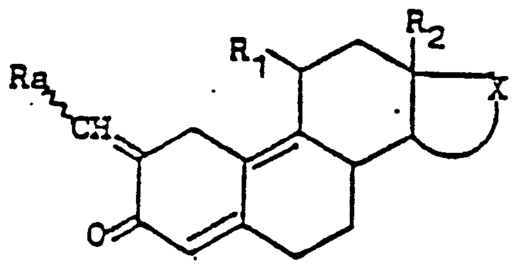

($I_D$)

in which $R_1$, $R_2$, X and Ra retain the same significance as previously, a product with the formula II is submitted to the action of a formylation reagent so as to obtain a product with the formula III

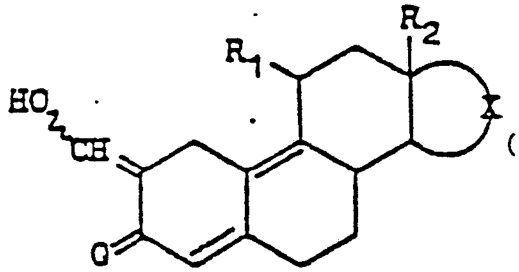

(III)

which product with the formula III, is either made to react with an acylation reagent, or is made to react with an amine with the formula

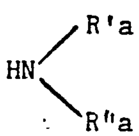

in which R'a and R"a retain the same significance as previously;

e) to prepare the products with the formula $I_E$:

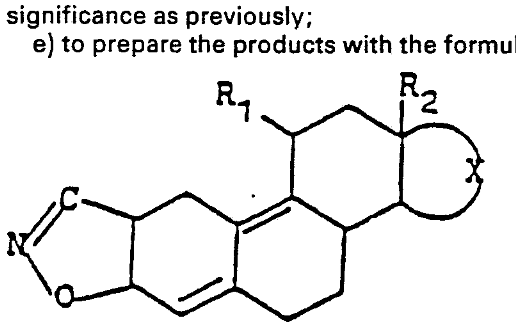

($I_E$)

in which $R_1$, $R_2$ and X retain the same significance as previously, hydroxylamine is made to react on a product with the formula III;

f) to prepare the products with the formula $I'_A$

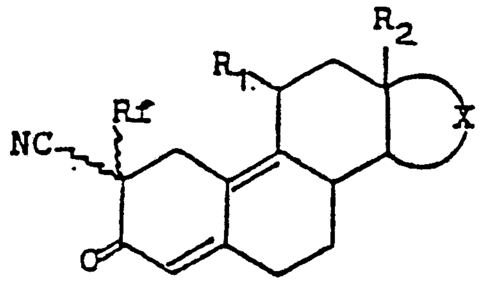

($I'_A$)

in which Rf represents a hydrogen atom or an alkyl radical and $R_1$, $R_2$ and X retain the same significance as previously, a base is made to react on the products with the formula IE so as to obtain the products with the formula $I'_A$ in which Rf represents a hydrogen atom and if desired the products thus obtained are submitted to the action of a strong base and an alkyl halogenide so as to obtain the products with the formula $I'_A$ in which Rf represents an alkyl radical.

2). Process according to claim 1, characterized in that the said process is carried out, starting with products with the formula (II) in which X represents the residue of a ring with the formula

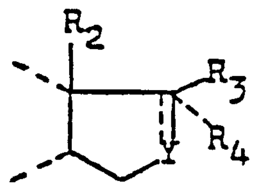

in which $R_2$ retains the same significance as in claim 1, the dotted line at 16-17 symbolizes the possible presence of a double bond, Y represents a radical

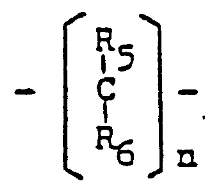

in which n represents the number 1 or 2, $R_5$ represents a hydrogen atom, an alkyl radical containing from 1 to 8 carbon atoms, an alkenyl or alkynyl radical containing from 2 to 8 carbon atoms, an aryl radical containing from 6 to 14 carbon atoms, or an aralkyl radical containing from 7 to 15 carbon atoms, $R_6$, identical to or different from $R_5$, can take one of the values indicated for $R_5$ and can also represent a hydroxyl radical, $R_3$ and $R_4$ identical or different, represent

either a hydrogen atom,

or a radical chosen from the group formed by the radicals OH, $Oalk_4$, $O-CO-alk_5$ in which $alk_4$ and $alk_5$ represent an alkyl radical containing from 1 to 8 carbon atoms or an aralkyl radical containing from 7 to 15 carbon atoms,

or an alkenyl or alkynyl radical containing from 2 to 8 carbon atoms,

or a radical

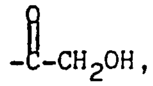

or a radical $-COCH_2OCOalk_6$, in which $alk_6$ represents an alkyl radical containing from 1 to 8 carbon atoms possibly substituted or an aralkyl radical containing from 7 to 15 carbon atoms,

or a radical $CO-CO_2H$,

or a radical $CO-CO_2-alk_7$ in which $alk_7$ represents an alkyl radical containing from 1 to 8 carbon atoms,

or a radical 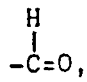

or a radical 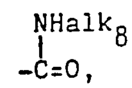

in which $alk_8$ represents an alkyl radical containing from 1 to 8 carbon atoms or an aralkyl radical containing from 7 to 15 carbon atoms,

or a radical $-C \equiv N$,

or $R_3$ and $R_4$ together form a radical

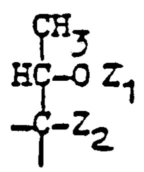

in which $Z_1$ represents a hydrogen atom, an alkyl radical or an acyl radical containing from 1 to 8 carbon atoms and $Z_2$ an alkyl radical containing from 1 to 8 carbon atoms,

or $R_3$ and $R_4$ together form a radical

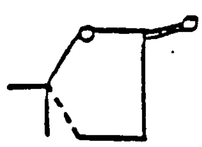

3) Process according to claim 2, characterized in that the said process is carried out, starting with products with the formula (II) in which the ring D does not carry an unsaturation, $R_5$ and $R_6$ represent a hydrogen atom and n is equal to 1.

4) Process acording to claim 2, characterized in that the said process is carried out, starting with products with the formula (II) in which $R_3$ represents a radical OH or $OCOalk_5$ and $R_4$ represents an alkenyl or alkynyl radical having at the most 4 carbon atoms, $alk_5$ keeping the same significance as in claim 2.

5) Process according to any one of the claims 1 to 4, characterized in that the said process is carried out, starting with products with the formula (II) in which $R_1$ represents an aryl or aralkyl radical carrying an amine function

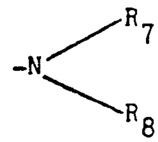

in which $R_7$ and $R_8$ represent an alkyl radical containing from 1 to 8 carbon atoms or a primary, secondary or tertiary alkyl radical containing from 1 to 8 carbon atoms, including one or more heteroatoms chosen from the group composed of oxygen, nitrogen and sulphur, of which at least one is a nitrogen atom, or substituted by a heterocycle including at least one nitrogen atom.

6) Process according to any one of the claims 1 to 4, characterized in that the said process is carried out, starting with products with the formula (II) in which $R_1$ represents a 2, 3 or 4-pyridyl radical,

a radical

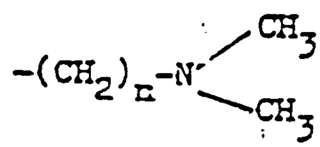 $(n \geq 3)$,

a radical:

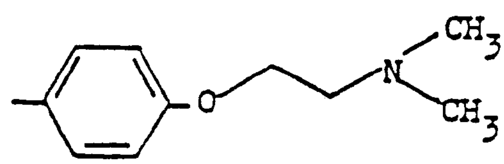 ,

a radical

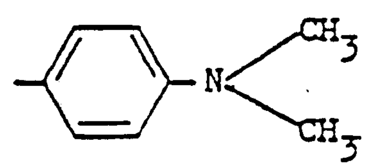

a radical

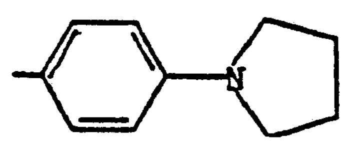

or a radical

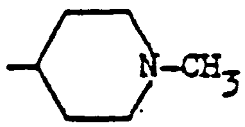

7) Process according to any one of the claims 1 to 4,
characterized in that the said process is carried out, starting with products with the formula (II) in which $R_1$ represents a thienyl or a furyl radical, or a cycloalkyl radical having from 3 to 6 carbon atoms, or a phenyl radical possibly substituted by one or more radicals chosen from among the hydroxy, halogen, trifluoromethyl, alkyl, alkoxy, alkylthio radicals possibly oxidized in the form of sulphoxide or sulphone, it being understood that the rings A and B do not represent the group

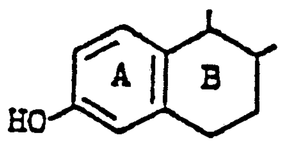

8) Process according to any one of the claims 1 to 4, characterized in that the said process is carried out, starting with products with the formula (II) in which $R_1$ represents a phenyl radical substituted by a radical chosen from the group formed by the chloro, fluoro, methylthio, methylsulphonyl, methoxy, hydroxy and allyloxy radicals.

9) Process according to any one of the claims 1 to 8, characterized in that the said process is carried out, starting with products with the formula (II) in which the rings A and B represent the group

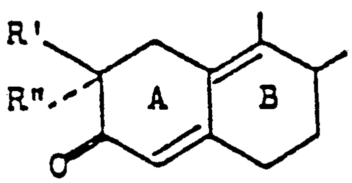

in which R' and R'' are such that
- either R' and R'' being identical each represent a methyl radical or a nitrile radical
- or one represents a hydrogen atom and the other represents a methyl or nitrile radical.

10) Process according to any one of the claims 1 to 8, characterized in that the said process is carried out, starting with products with the formula (II) in which the rings A and B represent the group

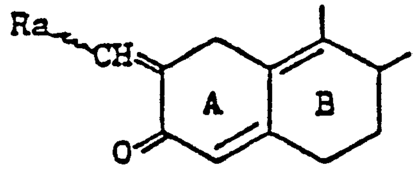

in which Ra represents a morpholine radical or an acetyloxy radical.

11) Process according to claim 1, characterized in that the said process is carried out, starting with products with the formula (II) in which the substituent

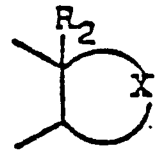

represents a radical

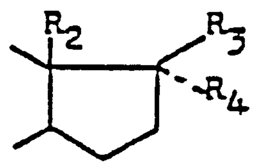

in which $R_2$ represents a methyl radical, $R_3$ represents a radical -OH or 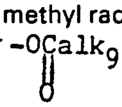

$alk_9$ being an alkyl radical having from 1 to 4 carbon atoms, $R_4$ represents an alkenyl or alkynyl radical having from 2 to 4 carbon atoms and $R_1$ represents a phenyl radical substituted by a radical -O$alk_{10}$, $alk_{10}$ being an alkyl radical having from 1 to 4 carbon atoms, or

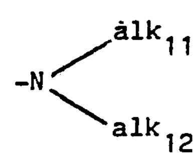

$alk_{11}$ and $alk_{12}$ being alkyl radicals having from 1 to 4 carbon atoms.

12) Process acording to any one of the claims 1 to 9, characterized in that any one of the products are prepared answering to the following formulae:
- 2,2-dimethyl-17β-hydroxy-11β-(4-dimethylaminophenyl)-17α-(prop-1-ynyl)estra-4,9-dien-3-one,
- 2,2-dicyano-11β-(4-dimethylaminophenyl)-17β-hydroxy-17α-(prop-1-ynyl)estra-4,9-dien-3-one,
- 2α-methyl-11β-(4-dimethylaminophenyl)-17β-hydroxy-17α-(prop-1-ynyl)estra-4,9-dien-3-one,
- 2 β-methyl-11β-(4-dimethylaminophenyl)-17β-hydroxy-17α -(prop-1-ynyl)estra-4,9-dien-3-one.
- 2-cyano-11β-(4-dimethylaminophenyl)-17 β-hydroxy-17α-(prop-1-ynyl)estra-4,9-dien-3-one,

and their salts.

13) Process according to any one of the claims 1 to 8, characterized in that any one of the products answering to the following formulae are prepared:

- 11 β -(4-dimethylaminophenyl)-17 α-(prop-1-ynyl)estra-1,3,5(10)-triene-3,17-diol,
- 17β-acetoxy-11β-(4-dimethylaminophenyl)-17α-(prop-1-ynyl)estra-1,3,5(10)-trien-3-ol,
- 11 β-(4-dimethylaminophenyl)-3-methoxy-17α-(prop-1-ynyl)estra-1,3,5(10)-trien-17 -ol,
- 17β -acetoxy-11β-(4-dimethylaminophenyl)-3-methoxy-17 α-(prop-1-ynyl)estra-1,3,5(10)-triene

and their salts.

14) Process according to any one of the claims 1 to 8, characterized in that any one of the products answering to the following formulae are prepared:

- 17β-hydroxy-11β-(3-methoxyphenyl)-17α-(prop-1-ynyl)estra-5(10)-en-3-one,
- E-17β-hydroxy-11β -(3-methoxyphenyl)-17α-(prop-1-enyl)estra-5(10)-en-one,
- 17β-hydroxy-11 β-(4-dimethylaminophenyl)-17α-(prop-1-ynyl)estr-5(10)-en-3-one,

and their salts.

15) Process according to any one of the claims 1 to 8 and 10, characterized in that any one of the products answering to the following formulae are prepared:

- 2[(acetyloxy)methylene]-11β-(4-dimethylaminophenyl)-17β-hydroxy-17α-(prop-1-ynyl)estra-4,9-dien-3-one,
- 11β -(4-dimethylaminophenyl)-17β-hydroxy-2-(4-morpholinylmethyl-ene)-17α-(prop-1-ynyl)estra-4,9-dien-3-one

and their salts.

16) Process acording to any one of the claims 1 to 8, characterized in that 11β-(4-dimethylaminophenyl)-17 α-(prop-1-ynyl)isoxazolo-[4,5-b]-estra-4,9-dien-17-ol and its salts are prepared.

17) Process according to any one of the claims 1 to 3, characterized in that any one of the products answering to the following formulae are prepared:

- 11β-[4-(2-dimethylaminoethoxy)phenyl]-estra-1,3,5(10)-triene-3,17β-diol,
- 11β -[4-(2-dimethylaminoethoxy)phenyl]-estra-1,3,5(10)-trien-17β-ol,
- 3-(2-dimethylamino)ethoxy-11β-phenyl-estra-1,3,5(10)-trien-17β-ol,

and their salts.